# EUROPÄISCHE PATENTSCHRIFT

(11) **EP 1 513 843 B1**
(45) Veröffentlichungstag und Bekanntmachung des Hinweises auf die Patenterteilung: **04.07.2007**
(21) Anmeldenummer: 03732394.6
(22) Anmeldetag: 16.05.2003
(51) Int. Cl.: C07D 487/04, A01N 43/90

(54) **PYRAZOLOPYRIMIDINE UND IHRE VERWENDUNG ZUR BEKÄMPFUNG VON SCHÄDLICHEN ORGANISMEN**
PYRAZOLOPYRIMIDINES AND THE USE THEREOF FOR CONTROLLING HARMFUL ORGANISMS
PYRAZOLOPYRIMIDINES ET LEUR UTILISATION COMME PESTICIDES

(30) Priorität: 29.05.2002 DE 10223917
(43) Veröffentlichungstag der Anmeldung: 16.03.2005
(73) Patentinhaber: Bayer CropScience AG, 40789 Monheim (DE)
(72) Erfinder: GEBAUER, Olaf, 50737 Köln (DE); GREUL, Jörg, Nico, 42799 Leichlingen (DE); GAYER, Herbert, 40789 Monheim (DE); KRÜGER, Bernd-Wieland, 51467 Bergisch Gladbach (DE); ELBE, Hans-Ludwig, 42329 Wuppertal (DE); DUNKEL, Ralf, 40789 Monheim (DE); GUTH, Oliver, 51371 Leverkusen (DE); VOERSTE, Arnd, 50677 Köln (DE); HILLEBRAND, Stefan, 41462 Neuss (DE); HERRMANN, Stefan, 40764 Langenfeld (DE); HEINEMANN, Ulrich, 42799 Leichlingen (DE); EBBERT, Ronald, 40789 Monheim (DE); KUCK, Karl-Heinz, 40764 Langenfeld (DE); LÖSEL, Peter, 51371 Leverkusen (DE); WACHENDORFF-NEUMANN, Ulrike, 56566 Neuwied (DE); MAULER-MACHNIK, Astrid, 42799 Leichlingen (DE)
(86) Internationale Anmeldenummer: PCT/EP2003/005159
(87) Internationale Veröffentlichungsnummer: WO 2004/000844

(56) Entgegenhaltungen:
- EP-A- 0 834 513
- DE-A- 3 130 633
- FR-A- 2 794 745
- US-A1- 2002 049 318

## Beschreibung

Die vorliegende Erfindung betrifft neue Pyrazolopyrimidine, mehrere Verfahren zu ihrer Herstellung und ihre Verwendung zur Bekämpfung von schädlichen Organismen. Die Erfindung betrifft außerdem neue Zwischenprodukte sowie Verfahren zu deren Herstellung.

Es ist bereits bekannt geworden, dass bestimmte Pyrazolopyrimidine fungizide Eigenschaften besitzen (vergleiche DE-A 3 130 633 oder FR-A 2 794 745). Die Wirkung dieser Stoffe ist gut, lässt aber bei niedrigen Aufwandmengen in manchen Fällen zu wünschen übrig.

Es wurden nun neue Pyrazolopyrimidine der Formel 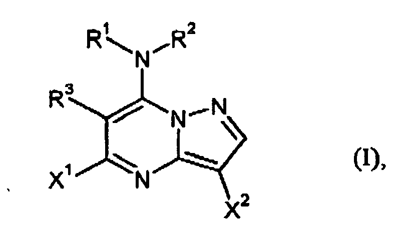 in welcher
- R¹: steht für Hydroxy, Amino, für gegebenenfalls durch Halogen, Cyano, Hydroxy, Amino, Phenyl, Heterocyclyl, Alkoxy mit 1 bis 4 Kohlenstoffatomen, Alkoxycarbonyl mit 1 bis 4 Kohlenstoffatomen im Alkoxyteil, Alkylamino mit 1 bis 4 Kohlenstoffatomen, Dialkylamino mit 2 bis 8 Kohlenstoffatomen, Cycloalkyl mit 3 bis 6 Kohlenstoffatomen, Halogencycloalkyl mit 3 bis 6 Kohlenstoffatomen und 1 bis 5 Halogenatomen, Alkylthio mit 1 bis 4 Kohlenstoffatomen, Oxo, Hydroxyimino und/oder Alkoximino mit 1 bis 4 Kohlenstoffatomen substituiertes Alkyl mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen,
für gegebenenfalls durch Halogen, Cycloalkyl, Cyano, Halogenalkyl mit 1 bis 2 Kohlenstoffatomen und 1 bis 5 Halogenatomen, Phenyl und/oder Heterocyclyl substituiertes Cycloalkyl mit 3 bis 7 Kohlenstoffatomen,
für gegebenenfalls durch Halogen, Cycloalkyl, Cyano, Phenyl und/oder Heterocyclyl substituiertes Alkoxy mit 1 bis 7 Kohlenstoffatomen,
für gegebenenfalls durch Halogen, Cycloalkyl, Cyano, Phenyl und/oder Heterocyclyl substituiertes Alkenyloxy mit 2 bis 6 Kohlenstoffatomen,
für gegebenenfalls durch Halogen, Cycloalkyl, Cyano, Phenyl und/oder Heterocyclyl substituiertes Alkinyloxy mit 2 bis 6 Kohlenstoffatomen,
für gegebenenfalls durch Halogen, Cycloalkyl, Cyano, Phenyl und/oder Heterocyclyl substituiertes Cycloalkyloxy mit 3 bis 7 Kohlenstoffatomen,
für gegebenenfalls durch Halogen, Cycloalkyl, Cyano, Phenyl und/oder Heterocyclyl substituiertes Alkylamino mit 1 bis 7 Kohlenstoffatomen,
für gegebenenfalls durch Halogen, Cycloalkyl, Cyano, Phenyl und/oder Heterocyclyl substituiertes Dialkylamino mit 1 bis 7 Kohlenstoffatomen in jedem der Alkylreste,
für gegebenenfalls durch Halogen, Cycloalkyl, Cyano, Phenyl und/oder Heterocyclyl substituiertes Alkenylamino mit 2 bis 6 Kohlenstoffatomen,
für gegebenenfalls durch Halogen, Cycloalkyl, Cyano, Phenyl und/oder Heterocyclyl substituiertes Alkinylamino mit 2 bis 6 Kohlenstoffatomen,
für gegebenenfalls durch Halogen, Cycloalkyl, Cyano, Phenyl und/oder Heterocyclyl substituiertes Cycloalkylamino mit 3 bis 7 Kohlenstoffatomen,
für gegebenenfalls durch Halogen, Cycloalkyl, Cyano, Phenyl und/oder Heterocyclyl substituiertes N-Cycloalkyl-N-alkyl-amino mit 3 bis 7 Kohlenstoffatomen im Cycloalkylteil und 1 bis 7 Kohlenstoffatomen in Alkylteil,
für gegebenenfalls durch Halogen, Cycloalkyl, Cyano, Phenyl und/oder Heterocyclyl substituiertes Alkylidenamino mit 2 bis 6 Kohlenstoffatomen,
oder
für gegebenenfalls durch Halogen, Alkyl, Cycloalkyl, Cyano, Phenyl und/oder Heterocyclyl substituiertes Heterocyclyl mit 5 oder 6 Ringgliedern,
wobei die zuvor genannten Heterocyclyl-Reste einfach bis dreifach, gleichartig oder verschieden substituiert sein können durch
Halogen, Hydroxy, Phenyl, 1,2-Dioxyethylen, Alkyl mit 1 bis 4 Kohlenstoffatomen, Halogenalkyl mit 1 oder 2 Kohlenstoffatomen und 1 bis 5 Halogenatomen, Alkoxy mit 1 bis 4 Kohlenstoffatomen, Alkylthio mit 1 bis 4 Kohlenstoffatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen und wobei die zuvor genannten Heterocyclylreste gesättigt oder teilweise ungesättigt sind,
und wobei die zuvor genannten Phenyl-Reste einfach bis dreifach, gleichartig oder verschieden substituiert sein können durch
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
in 2,3-Position verknüpftes 1,3-Propandiyl, 1,4-Butandiyl, Methylendioxy (-O-CH₂-O-) oder 1,2-Ethytendioxy (-O-CH₂-CH₂-O-), wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen.
- R²: steht für Wasserstoff,
für gegebenenfalls durch Halogen, Cycloalkyl mit 3 bis 6 Kohlenstoffatomen Alkoxy mit 1 bis 4 Kohlenstoffatomen, Alkylthio mit 1 bis 4 Kohlenstoffatomen, Oxo, Hydroximino und/oder Alkoximino mit 1 bis 4 Kohlenstoff atomen substituiertes Alkyl mit 1 bis 4 Kohlenstoffatomen,
für gegebenenfalls durch Halogen und/oder Cycloalkyl mit 3 bis 6 Kohlenstoffatomen substituiertes Alkenyl mit 2 bis 4 Kohlenstoffatomen,
für gegebenenfalls durch Halogen und/oder Cycloalkyl mit 3 bis 6 Kohlenstoffatomen substituiertes Alkinyl mit 2 bis 4 Kohlenstoffatomen oder
für gegebenenfalls durch Halogen und/oder Cycloalkyl mit 3 bis 6 Kohlenstoffatomen substituiertes Cycloalkyl mit 3 bis 6 Kohlenstoffatomen.
- R¹ und R²: stehen auch gemeinsam mit dem Stickstoffatom, an das sie gebunden sind, für einen 3- bis 6-gliedrigen heterocyclischen Ring, der gesättigt oder teilweise gesättigt ist, der neben dem bereits erwähnten Stickstoffatom noch ein weiteres Heteroatom aus der Reihe Stickstoff, Sauerstoff und Schwefel enthalten kann und der einfach bis dreifach, gleichartig oder verschieden substituiert sein kann durch
Halogen, Hydroxy, Cyano, Morpholinyl, Amino, einen annelierten Phenylring, eine Methylen- oder Ethylenbrücke,
Alkyl mit 1 bis 4 Kohlenstoffatomen,
Halogenalkyl mit 1 bis 4 Kohlenstoffatomen und 1 bis 9 gleichen oder verschiedenen Halogenatomen,
Alkylcarbonylamino mit 1 bis 4 Kohlenstoffatomen im Alkylteil,
Dialkylamino mit 2 bis 8 Kohlenstoffatomen,
Alkoxycarbonylamino mit 1 bis 4 Kohlenstoffatomen im Alkoxyteil, Di(alkoxycarbonyl)amino mit 2 bis 8 Kohlenstoffatomen in den Alkoxyteilen, Hydroxyalkyl mit 1 bis 4 Kohlenstoffatomen,
Alkoxycarbonyl mit 1 bis 4 Kohlenstoffatomen im Alkoxyteil und/oder
Alkylcarbonyl mit 1 bis 4 Kohlenstoffatomen im Alkylteil.
- R³: steht für Phenyl, das einfach bis vierfach, gleichartig oder verschieden substituiert sein kann durch
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
in 2,3-Position verknüpftes 1,3-Propandiyl, 1,4-Butandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen.
- X¹: steht für Wasserstoff, Fluor, Chlor oder Brom.
- X²: steht für Cyano, Fluor, Chlor, Brom, Iod, Nitro, Formyl, Halogenalkyl mit 1 bis 4 Kohlenstoffatomen und 1 bis 9 Fluor, Chlor und/oder Bromatomen, Alkyl mit 1 bis 4 Kohlenstoffatomen, Cycloalkyl mit 3 bis 6 Kohlenstoffatomen, Thiocarbamoyl, Alkoxycarbonyl mit 1 bis 4 Kohlenstoffatomen im Alkoxyteil, Alkylcarbonyl mit 1 bis 4 Kohlenstoffatomen im Alkylteil, Hydroximinoalkyl mit 1 bis 4 Kohlenstoffatomen im Alkylteil oder für Alkoxyiminoalkyl mit 1 bis 4 Kohlenstoffatomen im Alkoxyteil und 1 bis 4 Kohlenstoffatomen im Alkylteil.
sowie Säure-Additionssalze von denjenigen Verbindungen der Formel (I),
in denen
- R¹: für Amino steht,
gefunden.

Die erfindungsgemäßen Verbindungen können je nach Substitutionsmuster gegebenenfalls als Mischungen verschiedener möglicher isomerer Formen, insbesondere von Stereoisomeren, wie z. B. E- und Z-, threo- und erythro-, sowie optischen Isomeren, gegebenenfalls aber auch in Form von Tautomeren vorliegen. Ist R³ an beiden Atomen, die der Bindungsstelle benachbart sind, ungleich substituiert, können die betreffenden Verbindungen in einer besonderen Form der Stereoisomerie vorliegen und zwar als Atropisomere.

Weiterhin wurde gefunden, dass sich Pyrazolopyrimidine der Formel (I) herstellen lassen, indem man
a) Halogen-pyrazolopyrimidine der Formel 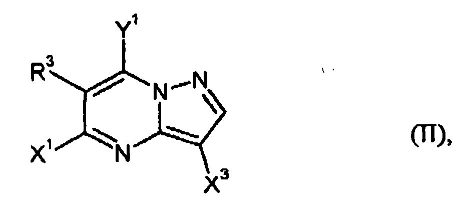 in welcher
   - R³ und X¹: die oben angegebenen Bedeutungen haben,
   - X³: für Halogen, Cyano, Nitro, Alkyl, Halogenalkyl, Cycloalkyl, Thiocarbamoyl, Alkoxycarbonyl oder Alkylcarbonyl steht und
   - Y¹: für Halogen steht,
   mit Aminen der Formel 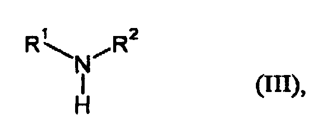 in welcher
   - R¹ und R²: die oben angegebenen Bedeutungen haben,
   gegebenenfalls in Gegenwart eines Verdünnungsmittels, gegebenenfalls in Gegenwart eines Katalysators und gegebenenfalls in Gegenwart eines Säureakzeptors, umsetzt,
   oder
b) Pyrazolopyrimidine der Formel (Ia) 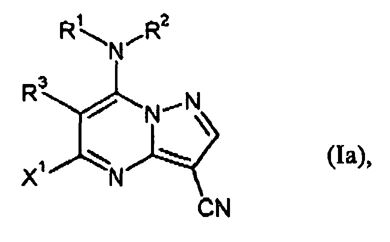 in welcher
   R¹, R², R³ und X¹ die oben angegebenen Bedeutungen haben,
   mit Diisobutyl-aluminiumhydrid in Gegenwart von wässriger Ammoniumchlorid-Lösung sowie in Gegenwart eines organischen Verdünnungsmittels umsetzt,
   oder
c) Pyrazolopyrimidine der Formel (Ib) 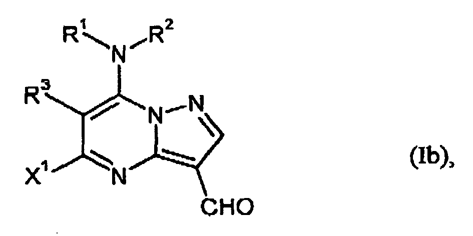 in welcher
   R¹, R², R³ und X¹ die oben angegebenen Bedeutungen haben,
   mit Amino-Verbindungen der Formel (IV)

   H₂N-OR⁴ (IV),

   in welcher
   - R⁴: für Wasserstoff oder Alkyl steht,
   in Gegenwart eines Verdünnungsmittels und gegebenenfalls in Gegenwart eines Katalysators umsetzt, wobei die Amino-Verbindungen der Formel (IV) auch in Form ihrer Säureadditions-Salze eingesetzt werden können,
   und gegebenenfalls an die so erhaltenen Verbindungen der Formel (I), in denen
   - R¹: für Amino steht,
   eine Säure addiert.

Schließlich wurde gefunden, dass sich die neuen Pyrazolopyrimidine der Formel (I) bzw. deren Säure-Additionssalze sehr gut zur Bekämpfung von Schadorganismen eignen. Sie zeigen vor allem eine starke Wirksamkeit gegen unerwünschte Mikroorganismen, wie Fungi und Bakterien. Außerdem besitzen die erfindungsgemäßen Stoffe auch eine sehr gute insektizide und nematizide Wirkung.

Überraschenderweise besitzen die erfindungsgemäßen Pyrazolopyrimidine der Formel (I) sowie deren Säure-Additionssalze eine wesentlich bessere Wirksamkeit gegen Schadorganismen als die konstitutionell ähnlichsten, vorbekannten Stoffe gleicher Wirkungsrichtung.

Die erfindungsgemäßen Pyrazolopyrimidine sind durch die Formel (I) allgemein definiert.
- R¹: steht bevorzugt für Hydroxy, Amino, Methyl, Ethyl, n-Propyl, i-Propyl, n-Butyl, i-Butyl, s-Butyl, t-Butyl, n-Pentyl, i-Pentyl, 1,2-Dimethylpropyl, 2,2-Dimethylpropyl, 1,2,2- Trimethylpropyl, oder
- R¹: steht bevorzugt für Methoxymethyl, 2-Methoxy-ethyl, Methylthiomethyl, 2-Methylthio-ethyl, Hydroximinomethyl, Methoximinomethyl, Acetylmethyl, 2-1-iydroximino-propyl, 2-Methoximino-propyl, Allyl, 2-Methyl-prop-2-enyl, Propargyl, 2,2,2-Trifluorethyl, 1-(Trifluormethyl)-ethyl, 3,3,3-Trifluorpropyl, Cyclopropylmethyl,
Cyclopropyloxy, Cyclobutyloxy, Cyclopentyloxy, Cyclohexyloxy, Difluormethoxy, Trifluormethoxy, Difluorchlormethoxy, Trifluorethoxy,
Methylamino, Ethylamino, n- oder i-Propylamino, n-, i-, s- oder t-Butylamino, Dimethylamino, Diethylamino, Trifluorethylamino, Cyclohexylmethylamino, 2-Cyanoethylamino, Allylamino, 1-Cyclopropylethylamino, Cyclopropylamino, Cyclobutylamino, Cyclopentylamino, Cyclohexylamino, 1-Methylethylidenamino,
für jeweils gegebenenfalls einfach oder zweifach, gleichartig oder verschieden durch Fluor, Chlor und/oder Methyl substituiertes Cyclopropyl, Cyclobutyl, Cyclopentyl, Cyclohexyl, Pyrrolidinyl, Piperidinyl, Morpholinyl, Thiamorpholinyl, Piperazinyl oder
für gegebenenfalls substituiertes Pyridylmethyloxy oder Thiazolylmethoxy,
oder
- R¹: steht bevorzugt für (2,2-Dichlorcyclopropyl)methyl, (2-Furyl)methyl, (2-Tetrahydrofuryl)methyl, (2-Tetrahydropyranyl)methyl, 1,3-Dioxolan-2-ylmethyl, 1-Cyclopropylethyl, Benzyloxy, 2,4-Dichlorbenzyloxy, 2,6-Dichlorbenzyloxy, 2-Chlorbenzyloxy, 2-Fluorcyclopropyl, 2-Hexahydropyranyloxy, 2-Thienylmethyl, 2-Trifluormethylcyclohexyl, 3-(Dimethylamino)-propyl, 3,5-bis-Trifluormethylcyclohexyl, 3,5-Dichlorbenzyloxy, 3-Aminopropyl, 3-Chlorbenzyloxy, 3-Trifluormethylbenzyloxy, 3-Trifluormethylcyclohexyl, 4-Trifluormethyl-cyclohexyl, 4-Chlorbenzyloxy, 4-Fluorbenzyloxy, 4-Trifluormethylbenzyloxy, -C(CH₃)₂-CF₃, -C(CH₃)₂-CH₂-COCH₃, -CH(CH₂OH)-COOCH₃, -CH(CH₃)-CH(O-CH₃)₂, -CH(CH₃)-CH=CH₂, -CH(CH₃)-CH₂-CH(CH₃)₂, -CH(CH₃)-CH₂-O-CH₃, -CH(CH₃)-CH₂-OH, -CH(CH₃)-COOCH₃, -CH(CH₃)-COO-t-butyl, -CH₂-C(CH₃)=CH₂, -CH₂-CH(OCH₃)₂, -CH₂-CH₂-CF₃, -CH₂-CH₂-Cl, -CH₂-CH₂-CN, -CH₂-CH₂-N(CH₃)₂, -CH₂-CH₂-N(CH₃)₂, -CH₂-CH₂-NH₂, -CH₂-CHF₂, -CH₂-CN, -CH₂-COOC₂H₅, -CH₂-COOCH₃, i-Butoxy, -NH-CH₂-CF₂-CHF₂, -NH-CH₂-CF₃, -NH-CH₂-CH(CH₃)₂, Methoxy, Ethoxy, i-Propoxy, t-Butoxy oder -O-CH(CH₃)-CH₂-CH₃,
wobei die zuvor genannten Thiazolyl- und Pyridyl-Reste im Falle von Thiazolyl einfach oder zweifach und im Falle von Pyridyl einfach bis dreifach, jeweils gleichartig oder verschieden substituiert sein können durch Fluor, Chlor, Brom, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, Difluormethoxy, Trifluormethoxy, Difluorchlormethoxy, Trifluorethoxy, Difluormethylthio, Difluorchlormethylthio, Dichlorfluormethylthio, Trifluormethylthio und/oder Phenyl,
und wobei die zuvor genannten Benzyloxy-Reste im Phenylteil einfach bis dreifach, gleichartig oder verschieden substituiert sein können durch
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
in 2,3-Position verknüpftes 1,3-Propandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl und/oder Trifluormethyl.
- R²: steht bevorzugt für Wasserstoff, Methyl, Ethyl, n- oder i-Propyl, n-, i-, s- oder t-Butyl, Methoxymethyl, 2-Methoxy-ethyl, Methylthiomethyl, 2-Methylthio-ethyl, Hydroximinomethyl, Methoximinomethyl, Acetylmethyl, 2-Hydroxyimino-propyl, 2-Methoxyimino-propyl, Allyl, Propargyl, 2,2,2-Trifluorethyl, 1-(1,1,1-Trifluormethyl)ethyl, Cyclopropylmethyl, Cyclobutylmethyl, Cyclopentylmethyl oder Cyclohexylmethyl.
- R¹ und R²: stehen bevorzugt gemeinsam mit dem Stickstoffatom, an welches sie gebunden sind, für 1-Pyrrolinyl, 3-Pyrrolinyl, Pyrrolidinyl, Dihydropyridinyl, Piperidinyl, Pyrazolinyl, Pyrazolidinyl, Imidazolinyl, Imidiazolidinyl, 1,2-Diazinan-yl, 1,3-Diazinan-yl, Piperazinyl, Oxazolinyl, Oxazolidinyl, Isoxazolyl, Isoxazolidinyl, Tetrahydropyridazinyl, Dihydrooxazinyl, Morpholinyl, Thiazolinyl, Thiazolidinyl oder Thiomorpholinyl, wobei die genannten Heterocyclen substituiert sein können durch
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Methylamino, Ethylamino, n- oder i-Propylamino, Dimethylamino, Diethylamino, Acetyl, Propionyl, Acetyloxa, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl, Cyclohexyl,
durch einen annelierten Phenylring oder
durch eine Methandiyl- oder Ethandiyl-Brücke,
oder
- R¹ und R²: stehen bevorzugt gemeinsam für eine Gruppierung der Formel

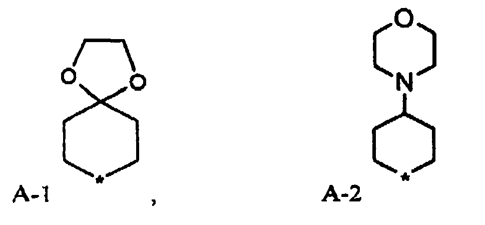

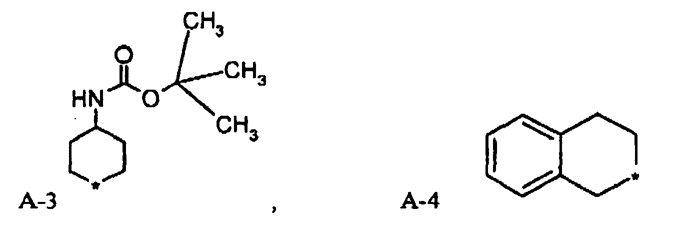

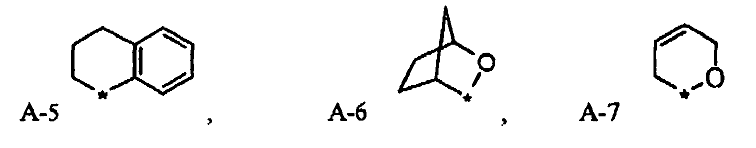

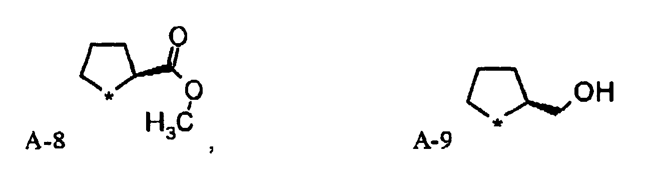

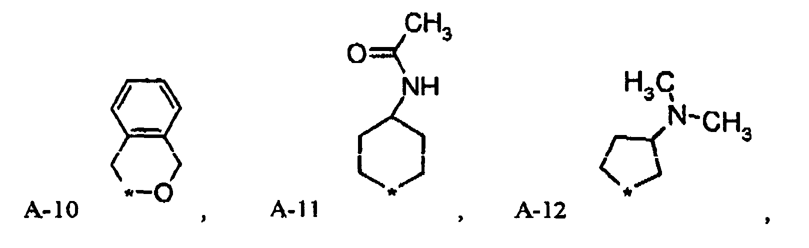

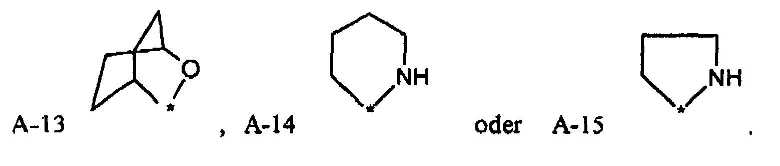

In diesen Gruppierungen ist die mit dem Stickstoffatom verbundene Stelle jeweils durch * bezeichnet.
- R³: steht bevorzugt für Phenyl, das einfach bis dreifach, gleichartig oder verschieden substituiert sein kann durch
Fluor, Chlor, Brom, Cyano, Nitro, Formyl, Methyl, Ethyl, n- oder i-Propyl, n-, i-, s- oder t-Butyl, Allyl, Propargyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
in 2,3-Position verknüpftes 1,3-Propandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl und/oder Trifluormethyl.
- X¹: steht bevorzugt für Wasserstoff, Fluor oder Chlor.
- X²: steht bevorzugt für Cyano, Fluor, Chlor, Brom, Tod, Formyl, Trifluormethyl, Methoxycarbonyl, Methylcarbonyl, Hydroximinomethyl, Methoximinomethyl, Thiocarbamoyl, Nitro, Methyl, Ethyl oder Cyclopropyl.
- R³: steht besonders bevorzugt für 2,4-, 2,5- oder 2,6-disubstituiertes Phenyl, oder für 2-substituiertes Phenyl oder für 2,4,6-trisubstituiertes Phenyl.

Eine besonders bevorzugte Gruppe sind Verbindungen der Formel (I), in welcher
- R¹: für Amino, Hydroxy, Methyl, Ethyl, n-Propyl, i-Propyl, n-Butyl, i-Butyl, s-Butyl, t-Butyl, 1,2-Dimethyl-propyl, 1,2,2- Trimethylpropyl, 2,2-Dimethylpropyl, Trifluormethyl, 2,2,2-Trifluormethyl, 2,2-Difluorethyl, 2,2,2-Trifluor-1-methyl-ethyl, 3,3,3-Trifluor-propyl, 2,2,2-Trifluor-1,1-dimethyl-ethyl, 3-Methyl-butyl, Allyl, 2-Methyl-prop-2-enyl, 2-Methoxyethyl, 2,2-Dimethoxyethyl, Cyclopropyl, Cyclopentyl, Cyclohexyl, 2-Fluorcyclopropyl, 2-Trifluormethyl-cyclohexyl, 3-Trifluorntethyl-cyclohexyl, 4-Trifluormethylcyclohexyl, 3,5-Di(trifluormethyl)-cyclohexyl, Cyclopropyl-methyl, Dichlorcyclopropyl-methyl, 1-Cyclohexyl-ethyl, 2-Furyl-methyl, 2-Tetrahydrofurylmethyl, 2-Thienyl-methyl, 1,3-Dioxolan-2-yl-methyl, Propargyl, Methoxycarbonylmethyl, Ethoxycarbonylmethyl, 2-Amino-ethyl, 3-Amino-propyl, 2-Dimethylamino-ethyl, Cyanomethyl, 2-Cyano-ethyl, 2-Vinyloxy-ethyl, Pyrrolidinyl, Piperidinyl, Morpholinyl, Thiomorpholinyl oder Piperazinyl steht,
- R²: für Wasserstoff, Methyl, Ethyl, n- oder i-Propyl, n-, i-, s- oder t-Butyl, Allyl, Propargyl, 2,2,2-Trifluorethyl, 1-(1,1,1-Trifluormethyl)ethyl, Cyclopropylmethyl, Cyclobutylmethyl, Cyclopentylmethyl, Cyclohexylmethyl oder Cyclopropyl steht oder
- R¹ und R²: gemeinsam mit dem Stickstoffatom, an das sie gebunden sind, für jeweils gegebenenfalls einfach oder zweifach, gleichartig oder verschieden durch Fluor, Chlor, Brom, Cyano, Hydroxy, Methyl, Ethyl, Trifluormethyl, Methylcarbonyl, Methylcarbonylamino oder Methoxycarbonyl substituiertes Pyrrolidinyl, Piperidinyl, Morpholinyl, Thiomorpholinyl, Piperazinyl, 5-Methyl-3,6-dihydro-1(2H)-pyridinyl, 5-Ethyl-3,6-dihydro-1-(2H)-pyridinyl oder Tetrahydro-1-(2H)-pyridazinyl stehen oder für eine Gruppierung der Formel 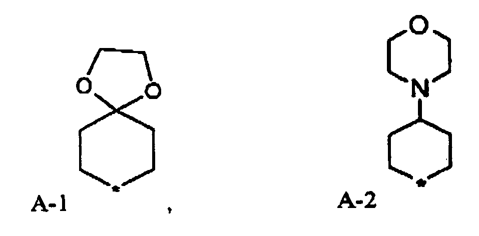 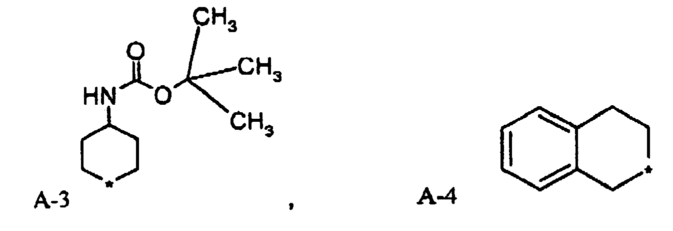 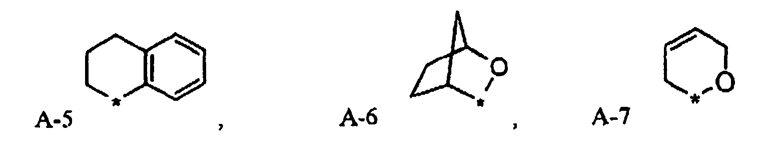 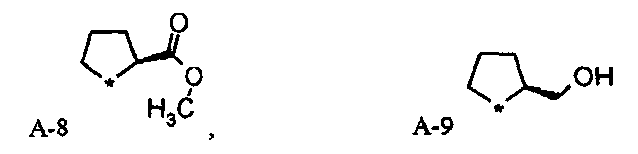 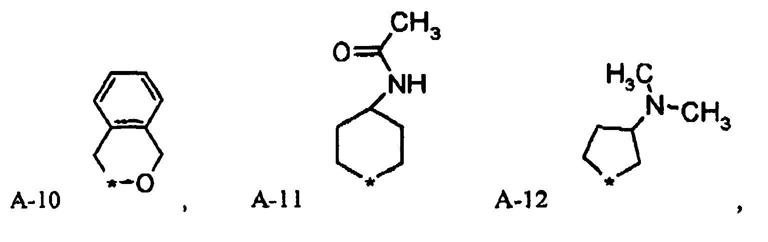 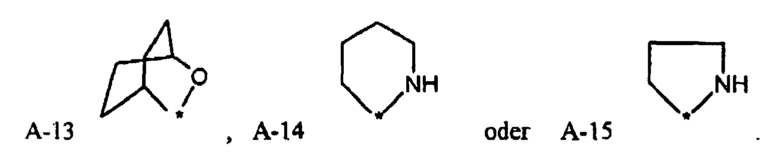 stehen,
- R³: für einfach bis dreifach in 2-, 4- und/oder 6-Position durch Fluor und/oder Chlor substituiertes Phenyl steht,
oder
- R³: für 2-Trifluormethylphenyl, 2-Chlor-5-nitro-phenyl oder 2-Chlor-4-methoxyphenyl steht,
- X¹: für Wasserstoff oder Chlor steht und
- X²: für Fluor, Chlor, Brom, Iod, Cyano, Nitro, Methyl, Cyclopropyl, Formyl, Thiocarbamoyl oder Methoximinomethyl steht.

Die zuvor genannten Reste-Definitionen können untereinander in beliebiger Weise kombiniert werden. Außerdem können auch einzelne Bedeutungen entfallen.

Bevorzugte erfindungsgemäße Verbindungen sind auch Adttitionsprodukte aus Säuren und denjenigen Pyrazolopyrimidinen der Formel (I), in denen
- R¹: für Amino steht und
- R², R³, X¹ und X²: diejenigen Bedeutungen haben, die für diese Reste als bevorzugt genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure, 1,5-Naphthalindisulfonsäure, Saccharin und Thiosaccharin.

Die oben aufgeführten allgemein oder in Vorzugsbereichen angegebenen Reste-definitionen gelten sowohl für die Endprodukte der Formel (I) als auch entsprechend für die jeweils zur Herstellung benötigten Ausgangsstoffe bzw. Zwischenprodukte.

Verwendet man 3-Cyano-5,7-dichlor-6-(2-chlor-phenyl)-pyrazolo[1,5-a]-pyrimidin und Methyl-ethyl-amin als Ausgangsstoffe, so kann der Verlauf des erfindungsgemäßen Verfahrens (a) durch das folgende Formelschema veranschaulicht werden.

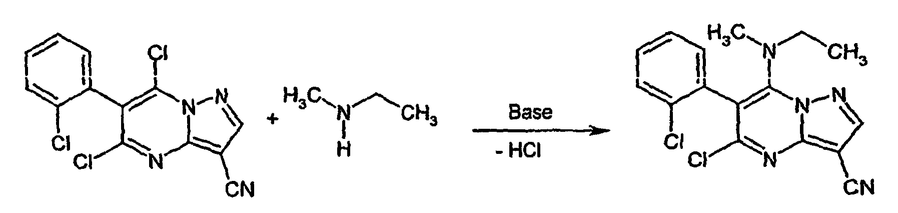

Verwendet man 3-Cyano-5-chlor-6-(2-chlor-4-fluor-phenyl)-7-(1,2,2-trimethyl-propylamino)-pyrazolo[1,5a]pyrimidin als Ausgangsstoff und Di-isobutyl-aluminiumhydrid als Reaktionskomponente, so kann der Verlauf des erfindungsgemäßen Verfahrens (b) durch das folgende Formelschema veranschaulicht werden.

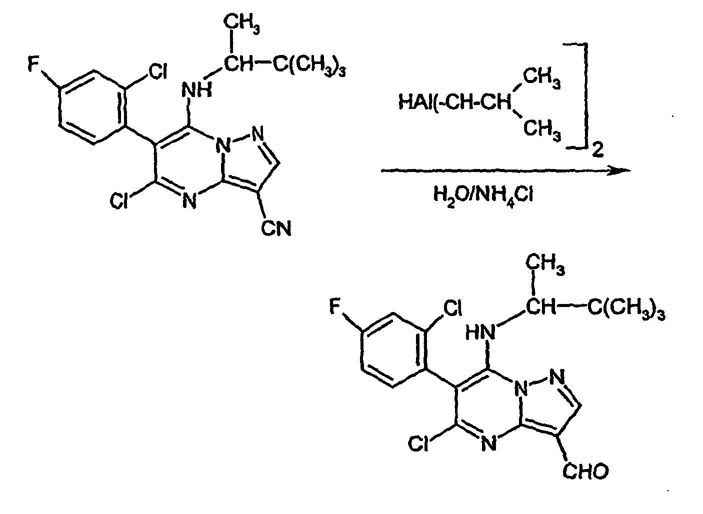

Verwendet man 3-Formyl-5-chlor-6-(2-chlor-4-fluor-phenyl)-7-(1,2,2-trimethyl-propylamino)-pyrazolo-[1,5a]pyrimidin und Methoxyamin-hydrochlorid als Ausgangsstoffe, so kann der Verlauf des erfindungsgemäßen Verfahrens (c) durch das folgende Formelschema veranschaulicht werden.

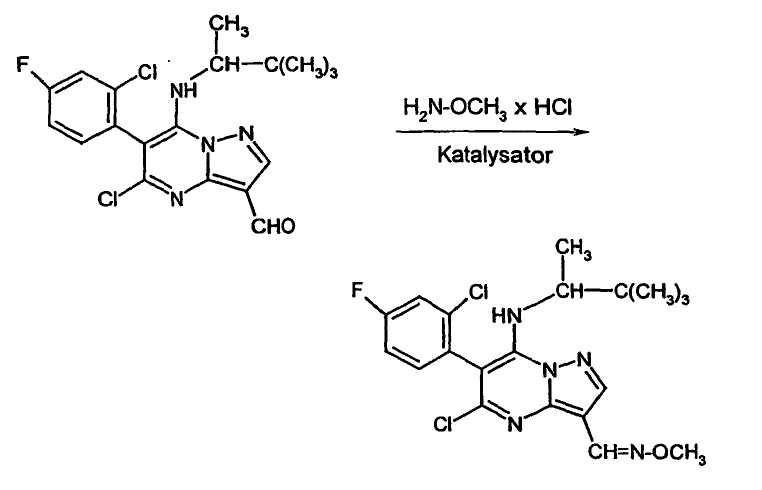

Die zur Durchführung des erfindungsgemäßen Verfahrens (a) als Ausgangsstoffe benötigten Halogenpyrazolopyrimidine sind durch die Formel (II) allgemein definiert. In dieser Formel (II) haben R³ und X¹ vorzugsweise diejenigen Bedeutungen, die bereits im Zusammenhang mit der Beschreibung der erfindungsgemäßen Verbindungen der Formel (I) für diese Reste als bevorzugt genannt wurden. Y¹ steht vorzugsweise für Fluor, Chlor oder Brom, besonders bevorzugt für Fluor oder Chlor.
- X³: steht vorzugsweise für Cyano, Fluor, Chlor, Brom, Iod, Nitro, Halogenalkyl mit 1 bis 4 Kohlenstoffatomen und 1 bis 9 Fluor-, Chlor- und/oder Bromatomen, Alkyl mit 1 bis 4 Kohlenstoffatomen, Cycloalkyl mit 3 bis 6 Kohlenstoffatomen, Thiocarbamoyl, Alkoxycarbonyl mit 1 bis 4 Kohlenstoffatomen im Alkoxyteil oder Alkylcarbonyl mit 1 bis 4 Kohlenstoffatomen im Alkylteil.
- X³: steht besonders bevorzugt für Cyano, Fluor, Chlor, Brom, Iod, Trifluormethyl, Methoxycarbonyl, Methylcarbonyl, Thiocarbamoyl, Nitro, Methyl, Ethyl oder Cyclopropyl.
- X³: steht ganz besonders bevorzugt für Fluor, Chlor, Brom, Iod, Cyano, Nitro, Methyl, Cyclopropyl oder Thiocarbamoyl.

Die Halogenpyrazolopyrimidine der Formel (II) sind neu. Auch diese Stoffe eignen sich zur Bekämpfung von Schädlingen, insbesondere zur Bekämpfung von unerwünschten Mikroorganismen.

Die Halogen-pyrazolopyrimidine der Formel (II) lassen sich herstellen,
indem man
d) Hydroxy-pyrazolopyrimidine der Formel 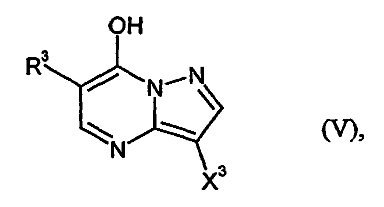 in welcher
   - R³ und X³: die oben angegebenen Bedeutungen haben,
   mit Halogenierungsmitteln, gegebenenfalls in Gegenwart eines Verdünnungsmittels umsetzt,
   oder
e) Dihydroxy-pyrazolopyrimidine der Formel 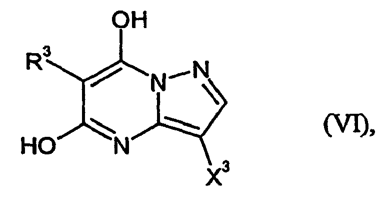 in welcher
   R³ und X³ die oben angegebenen Bedeutungen haben,
   mit Halogenierungsmitteln, gegebenenfalls in Gegenwart eines Verdünnungsmittels umsetzt.

Die zur Durchführung des erfindungsgemäßen Verfahrens (d) als Ausgangsstoffe benötigten Hydroxy-pyrazolopyrimidine sind durch die Formel (V) allgemein definiert. In dieser Formel haben R³ und X³ vorzugsweise diejenigen Bedeutungen, die bereits im Zusammenhang mit der Beschreibung der erfindungsgemäßen Verbindungen der Formeln (I) und (II) für diese Reste als bevorzugt genannt wurden.

Auch die Hydroxy-pyrazolopyrimidine der Formel (V) sind bisher noch nicht bekannt. Sie lassen sich herstellen, indem man
f) Acrylsäureester der Formel 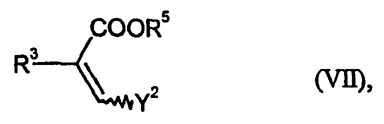 in welcher
   - R³: die oben angegebene Bedeutung hat,
   - R⁵: für Alkyl steht und
   - Y²: für Alkoxy oder Dialkylamino steht,
   mit Aminopyrazolen der Formel 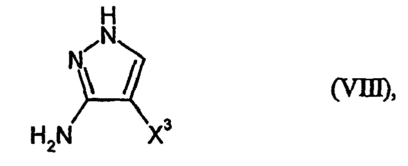 in welcher
   - X³: die oben angegebene Bedeutung hat,
   gegebenenfalls in Gegenwart eines Verdünnungsmittels und gegebenenfalls in Gegenwart einer starken Base umsetzt.

Die bei der Durchführung des erfindungsgemäßen Verfahrens (f) als Ausgangsstoffe benötigten Acrylsäureester sind durch die Formel (VII) allgemein definiert. In dieser Formel hat R³ vorzugsweise diejenigen Bedeutungen, die bereits im Zusammenhang mit der Beschreibung der erfindungsgemäßen Stoffe der Formel (I) für diesen Rest als bevorzugt genannt wurden. R⁵ steht vorzugsweise für Alkyl mit 1 bis 4 Kohlenstoffatomen, besonders bevorzugt für Methyl oder Ethyl. Y² steht vorzugsweise für Alkoxy mit 1 bis 4 Kohlenstoffatomen oder für Dialkylamino mit 1 bis 4 Kohlenstoffatomen in jeder Alkylgruppe. Besonders bevorzugt steht Y² für Methoxy, Ethoxy oder für Dimethylamino.

Die Acrylsäureester der Formel (VII) sind bekannt oder lassen sich nach bekannten Methoden herstellen (vgl. EP-A 0 165 448).

Die bei der Durchführung des erfindungsgemäßen Verfahrens (f) als Reaktionskomponenten benötigten Aminopyrazole sind durch die Formel (VIII) allgemein definiert. In dieser Formel hat X³ vorzugsweise diejenigen Bedeutungen, die bereits im Zusammenhang mit der Beschreibung der erfindungsgemäßen Stoffe der Formel (II) für diesen Substituenten als bevorzugt genannt wurden.

Die Aminopyrazole der Formel (VIII) sind bekannt oder lassen sich nach bekannten Methoden herstellen (vgl. Tetrahedron Lett. 21, 2029-2031 (1967); Liebigs Ann. Chem. 707, 141-146 (1967), Monatsh. Chem. 1998, 1329 (12), 1313-1318) und J. Med. Chem. 25 (1982), 239 ff).

Die bei der Durchführung des erfindungsgemäßen Verfahrens (e) als Ausgangsstoffe benötigten Dihydroxy-pyrazolo-pyrimidine sind durch die Formel (VI) allgemein definiert. In dieser Formel haben R³ und X³ vorzugsweise diejenigen Bedeutungen, die bereits im Zusammenhang mit der Beschreibung der erfindungsgemäßen Stoffe der Formeln (I) und (II) für diese Reste als bevorzugt genannt wurden.

Auch die Dihydroxy-pyrazolopyrimidine der Formel (VI) sind bisher noch nicht bekannt. Sie lassen sich herstellen, indem man
g) Malonester der Formel 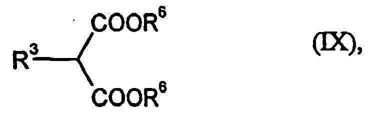 in welcher
   - R³: die oben angegebenen Bedeutung hat und
   - R⁶: für Alkyl steht,
   mit Aminopyrazolen der Formel 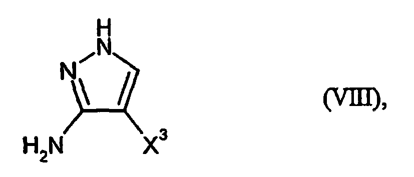 in welcher
   - X³: die oben angegebene Bedeutung hat,
   gegebenenfalls in Gegenwart eines Verdünnungsmittels und gegebenenfalls in Gegenwart einer starken Base umsetzt.

Die bei der Durchführung des erfindungsgemäßen Verfahrens (g) als Ausgangsstoffe benötigten Malonester sind durch die Formel (IX) allgemein definiert. In dieser Formel hat R³ vorzugsweise diejenigen Bedeutungen, die bereits im Zusammenhang mit der Beschreibung der erfindungsgemäßen Stoffe der Formel (I) für diesen Rest als bevorzugt genannt wurden. R⁶ steht vorzugsweise für Alkyl mit 1 bis 4 Kohlenstoffatomen, besonders bevorzugt für Methyl oder Ethyl.

Die Malonester der Formel (IX) sind bekannt oder lassen sich nach bekannten Methoden herstellen (vgl. US-A 6 156 925).

Als Verdünnungsmittel kommen bei der Durchführung der erfindungsgemäßen Verfahren (f) und (g) alle üblichen, inerten organischen Solventien in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Düsopropylether, Methyl-t-butylether, Methyl-t-amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan; Alkohole, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether; Amine, wie Tri-n-butylamin oder Carbonsäuren, wie Essigsäure.

Als starke Basen kommen bei der Durchführung der erfindungsgemäßen Verfahren (f) und (g) vorzugsweise Erdalkalimetall- oder Alkalimetall-hydride oder -alkoholate sowie Alkalimetallamide in Frage. Beispielhaft genannt seien Natriumhydrid, Natriumamid, Natrium-methylat, Natrium-ethylat und Kalium-tert.-butylat.

Bei der Durchführung der erfindungsgemäßen Verfahren (f) und (g) sowie auch bei der Durchführung der anderen erfindungsgemäßen Verfahren arbeitet man im Allgemeinen unter Atmosphärendruck. Es ist aber auch möglich, unter erhöhtem Druck oder, -sofern keine leicht flüchtigen Reaktionskomponenten enthalten sind-, unter vermindertem Druck zu arbeiten.

Die Reaktionstemperaturen können bei der Durchführung der erfindungsgemäßen Verfahren (f) und (g) jeweils innerhalb eines größeren Bereiches variiert werden. Bei Abwesenheit von Basen arbeitet man im Allgemeinen bei Temperaturen zwischen 100°C und 250°C, vorzugsweise zwischen 120°C und 200°C. Bei Anwesenheit von Basen arbeitet man im Allgemeinen bei Temperaturen zwischen 20°C und 120°C, vorzugsweise zwischen 20°C und 80°C.

Bei der Durchführung des erfindungsgemäßen Verfahrens (f) setzt man auf 1 Mol an Acrylsäureester der Formel (VII) im Allgemeinen 1 bis 15 Mol, vorzugsweise 1 bis 8 Mol an Aminopyrazol der Formel (VIII) ein. Die Aufarbeitung erfolgt nach üblichen Methoden.

Bei der Durchführung des erfindungsgemäßen Verfahrens (g) setzt man auf 1 Mol an Malonester der Formel (IX) im Allgemeinen 1 bis 15 Mol, vorzugsweise 1 bis 8 Mol an Aminopyrazol der Formel (VIII) ein. Die Aufarbeitung erfolgt nach üblichen Methoden.

Als Halogenierungsmittel kommen bei der Durchführung der erfindungsgemäßen Verfahren (d) und (e) jeweils alle üblichen Reagenzien in Betracht, die für einen Austausch von an Kohlenstoff gebundene Hydroxygruppen gegen Halogen geeignet sind. Vorzugsweise verwendbar sind Phosphortrichlorid, Phosphortribromid, Phosphorpentachlorid, Phosphoroxychlorid, Phosgen, Thionylchlorid, Thionylbromid oder deren Gemische. Die entsprechenden Fluor-Verbindungen der Formel (II) lassen sich aus den Chlor- oder Brom-Verbindungen durch Umsetzung mit Kaliumfluorid herstellen.

Als Verdünnungsmittel kommen bei der Durchführung der erfindungsgemäßen Verfahren (d) und (e) jeweils alle für derartige Halogenierungen üblichen organischen Solventien in Frage. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan.

Als Verdünnungsmittel kann aber auch das Halogenierungsmittel selbst oder ein Gemisch aus Halogenierungsmittel und einem der genannten Verdünnungsmittel dienen.

Die Reaktionstemperaturen können bei der Durchführung der erfindungsgemäßen Verfahren (d) und (e) jeweils innerhalb eines größeren Bereiches variiert werden. Im Allgemeinen arbeitet man bei Temperaturen zwischen 20°C und 150°C, vorzugsweise zwischen 40°C und 120°C.

Bei der Durchführung der erfindungsgemäßen Verfahrens (d) und (e) setzt man auf 1 Mol an Hydroxypyrazolopyrimidin der Formel (V) bzw. an Dihydroxy-pyrazolo-pyrimidin der Formel (VI) jeweils einen Überschuß an Halogenierungsmittel ein. Die Aufarbeitung erfolgt jeweils nach üblichen Methoden.

Die weiterhin zur Durchführung des erfindungsgemäßen Verfahrens (a) als Ausgangsstoffe benötigten Amine sind durch die Formel (III) allgemein definiert. In dieser Formel haben R¹ und R² vorzugsweise diejenigen Bedeutungen, die bereits im Zusammenhang mit der Beschreibung der erfindungsgemäßen Verbindungen der Formel (I) für R¹ und R² als bevorzugt angegeben wurden.

Die Amine der Formel (III) sind teilweise bekannt.

Neu sind Amine der Formel 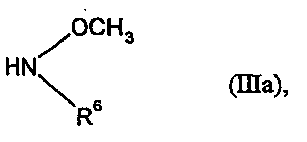 in welcher
- R⁷: für Isobutyl, 2-Methoxyethyl oder für 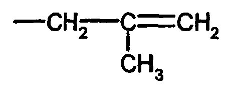
steht

Die Amine der Formel (IIIa) lassen sich herstellen, indem man
h) in einer ersten Stufe N-Methoxycarbaminsäure-ethylester der Formel 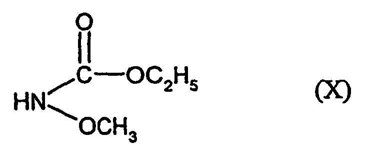 mit Halogenverbindungen der Formel

   R⁷-X⁴ (XI),

   in welcher
   - R⁷: die oben angegebenen Bedeutungen hat und
   - X⁴: für Brom oder Iod steht,
   in Gegenwart einer Base und in Gegenwart eines Verdünnungsmittels umsetzt und die entstehenden Carbamate der Formel 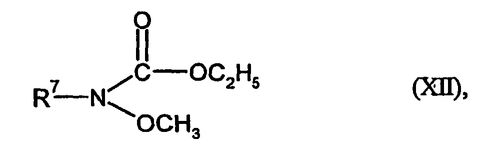 in welcher
   - R⁷: die oben angegebenen Bedeutungen hat,
   in einer zweiten Stufe mit Kaliumhydroxid in Gegenwart von Ethanol und Wasser umsetzt.

Neu sind auch Amine der Formel 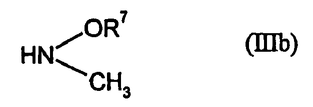 in welcher
- R⁷: die oben angegebenen Bedeutungen hat.

Die Amine der Formel (IIIb) lassen sich herstellen, indem man
i) in einer ersten Stufe N-Hydroxy-N-methyl-carbaminsäure-ethylester der Formel 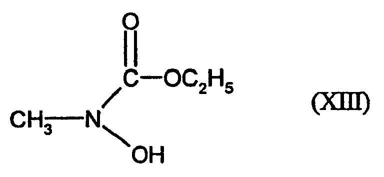 mit Halogenverbindungen der Formel

   R⁷-X⁴ (XI),

   in welcher
   - R⁷ und X⁴: die oben angegebenen Bedeutungen haben,
   in Gegenwart einer Base und in Gegenwart eines Verdünnungsmittels umsetzt und die entstehenden Carbamate der Formel 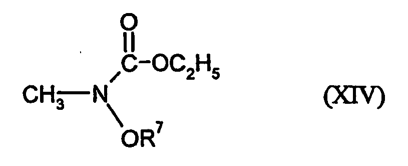 in welcher
   - R⁷: die oben angegebenen Bedeutungen hat,
   in einer zweiten Stufe mit Kaliumhydroxid in Gegenwart von Ethanol und Wasser umsetzt.

Neu sind auch Trifluorisopropylamine der Formel 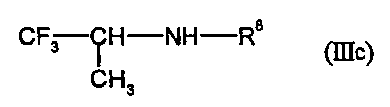 in welcher
- R⁸: für Methyl, Ethyl oder Propyl steht.

Die Trifluorisopropylamine der Formel (IIIc) lassen sich herstellen, indem man
j) in einer ersten Stufe N-Trifluorisopropyl-carbaminsäure-ethylester der Formel 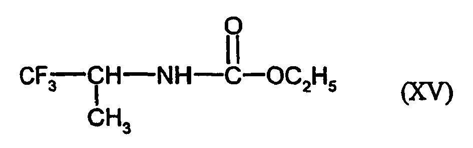 mit Halogenverbindungen der Formel

   R⁸-X⁴ (XVI)

   in welcher
   - R⁸ und X⁴: die oben angegebenen Bedeutungen haben,
   in Gegenwart einer Base und in Gegenwart eines Verdünnungsmittels umsetzt und die entstehenden Carbamate der Formel 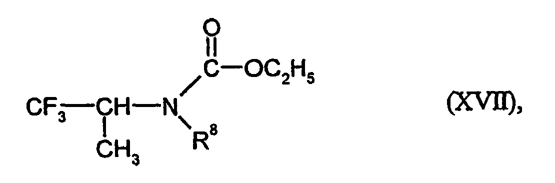 in welcher
   - R⁸: die oben angegebenen Bedeutungen hat,
   in einer zweiten Stufe mit Kaliumhydroxid in Gegenwart von Ethanol und Wasser umsetzt.

Neu ist schließlich auch das 3-Trifluor-methyl-3-amino-propen der Formel

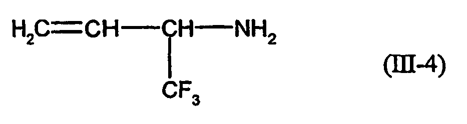

Das 3-Trifluormethyl-3-amino-propen der Formel (III-4) lässt sich herstellen, indem man
k) das Carbamat der Formel 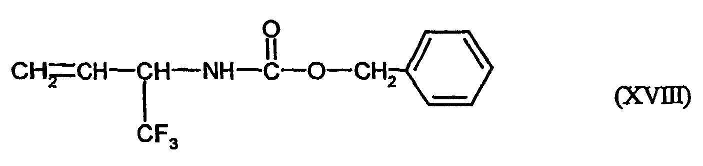 mit wässriger Salzsäure umsetzt.

Die bei der Durchführung der Verfahren (h)-(j) als Ausgangsstoffe benötigten Verbindungen der Formeln (X), (XI), (XIII), (XV), (XVI) und (XVIII) sind bekannt oder lassen sich nach bekannten Methoden herstellen.

Bei der Durchführung der ersten Stufe der erfindungsgemäßen Verfahren (h), (i) und (j) kommen jeweils alle für derartige Umsetzungen üblichen anorganischen und organischen Säureakzeptoren in Frage.

Vorzugsweise verwendbar sind Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat und Natriumhydrogencarbonat, und außerdem Ammonium-Verbindungen, wie Ammoniumhydroxid, Ammoniumacetat und Ammoniumcarbonat.Als organische Basen seien genannt: tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel kommen bei der Durchführung der ersten Stufe der Verfahren (h), (i) und (j) jeweils alle üblichen inerten organischen Solventien in Betracht. Vorzugsweise verwendbar sind Ether, wie Diethylether, Dioxan, Tetrahydrofuran, 1,2-Dimethoxyethan, 1,2-Diethoxyethan oder Anisol; Amide, wie N,N-Dimethylformamid, N,N-Dimethylacetamid, N-Methylformanilid oder N-Methylpyrrolidon; Sulfone, wie Sulfolan; Alkohole wie Methanol, Ethanol, Isopropanol, tert.Butanol, n-Butanol.

Die Reaktionstemperaturen können bei der Durchführung der ersten Stufe der Verfahren (h), (i) und (j) jeweils innerhalb eines größeren Bereiches variiert werden.

Im Allgemeinen arbeitet man bei Temperaturen zwischen 0°C und 150°C, vorzugsweise zwischen 10°C und 100°C.

Bei der Durchführung der ersten Stufe der Verfahren (h), (i) und (j) arbeitet man im allgemeinen jeweils unter Atmosphärendruck. Es ist jedoch auch möglich, unter erhöhtem Druck oder, sofern keine niedrig siedenden Komponenten an der Umsetzung beteiligt sind, unter vermindertem Druck zu arbeiten.

Bei der Durchführung der ersten Stufe der Verfahren (h), (i) und (j) setzt man
- auf 1 mol an N-Methoxy-carbaminsäure-ethylester der Formel (X) im Allgemeinen 0,5 bis 15 mol, vorzugsweise 1 bis 5 mol an Halogenverbindung der Formel (XI) ein, bzw.
- auf 1 mol an N-Hydroxy-N-methyl-carbaminsäure-ethylester der Formel (XIII) im Allgemeinen 0,5 bis 15 mol, vorzugsweise 1 bis 5 mol an Halogenverbindung der Formel (XI) ein, bzw.
- auf 1 mol an N-Trifluorisopropyl-carbaminsäure-ethylester der Formel (XV) im Allgemeinen 0,5 bis 15 mol, vorzugsweise 1 bis 5 mol an Halogenverbindung der Formel (XVI) ein.

Die Aufarbeitung erfolgt jeweils nach üblichen Methoden, beispielsweise durch Extraktion und anschließende Trocknung oder durch Fällung mit anschließender Filtration und Trocknung. Gegebenenfalls noch vorhandene Verunreinigungen können nach üblichen Methoden entfernt werden.

Die bei der Durchführung der ersten Stufe der Verfahren (h), (i) und (j) als Zwischenprodukte erhaltenen Verbindungen der Formeln (XII), (XIV) und (XVII) sind neu.

Auch bei der Durchführung der zweiten Stufe der Verfahren (h), (i) und (j) können die Reaktionstemperaturen jeweils innerhalb eines größeren Bereiches variiert werden. Im Allgemeinen arbeitet man bei Temperaturen zwischen 0°C und 100°C, vorzugsweise zwischen 10°C und 80°C.

Auch bei der Durchführung der zweiten Stufe der Verfahren (h), (i) und (j) arbeitet man im Allgemeinen jeweils unter Atmosphärendruck. Es ist jedoch wiederum möglich, jeweils auch unter erhöhtem Druck oder, sofern die zu isolierenden Produkte keine sehr niedrigen Siedepunkte aufweisen, unter vermindertem Druck zu arbeiten.

Bei der Durchführung der zweiten Stufe der Verfahren (h), (i) und (j) setzt man auf 1 mol an einer Verbindung der Formel (XII), (XIV) oder (XVII) jeweils bis zu 10 mol an Kaliumhydroxid ein. Die Aufarbeitung erfolgt nach üblichen Methoden. Dabei werden die Amine zweckmäßigerweise im Allgemeinen durch Hinzufügen von Säure, vorzugsweise wässriger Salzsäure, in Form ihrer Salze isoliert.

Bei der Durchführung des Verfahrens (k) können die Reaktionstemperaturen ebenfalls in einem größeren Bereich variiert werden. Im Allgemeinen arbeitet man bei Temperaturen zwischen 10°C und 150°C, vorzugsweise bei Rückflusstemperatur.

Im Allgemeinen arbeitet man bei der Durchführung des Verfahrens (k) unter Atmosphärendruck. Es ist aber auch möglich, unter erhöhtem Druck zu arbeiten.

Bei der Durchführung des Verfahrens (k) setzt man auf 1 mol an Carbamat der Formel (XVIII) einem Überschuss, vorzugsweise bis zu 10 mol an wässriger Salzsäure ein. Die Aufarbeitung erfolgt wiederum nach üblichen Methoden.

Als Verdünnungsmittel kommen bei der Durchführung des erfindungsgemäßen Verfahrens (a) alle üblichen, inerten organischen Solventien in Betracht. Vorzugsweise verwendbar sind aliphatische, alicyclische oder aromatische Kohlenwasserstoffe, wie Petrolether, Hexan, Heptan, Cyclohexan, Methylcyclohexan, Benzol, Toluol, Xylol oder Decalin; halogenierte Kohlenwasserstoffe, wie Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Dioxan, Tetrahydrofuran, 1,2- Dimethoxyethan oder 1,2-Diethoxyethan; Amide, wie N,N-Dimethylformamid, N,N-Dimethylacetamid oder N-Methylpyrrolidon; Ester wie Essigsäuremethylester oder Essigsäureethylester; Sulfoxide, wie Dimethylsulfoxid; Sulfone, wie Sulfolan.

Als Katalysatoren kommen bei der Durchführung des erfindungsgemäßen Verfahrens alle für derartige Umsetzungen üblichen Reaktionsbeschleuniger in Frage. Vorzugsweise verwendbar sind Alkalimetallfluoride, wie Kaliumfluorid oder Caesiumfluorid.

Als Säureakzeptoren kommen bei der Durchführung des erfindungsgemäßen Verfahrens (a) alle für derartige Umsetzungen üblichen Säurebindemittel in Frage. Vorzugsweise verwendbar sind Ammoniak sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (a) in einem größeren Bereich variiert werden. Im Allgemeinen arbeitet man bei Temperaturen zwischen 0°C und 150°C, vorzugsweise bei Temperaturen zwischen 0°C und 80°C.

Bei der Durchführung des erfindungsgemäßen Verfahrens (a) setzt man auf 1 mol an Halogen-pyrazolo-pyrimidin der Formel (II) im Allgemeinen 0,5 bis 10 mol, vorzugsweise 0,8 bis 2 mol an Amin der Formel (III) ein. Die Aufarbeitung erfolgt nach üblichen Methoden.

Die bei der Durchführung des erfindungsgemäßen Verfahrens (b) als Ausgangsstoffe benötigten Pyrazolopyrimidine sind durch die Formel (Ia) allgemein definiert. In dieser Formel haben R¹, R², R³ und X¹ vorzugsweise diejenigen Bedeutungen, die bereits im Zusammenhang mit der Beschreibung der erfindungsgemäßen Stoffe der Formel (I) für diese Reste als bevorzugt genannt wurden.

Bei den Pyrazolopyrimidinen der Formel (Ia) handelt es sich um erfindungsgemäße Stoffe, die sich nach dem erfindungsgemäßen Verfahren (a) herstellen lassen.

Als Verdünnungsmittel kommen bei der Durchführung des erfindungsgemäßen Verfahrens (b) alle üblichen inerten, organischen Solventien in Frage. Vorzugsweise verwendbar sind aliphatische oder aromatische, gegebenenfalls halogenierte Kohlenwasserstoffe, wie Toluol, Dichlormethan, Chloroform oder Tetrachlorkohlenstoff.

Die Reaktionstemperaturen können bei der Durchführung des erfindungsgemäßen Verfahrens (b) innerhalb eines bestimmten Bereiches variiert werden. Im Allgemeinen arbeitet man bei Temperaturen zwischen -80°C und +20°C, vorzugsweise zwischen -60°C und +10°C.

Im Allgemeinen arbeitet man bei der Durchführung des erfindungsgemäßen Verfahrens (b) unter Atmosphärendruck. Es ist aber auch möglich, unter erhöhtem Druck zu arbeiten.

Bei der Durchführung des erfindungsgemäßen Verfahrens (b) setzt man auf 1 mol an Pyrazolopyrimidin der Formel (Ia) im Allgemeinen eine äquivalente Menge oder auch einen Überschuss, vorzugsweise 1,1 bis 1,2 mol an Di-isobutyl-aluminiumhydrid ein und fügt anschließend einen Überschuss an wässriger Ammoniumchlorid-Lösung hinzu. Die Aufarbeitung erfolgt nach üblichen Methoden. Im Allgemeinen verfährt man in der Weise, dass man das Reaktionsgemisch ansäuert, die organische Phase abtrennt, die wässrige Phase mit einem mit Wasser wenig mischbaren organischen Solvens extrahiert, die vereinigten organischen Phasen wäscht, trocknet und unter vermindertem Druck einengt.

Die bei der Durchführung des erfindungsgemäßen Verfahrens (c) als Ausgangsstoffe benötigten Pyrazolopyrimidine sind durch die Formel (Ib) allgemein definiert. In dieser Formel haben R¹, R², R³ und X¹ vorzugsweise diejenigen Bedeutungen, die bereits im Zusammenhang mit der Beschreibung der erfindungsgemäßen Stoffe der Formel (I) für diese Reste als bevorzugt genannt wurden.

Bei den Pyrazolopyrimidinen der Formel (Ib) handelt es sich um erfindungsgemäße Stoffe, die sich nach dem erfindungsgemäßen Verfahren (b) herstellen lassen.

Die bei der Durchführung des erfindungsgemäßen Verfahrens (c) als Reaktionskomponenten benötigten Amino-Verbindungen sind durch die Formel (IV) allgemein definiert. In dieser Formel steht R⁴ vorzugsweise für Wasserstoff oder Alkyl mit 1 bis 4 Kohlenstoffatomen, besonders bevorzugt für Wasserstoff, Methyl oder Ethyl.

Als Reaktionskomponenten in Betracht kommen auch Säureadditions-Salze, vorzugsweise Chlorwasserstoff-Additions-Salze von Amino-Verbindungen der Formel (IV).

Sowohl die Amino-Verbindungen der Formel (IV) als auch deren Säureadditions-Salze sind bekannt oder lassen sich nach bekannten Methoden herstellen.

Als Verdünnungsmittel kommen bei der Durchführung des erfindungsgemäßen Verfahrens (c) alle üblichen inerten, organischen Solventien in Frage. Vorzugsweise verwendbar sind Alkohole, wie Methanol, Ethanol, n-Propanol oder Isopropanol.

Als Katalysatoren kommen bei der Durchführung des erfindungsgemäßen Verfahrens (c) alle für derartige Umsetzungen üblichen Reaktionsbeschleuniger in Betracht Vorzugsweise verwendbar sind saure oder basische Katalysatoren, wie z.B. der unter der Bezeichnung Amberlyst A-21^{®} im Handel befindliche, schwach basische Ionenaustaucher.

Die Reaktionstemperaturen können bei der Durchführung des erfindungsgemäßen Verfahrens (c) innerhalb eines bestimmten Bereiches variiert werden. Im Allgemeinen arbeitet man bei Temperaturen zwischen 0°C und 80°C, vorzugsweise zwischen 10°C und 60°C.

Im Allgemeinen arbeitet man bei der Durchführung des erfindungsgemäßen Verfahrens (c) unter Atmosphärendruck. Es ist aber auch möglich, unter erhöhtem Druck zu arbeiten.

Bei der Durchführung des erfindungsgemäßen Verfahrens (c) setzt man auf 1 mol an Pyrazolopyrimidin der Formel (Ib) im Allgemeinen eine äquivalente Menge oder einen Überschuss, vorzugsweise zwischen 1,1 und 1,5 mol an Amino-Verbindung der Formel (IV) oder eines Säureadditions-Salzes davon ein. Die Aufarbeitung erfolgt nach üblichen Methoden. Im Allgemeinen geht man so vor, dass man das Reaktionsgemisch gegebenenfalls filtriert, dann einengt und reinigt.

Zur Herstellung von Säureadditions-Salzen von Pyrazolopyrimidinen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Die erfindungsgemäßen Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie können vorzugsweise als Pflanzenschutzmittel eingesetzt werden. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.

Aus der Ordnung der Diplopoda z.B. Blaniulus guttulatus.

Aus der Ordnung der Chilopoda z.B. Geophilus carpophagus, Scutigera spp..

Aus der Ordnung der Symphyla z.B. Scutigerella immaculata.

Aus der Ordnung der Thysanura z.B. Lepisma saccharina.

Aus der Ordnung der Collembola z.B. Onychiurus armatus.

Aus der Ordnung der Orthoptera z.B. Acheta domesticus, Gryllotalpa spp., Locusta migratoria migratorioides, Melanoplus spp., Schistocerca gregaria.

Aus der Ordnung der Blattaria z.B. Blatta orientalis, Periplaneta americana, Leucophaea maderae, Blattella germanica.

Aus der Ordnung der Dermaptera z.B. Forficula auricularia.

Aus der Ordnung der Isoptera z.B. Reticulitermes spp..

Aus der Ordnung der Phthiraptera z.B. Pediculus humanus corporis, Haematopinus spp., Linognathus spp., Trichodectes spp., Damalinia spp..

Aus der Ordnung der Thysanoptera z.B. Hercinothrips femoralis, Thrips tabaci, Thrips palmi, Frankliniella occidentalis.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus spp., Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.

Aus der Ordnung der Lepidoptera z.B. Pectinophora gossypiella, Bupalus piniarius, Cheimatobia brumata, Lithocolletis blaneardella, Hyponomeuta padella, Plutella xylostella, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Mamestra brassicae, Panolis flammea, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana, Cnaphalocerus spp., Öulema oryzae.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica, Lissorhoptrus oryzophilus.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Hylemyia spp., Liriomyza spp..

Aus der Ordnung der Siphonaptera z.B. Xenopsylla cheopis, Ceratophyllus spp..

Aus der Klasse der Arachnida z.B. Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp., Brevipalpus spp..

Zu den pflanzenparasitären Nematoden gehören z.B. Pratylenchus spp., Radopholus similis, Ditylenchus dipsaci, Tylenchulus semipenetrans, Heterodera spp., Globodera spp., Meloidogyne spp., Aphelenchoides spp., Longidorus spp., Xiphinema spp., Trichodorus spp., Bursaphelenchus spp..

Die Wirkstoffe lassen sich mit besonders gutem Erfolg zur Bekämpfung von pflanzenschädigenden Insekten, wie beispielsweise gegen die Raupen der Kohlschabe (Plutella maculipennis), einsetzen.

Die erfindungsgemäßen Stoffe weisen auch eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen genannt, die unter die oben aufgezählten Oberbegriffe fallen:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder
Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha,
Venturia-Arten, wie beispielsweise Venturia inaequalis;
Pyrenophora Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die erfindungsgemäßen Wirkstoffe weisen auch eine sehr gute stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, daß die behandelten Pflanzen bei nachfolgender Inokolation mit unerwünschten Mikroorgansimen weitgehende Resistenz gegen diese Mirkroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der erfindungsgemäßen Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Getreidekrankheiten, wie beispielsweise gegen Fusarium-Arten, von Krankheiten im Wein-, Obst- und Gemüseanbau, wie beispielsweise gegen Botrytis-, Venturia-, Alternaria-Arten, oder von Reiskrankheiten, wie beispielsweise gegen Pyricularia-Arten, einsetzen.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide sowie zur Beeinflussung des Pflanzenwachstums verwendet werden. Sie lassen sich gegebenenfalls auch als Zwischen- und Vorprodukte für die Synthese weiterer Wirkstoffe einsetzen.

Mit den erfindungsgemäßen Wirkstoffen können Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die Behandlung der Pflanzen und Pflanzenteile mit den erfindungsgemäßen Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt:
Alternaria, wie Alternaria tenuis,
Aspergillus, wie Aspergillus niger,
Chaetomium, wie Chaetomium globosum,
Coniophora, wie Coniophora puetana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im Allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
Fungizide:
   Aldimorph, Ampropylfos, Ampropylfos-Kalium, Andoprim, Anilazin, Azaconazol, Azoxystrobin,
   Benalaxyl, Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat,
   Calciumpolysulfid, Carpropamid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenhexamid, Fenitropan, Fenpiclonil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox, Fluoxastrobin,
   Guazatin,
   Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Iprovalicarb, Irumamycin, Isoprothiolan, Isovaledione,
   Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrotbal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur, Prothioconazole,
   Quinconazol, Quintozen (PCNB), Quinoxyfen
   Schwefel und Schwefel-Zubereitungen, Spiroxamine
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
   Uniconazol,
   Validamycin A, Vinclozolin, Viniconazol,
   Zarilamid, Zineb, Ziram sowie
   Dagger G,
   OK-8705,
   OK-8801,
   α-(1,1-Dimethylethyl)-β-(2-phenoxyethyl)-1H-1,2,4-triazol-1-ethanol,
   α-(2,4-Dichlorphenyl)-β-fluor-β-propyl-1H-1,2,4-triazol-1-ethanol,
   α-(2,4-Dichlorphenyl)-β-methoxy-α-methyl-1H-1,2,4-triazol-1-ethanol,
   α-(5-Methyl-1,3-dioxan-5-yl)-β-[[4-(trifluormethyl)-phenyl]-methylen]-1H-1,2,4-triazol-1-ethanol,
   (5RS,6RS)-6-Hydroxy-2,2,7,7-tetramethyl-5-(1H-1,2,4-triazol-1-yl)-3-octanon,
   (E)-α-(Methoxyimino)-N-methyl-2-phenoxy-phenylacetamid,
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlorphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4-trifluormethyl-1,3-thiazol-5-carboxanilid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-β-D-glycopyranosyl)-α-D-glucopyranosyl]-amino]-4-methoxy-1H-pyrrolo[2,3-d]pyrimidin5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid,
   2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid,
   4-Methyl-tetrazolo[1,5-a]quinazolin-5(4H)-on,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholin-hydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,6-Dirnethylphenyl)-2-methoxy-N-(tetrahydro-2-oxo-3-furanyl)-acetamid,
   N-(2,6-Dimethylphenyl)-2-methoxy-N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin -Natriumsalz,
   O,O-Diethyl-[2-(dipropylamino)-2-oxoethyl]-ethylphosphoramidothioat,
   O-Methyl-S-phenyl-phenylpropylphosphoramidothioate,
   S-Methyl-1,2,3-benzothiadiazol-7-carbothioat,
   spiro[2H]-1-Benzopyran-2,1'(3'H)-isobenzofuran]-3'-on,
   4-[3,4-Dimethoxyphenyl)-3-(4-fluorphenyl)-acryloyl]-morpholin
**Bakterizide:**
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide** / **Akarizide** / **Nematizide:**
   Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
   Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
   Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
   Eflusilanate, Emamectin, Empenthrin, Endosulfan, Entomopfthora spp., Esfenvalerate, Ethiofencarb, Ethion, Ethoprophos, Etofenprox, Etoxazole, Etrimfos, Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
   Granuloseviren
   Halofenozide, HCH, Heptenophos, Hexaflumuron, Hexythiazox, Hydroprene,
   Imidacloprid, Indoxacarb, Isazofos, Isofenphos, Isoxathion, Ivermectin,
   Kempolyederviren
   Lambda-cyhalothrin, Lufenuron
   Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
   Naled, Nitenpyram, Nithiazine, Novaluron
   Omethoat, Oxamyl, Oxydemethon M
   Paecilomyces fumosoroseus, Parathion A, Parathion M, Permethrin, Phenthoat, Phorat, Phosalone, Phosmet, Phosphamidon, Phoxim, Pirimicarb, Pirimiphos A, Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen,
   Quinalphos,
   Ribavirin
   Salithion, Sebufos, Silafluofen, Spinosad, Spirodiclofen, Sulfotep, Sulprofos,
   Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
   Vamidothion, Vaniliprole, Verticillium lecanii
   YI 5302
   Zeta-cypermethrin, Zolaprofos
   (1R-cis)-[5-(Phenylmethyl)-3-furanyl]-methyl-3-[(dihydro-2-oxo-3(2H)-furanyliden)-methyl]-2,2-dimethylcyclopropancarboxylat
   (3-Phenoxyphenyl)-methyl-2,2,3,3-tetramethylcyclopropanecarboxylat
   1-[(2-Chlor-5-thiazolyl)methyl]tetrahydro-3,5-dimethyl-N-nitro-1,3,5-triazin-2(1H)-imin
   2-(2-Chlor-6-fluorphenyl)-4-[4-(1,1-dimethylethyl)phenyl]-4,5-dihydro-oxazol
   2-(Acetlyoxy)-3-dodecyl-1,4-naphthalindion
   2-Chlor-N-[[[4-(1-phenylethoxy)-phenyl]-amino]-carbonyl]-benzamid
   2-Chlor-N-[[[4-(2,2-dichlor-1,1-difluorethoxy)-phenyl]-amino]-carbonyl]-benzamid
   3-Methylphenyl-propylcarbamat
   4-[4-(4-Ethoxyphenyl)-4-methylpentyl]-1-fluor-2-phenoxy-benzol
   4-Chlor-2-(1,1-dimethylethyl)-5-[[2-(2,6-dimethyl-4-phenoxyphenoxy)ethyl]thio]-3(2H)-pyridazinon
   4-Chlor-2-(2-chlor-2-methylpropyl)-5-[(6-iod-3-pyridinyl)methoxy]-3(2H)-pyridazinon
   4-Chlor-5-[(6-chlor-3-pyridinyl)methoxy]-2-(3,4-dichlorphenyl)-3(2H)-pyridazinon
   Bacillus thuringiensis strain EG-2348
   Benzoesäure [2-benzoyl-1-(1,1-dimethylethyl)-hydrazid
   Butansäure 2,2-dimethyl-3-(2,4-dichlorphenyl)-2-oxo-1-oxaspiro[4.5]dec-3-en-4-yl-ester
   [3-[(6-Chlor-3-pyridinyl)methyl]-2-thiazolidinyliden]-cyanamid
   Dihydro-2-(nitromethylen)-2H-1,3-thiazine-3(4H)-carboxaldehyd
   Ethyl-[2-[[1,6-dihydro-6-oxo-1-(phenylmethyl)-4-pyridazinyl]oxy]ethyl]-carbamat
   N-(3,4,4-Trifluor-1-oxo-3-butenyl)-glycin
   N-(4-Chlorphenyl)-3-[4-(difluormethoxy)phenyl]-4,5-dihydro-4-phenyl-1H-pyrazol-1-carboxamid
   N-[(2-Chlor-5-thiazolyl)methyl]-N'-methyl-N"-nitro-guanidin
   N-Methyl-N'-(1-methyl-2-propenyl)-1,2-hydrazindicarbothioamid
   N-Methyl-N'-2-propenyl-1,2-hydrazindicarbothioamid
   O,O-Diethyl-[2-(dipropylamino)-2-oxoethyl]-ethylphosphoramidothioat
   N -Cyanomethyl-4-trifluormethyl-nicotinamid
   3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze ( z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Die erfindungsgemäßen Wirkstoffe können ferner beim Einsatz als Insektizide in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucotn® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Link® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführten Pflanzen können besonders vorteilhaft erfindungsgemäß mit den Verbindungen der allgemeinen Formel I bzw. den erfindungsgemäßen Wirkstoff mischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

Die Erfindung wird durch die folgenden Beispiele veranschaulicht.

### Herstellungsbeispiele

### Beispiele 1 und 2

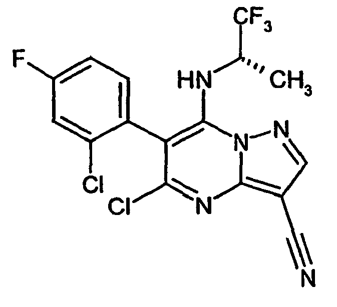

2,5 g (7,3 mMol) 3-Cyano-5,7-dichlor-6-(2-chlor-4-fluorphenyl)-pyrazolo[1,5-a]pyrimidin werden mit 0,425 g (7,3 mMol) Kaliumfluorid in 7,8 g Acetonitril 3 Stunden bei 60°C gerührt. Dann gibt man 3,31 g (29,3 mMol) (S)-1,1,1-Trifluoroprop-2-ylamin dazu und rührt weitere 15 Stunden bei 80°C. Man destilliert das Lösungsmittel unter vermindertem Druck ab und behandelt den Rückstand mit Dichlormethan und 1 N wässriger Salzsäure. Die organische Phase wird abgetrennt, über Natriumsulfat getrocknet, und das Lösungsmittel wird unter vermindertem Druck abdestilliert. Der Rückstand wird mit einer Mischung aus 4 Teilen Cyclohexan und 1 Teil Essigsäureethylester an Kieselgel chromatographiert. Es werden zwei verschiedene Produktfraktionen isoliert (Fraktion 1 und Fraktion 2).

Fraktion 1 (1,2 g) wird nochmals mit einem Gemisch aus 9 Teilen n-Hexan und 1 Teil Aceton an Kieselgel chromatographiert. Man erhält 0,8 g (21 % der Theorie) an 3-Cyano-5-chlor-6-(2-chlor-4-fluorphenyl)-7-(S)-1',1',1'-trifluoroprop-2-ylamino-pyrazolo[1,5-a]pyrimidin als Atropisomeres A (Beispiel 1) (Gehalt: 80,4 %)
HPLC: logP = 3,88 (Isomer AS)
¹H-NMR (DMSO-d6, Tetramethylsilan): δ = 1.37, 1.38 (3H); 4.88, 4.90 (1H); 7.43-7.59 (1H); 7.60-7.66 (1H); 7.72-7.78 (1H); 8.06, 8.08 (1H, NH); 8.83 (1H) ppm.

Die zuletzt isolierte Fraktion 2 enthält 0,9 g (29,3 % der Theorie) 3-Cyano-5-chlor-6-(2-chlor-4-fluorphenyl)-7-(S)-1',1',1'-trifluorprop-2-ylamino-pyrazolo[1,5-a]pyrimidin als Atropisomeres B (Beispiel 2) (Gehalt: 99,3 %)
HPLC: logP = 3,91 (Isomer BS)
¹H-NMR (DMSO-d6, Tetramethylsilan): δ = 1.29, 1.31 (3H); 4.61, 4.63 (1H); 7.42-7.47 (1H); 7.58-7.61 (1H); 7.73-7.76 (1H); 8.10, 8.12 (1H, NH); 8.84 (1H) ppm.

### Beispiel 3

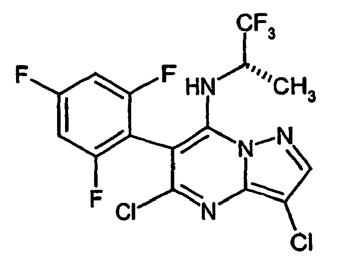

Zu einer Lösung von 0,5 g (1,4 mMol) 3,5,7-Trichlor-6-(2,4,6-trifluorphenyl)pyrazolo[1,5-a]pyrimidin in 12,5 ml Acetonitril gibt man 0,165 g (237,5 mMol) Kaliumfluorid und 0,481 g (4,26 mMol) (S)-1,1,1-Trifluorprop-2-ylamin und rührt 16 Stunden bei 80°C. Nach dem Abkühlen werden 1N Salzsäure und Dichlormethan zugegeben. Das Reaktionsgemisch wird filtriert und das Filtrat wird eingeengt. Der Rückstand wird mit Methyl-t-butylether/Petrolether (1:100) an einer Kieselgelkartusche chromatografiert. Man erhält 0,25 g (40 % der Theorie) an N-[3,5-Dichlor-6-(2,4,6-trifluorphenyl)pyrazolo[1,5-a]pyrimidin-7-yl]-N-[(1S)-2,2,2-trifluor-1-methylethyl]amin.
HPLC; logP = 4,43

### Beispiel 4

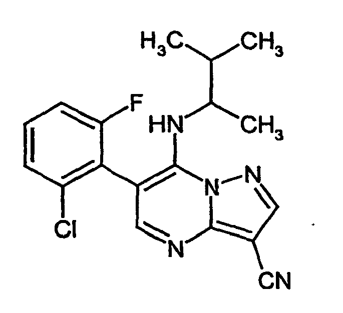

0,1 g (0,33 mMol) 7-Chlor-6-(2-chlor-6-fluorphenyl)pyrazolo[1,5-a]pyrimidin-3-carbonitril und 0,028 g (0,33 mMol) 1,2-Dimethylpropylamin werden in 5 ml Dichlormethan gelöst. Es werden 0,05 ml Triethylamin zugegeben, und das Reaktionsgemisch wird 16 Stunden bei Raumtemperatur gerührt. Die Reaktionsmischung wird mit 1N Salzsäure verrührt, dann filtriert und das Filtrat wird unter vermindertem Druck eingeengt. Der Rückstand wird mit Methyl-t-butylether/Petrolether (1:9) an einer Kieselgelkartusche chromatografiert. Man erhält 0,1 g (89 % der Theorie) an 6-(2-Chlor-6-fluorphenyl)-7-[(1,2-dimethylpropyl)amino]pyrazolo[1,5-a]pyrimidin-3-carbonitril.
HPLC; logP = 3,78

### Beispiel 5

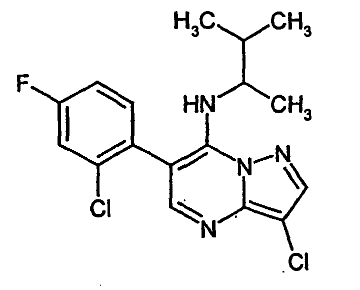

0,1 g (0,316 mMol) 7-Chlor-6-(2-chlor-6-fluorphenyl)pyrazolo[1,5-a]pyrimidin-3-carbonitril und 0,028 g (0,316 mMol) 1,2-Dimethylpropylamin werden in 4 ml Acetonitril gelöst. Es werden 0,044 g (0,316 mMol) Kaliumcarbonat zugegeben, und das Reaktionsgemisch wird 16 Stunden bei 60°C gerührt. Zur Reaktionsmischung gibt man 20 ml Ether und 10 ml 1N Salzsäure. Die organische Phase wird abgetrennt, über Natriumsulfat getrocknet und unter vermindertem Druck eingeengt. Der Rückstand wird mit Methyl-t-butylether/Petrolether (1:30) an einer Kieselgelkartusche chromatografiert. Man erhält 0,08 g (67 % der Theorie) an N-[3-chlor-6-(2-chlor-4-fluorphenyl)pyrazolo[1,5-a]pyrimidin-7-yl]-N-(1,2-dimethylpropyl)amin.
HPLC; logP = 4,53

Nach den zuvor angegebenen Methoden werden auch die in der folgenden Tabelle 1 aufgeführten Verbindungen der Formel 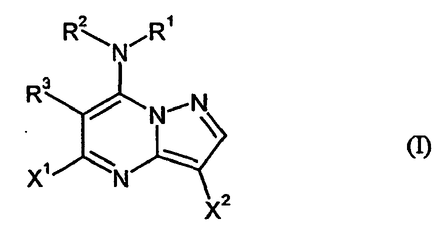 hergestellt.

**Tabelle 1**

| **Bsp. Nr.** | **R¹** | **R²** | **R³** | **X¹** | **X²** | **Isomer* *** | **logP** | **Fp.: (°C)** |
|---|---|---|---|---|---|---|---|---|
| 6 | -CH₂-C(CH₃)=CH₂ | -C₂H₅ | 2,4,6-Trifluorphenyl | -Cl | -CN | | 4,6 | |
| 7 | 2,2,2-Trifluor-1-methylethyl | -H | 2,4,6-Trifluorphenyl | -Cl | -CN | S | 3,69 | |
| 8 | -CH₂-C(CH₃)=CH₂ | -C₂H₅ | 2-Fluorphenyl | -Cl | -CN | | 4,38 | |
| 9 | 2-Methoxyethyl | -C₂H₅ | 2-Fluorphenyl | -Cl | -CN | | 3,52 | |
| 10 | Cyclopentyl | -H | 2-Fluorphenyl | -Cl | -CN | | 3,89 | |
| 11 | Cyclopropylmethyl | -H | 2-Fluorphenyl | -Cl | -CN | | 3,47 | |
| 12 | 2,2,2-Trifluor-1-methytothyl | -H | 2-Chlor-6-fluorphenyl | -Cl | -CN | S | 3,73 | |
| 13 | -CH₂-C(CH₃)=CH₂ | -C₂H₅ | 2-Chlor-6-fluorphenyl | -Cl | -CN | | 4,68 | |
| 14 | -CH₂-CH₂-O-CH₂-CH₂- | * | 2-Chlor-6-fluorphenyl | -Cl | -CN | | 3,26 | |
| 15 | n-Butyl | -C₂H₅ | 2-Chlor-6-fluorphenyl | -Cl | -CN | | 4,92 | |
| 16 | i-Butyl | -H | 2-Chlor-6-fluorphenyl | -Cl | -CN | | 3,94 | |
| 17 | -CH₂-C(CH₃)₃ | -H | 2-Chlor-6-fluorphenyl | -Cl | -CN | | 4,41 | |
| 18 | -CH₂-C(CH₃)=CH₂ | -H | 2-Chlor-6-fluorphenyl | -Cl | -CN | | 3,65 | |
| 19 | -CH₂-CH₂-CH₂-CH₂- | * | 2-Chlor-6-fluorphenyl | -Cl | -CN | | 3,82 | |
| 20 | -C₂H₅ | -C₂H₅ | 2-Chlor-6-fluorphenyl | -Cl | -CN | | 4,13 | |
| 21 | -CH₂-CH₂-CH₂-CH₂-CH₂- | * | 2-Chlor-6-fluorphenyl | -Cl | -CN | | 4,32 | |
| 22 | Cyclopentyl | -H | 2-Chlor-6-fluorphenyl | -Cl | -CN | | 4,13 | |
| 23 | i-Propyl | -H | 2-Chlor-6-fluorphenyl | -Cl | -CN | | 3,65 | |
| 24 | 2-Methoxyethyl | -H | 2-Chlor-6-fluorphenyl | -Cl | -CN | | 3,22 | |
| 25 | Cyclopropyl | -H | 2-Chlor-6-fluorphenyl | -Cl | -CN | | 3,37 | |
| 26 | -CH₂-CH₂-S-CH₂-CH₂- | * | 2-Chlor-6-fluorphenyl | -Cl | -CN | | 3,9 | |
| 27 | -CH₂-CH₂-CH(CF₃)-CH₂-CH₂- | * | 2-Chlor-6-fluorphenyl | -Cl | -CN | | 4,37 | |
| 28 | -CH₂-CH₂-CH(CH₃)-CH₂-CH₂- | * | 2-Chlor-6-fluorphenyl | -Cl | -CN | | 4,77 | |
| 29 | Cyclopropylmethyl | -H | 2-Chlor-6-fluorphenyl | -Cl | -CN | | 3,74 | |
| 30 | 2-Butyl | -H | 2-Chlor-6-fluorphenyl | -Cl | -CN | | 3,94 | |
| 31 | -CH₂-CH₂-CH=CH-CH₂- | * | 2-Chlor-6-fluorphenyl | -Cl | -CN | | 4,08 | |
| 32 | -CH₂-CH₂-CH₂-CH(CH₃)-CH₂- | * | 2-Chlor-6-fluorphenyl | -Cl | -CN | | 4,77 | |
| 33 | -CH₂-CH₂-CH=C(CH₃)-CH₂- | * | 2-Chlor-6-fluorphenyl | -Cl | -CN | | 4,51 | |
| 34 | -CH₂-CH₂-CHF-CH₂CH₂- | * | 2-Chlor-6-fluorphenyl | -Cl | -CN | | 3,82 | |
| 35 | Allyl | -C₂H₅ | 2-Chlor-6-fluorphenyl | -Cl | -CN | | 4,32 | |
| 36 | (2-Furyl)methyl | -C₂H₅ | 2-Chlor-6-fluorphenyl | -Cl | -CN | | 4,32 | |
| 37 | (2-Tetrahydrofuryl)methyl | -C₂H₅ | 2-Chlor-6-fluorphenyl | -Cl | -CN | | 4,08 | |
| 38 | 2-Methoxyethyl | -C₂H₅ | 2-Chlor-6-fluorphenyl | -Cl | -CN | | 3,86 | |
| 39 | -CH₂-COOC₂H₅ | -C₂H₅ | 2-Chlor-6-fluorphenyl | -Cl | -CN | | 3,86 | |
| 40 | Propargyl | -CH₃ | 2-Chlor-6-fluorphenyl | -Cl | -CN | | 3,53 | |
| 41 | -CH₂-COOC₂H₅ | -CH₃ | 2-Chlor-6-fluorphenyl | -Cl | -CN | | 3,57 | |
| 42 | 1,3-Dioxolan-2-ylmethyl | -CH₃ | 2-Chlor-6-fluorphenyl | -Cl | -CN | | 3,49 | |
| 43 | Allyl | -CH₃ | 2-Chlor-6-fluorphenyl | -Cl | -CN | | 4,03 | |
| 44 | (2-Furyl)-methyl | -CH₃ | 2-Chlor-6-fluorphenyl | -Cl | -CN | | 3,99 | |
| 45 | -CH₂-C(CH₃)=CH₂ | -CH₃ | 2-Chlor-6-fluorphenyl | -Cl | -CN | | 4,37 | |
| 46 | i-Butyl | -CH₃ | 2-Chlor-6-fluorphenyl | -Cl | -CN | | 4,51 | |
| 47 | (2-Tetrahydrofuryl)methyl | n-Propyl | 2-Chlor-6-fluorphenyl | -Cl | -CN | | 4,51 | |
| 48 | i-Butyl | -H | 2,4,6-Trifluorphenyl | -Cl | -CN | | 3,85 | |
| 49 | -CH₂-C(CH₃)₃ | -H | 2,4,6-Trifluorphenyl | -Cl | -CN | | 4,26 | |
| 50 | 2-Butyl | -H | 2,4,6-Trifluorphenyl | -Cl | -CN | | 3,89 | |
| 51 | Cyclopentyl | -H | 2,4,6-Trifluorphenyl | -Cl | -CN | | 4,01 | |
| 52 | -i-Propyl | -H | 2,4,6-Trifluorphenyl | -Cl | -CN | | 3,54 | |
| 53 | Cyclopropyl | -H | 2,4,6-Trifluorphenyl | -Cl | -CN | | 3,25 | |
| 54 | Cyclopropylmethyl | -H | 2,4,6-Trifluorphenyl | -Cl | -CN | | 3,63 | |
| 55 | -CH₂-C(CH₃)=CH₂ | -H | 2,4,6-Trifluorphenyl | -Cl | -CN | | 3,54 | |
| 56 | 1,3-Dioxolan-2-ylmethyl | -CH₃ | 2,4,6-Trifluorphenyl | -Cl | -CN | | 3,33 | |
| 57 | 2-Methoxyethyl | -C₂H₅ | 2,4,6-Trifluorphenyl | -Cl | -CN | | 3,74 | |
| 58 | -CH₂-CH₂CH₂-CH₂-CH(CH₃)- | * | 2,4,6-Trifluorphenyl | -Cl | -CN | | 4,5 | |
| 59 | 2-Butyl | -H | 2-Fluorphenyl | -Cl | -CN | | 3,7 | |
| 60 | -CH₂-CH₂-CF₃ | -H | 2-Fluorphenyl | -Cl | -CN | | 3,34 | |
| 61 | n-Propyl | -H | 2-Fluorphenyl | -Cl | -CN | | 3,38 | |
| 62 | i-Propyl | -H | 2-Fluorphenyl | -Cl | -CN | | 3,36 | |
| 63 | Cyclohexyl | -H | 2-Fluorphenyl | -Cl | -CN | | 4,2 | |
| 64 | 1-Cyclohexylethyl | -H | 2-Fluorphenyl | -Cl | -CN | | 4,91 | |
| 65 | 2-Methoxyethyl | -H | 2-Fluorphenyl | -Cl | -CN | | 2,89 | |
| 66 | Cyclopropyl | -H | 2-Fluorphenyl | -Cl | -CN | | 3,11 | |
| 67 | -CH₂-CF₃ | -H | 2-Fluorphenyl | -Cl | -CN | | 3,15 | |
| 68 | -CH₂-C(CH₃)=CH₂ | -H | 2-Fluorphenyl | -Cl | -CN | | 3,39 | |
| 69 | 3-Trifluormethylcyclo-hexyl | -H | 2-Fluorphenyl | -Cl | -CN | | 4,15 | |
| 70 | 2-Trifluormethylcyclohexyl | -H | 2-Fluorphenyl | -Cl | -CN | | 4,26 | |
| 71 | 3,5-bis-Trifluormethylcyclohexyl | -H | 2-Fluorphenyl | -Cl | -CN | | 4,26 | |
| 72 | -C₂H₅ | -C₂H₅ | 2-Fluorphenyl | -Cl | -CN | | 3,8 | |
| 73 | -CH₂-CH₂-O-CH₂-CH₂- | * | 2-Fluorphenyl | -Cl | -CN | | 2,88 | |
| 74 | 2,2,2-Trifluor-1-methylethyl | -H | 2-Fluorphenyl | -Cl | -CN | S | 3,49 | |
| 75 | -CH(CH₃)-CH₂-CH(CH₃)₂ | -H | 2-Fluorphenyl | -Cl | -CN | | | |
| 76 | i-Butyl | -H | 2-Chlorphenyl | -Cl | -CN | | 4 | |
| 77 | -CH₂-C(CH₃)₃ | -H | 2-Chlorphenyl | -Cl | -CN | | 4,47 | |
| 78 | 2-Butyl | -H | 2-Chlorphenyl | -Cl | -CN | | 3,98 | |
| 79 | Cyclopentyl | -H | 2-Chlorphenyl | -Cl | -CN | | 4,19 | |
| 80 | i-Propyl | -H | 2-Chlorphenyl | -Cl | -CN | | 3,64 | |
| 81 | Cyclopropyl | -H | 2-Chlorphenyl | -Cl | -CN | | 3,38 | |
| 82 | Cyclopropylmethyl | -H | 2-Chlorphenyl | -Cl | -CN | | 3,74 | |
| 83 | -CH₂-C(CH₃)=CH₂ | -H | 2-Chlorphenyl | -Cl | -CN | | 3,68 | |
| 84 | -CH(CH₃)-CH₂-CH(CH₃)₂ | -H | 2-Chlorphenyl | -Cl | -CN | | 4,7 | |
| 85 | 1,3-Dioxolan-2-ylmethyl | -CH₃ | 2-Chlorphenyl | -Cl | -CN | | 3,42 | |
| 86 | Allyl | -CH₃ | 2-Chlorphenyl | -Cl | -CN | | 4,03 | |
| 87 | 2-Methoxyethyl | -CH₃ | 2-Chlorphenyl | -Cl | -CN | | 3,5 | |
| 88 | -CH₂-C(CH₃)=CH₂ | -CH₃ | 2-Chlorphenyl | -Cl | -CN | | 4,39 | |
| 89 | -CH₂-C(CH₃)=CH₂ | -C₂H₅ | 2-Chlorphenyl | -Cl | -CN | | 3,68 | |
| 90 | Allyl | -C₂H₅ | 2-Chlorphenyl | -Cl | -CN | | 4,32 | |
| 91 | -CH₂-CH₂-CH₂-CH(CH₃)- | * | 2-Chlorphenyl | -Cl | -CN | | 4,18 | |
| 92 | -CH₂-CH₂-CH₂-CH₂- | * | 2-Chlorphenyl | -Cl | -CN | | 3,82 | |
| 93 | -CH₂-CH₂-CH=CH-CH₂- | * | 2-Chlorphenyl | -Cl | -CN | | 4,1 | |
| 94 | -CH₂-CH₂-CH₂-CH₂-CH(CH₃)- | * | 2-Chlorphenyl | -Cl | -CN | | 4,69 | |
| 95 | -CH₂-CH₂-CH(CH₃)CH₂-CH₂- | * | 2-Chlorphenyl | -Cl | -CN | | 4,78 | |
| 96 | -CH₂-CH₂-CH=C(CH₃)-CH₂- | * | 2-Chlorphenyl | -Cl | -CN | | 4,52 | |
| 97 | -CH₂-CH₂-CH(CF₃)-CH₂-CH₂- | * | 2-Chlorphenyl | -Cl | -CN | | 4,35 | |
| 98 | -CH₂-CH₂-CH₂-CH₂-CH₂- | * | 2-Chlorphenyl | -Cl | -CN | | 4,36 | |
| 99 | -CH₂-CH₂-O-CH₂-CH₂- | * | 2-Chlorphenyl | -Cl | -CN | | 3,17 | |
| 100 | -CH₂-CH₂-S-CH₂-CH₂- | * | 2-Chlorphenyl | -Cl | -CN | | 3,88 | |
| 101 | -CH₂-CH₂-N(CH₃)₂ | -C₂H₅ | 2-Chlor-6-Fluorphenyl | -Cl | -CN | | 1,9 | |
| 102 | -CH(CF₃)-CH₂-CH₂-CH₂- | * | 2-Chlor-6-Fluorphenyl | -Cl | -CN | | 4,18 | |
| 103 | 2,2,2-Trifluor-1-methylethyl | -H | 2-Chlorphenyl | -Cl | -CN | S | 3,79 | |
| 104 | -CH₂-CH₂-CH₂-CH₂-CH₂ | * | 2-Fluorphenyl | -Cl | -CN | | 4,05 | |
| 105 | -CH₂-C(CH₃)=CH₂ | -C₂H₅ | 2,4-Difluorphenyl | -Cl | -CN | | 4,47 | 95-98 |
| 106 | -CH₂-C(CH₃)=CH₂ | -C₂H₅ | 2,4,6-Trifluorphenyl | -Cl | -Cl | | 5,55 | |
| 107 | Allyl | -CH₃ | 2,4,6-Trifluorphenyl | -Cl | -CN | | 3,87 | |
| 108 | i-Butyl | -CH₃ | 2,4,6-Trifluorphenyl | -Cl | -CN | | 4,37 | |
| 109 | 2-Methoxyethyl | -CH₃ | 2,4,6-Trifluorphenyl | -Cl | -CN | | 3,44 | |
| 110 | -CH₂-C(CH₃)=CH₂ | -CH₃ | 2,4,6-Trifluorphenyl | -Cl | -CN | | 4,24 | |
| 111 | Allyl | -C₂H₅ | 2,4,6-Trifluorphenyl | -Cl | -CN | | 4,23 | |
| 112 | -CH₂-CH₂-CH₂-CH(CH₃)- | * | 2,4,6-Trifluorphenyl | -Cl | -CN | | 4,09 | |
| 113 | -CH(CF₃)-CH₂-CH₂-CH₂- | * | 2,4,6-Trifluorphenyl | -Cl | -CN | | 4,12 | |
| 114 | -CH₂-CH₂-CH₂-CH₂- | * | 2,4,6-Trifluorphenyl | -Cl | -CN | | 3,71 | |
| 115 | -CH₂-CH₂-CH=CH-CH₂- | * | 2,4,6-Trifluorphenyl | -Cl | -CN | | 3,96 | |
| 116 | -CH₂-CH₂-CH₂-CH(CH₃)-CH₂- | * | 2,4,6-Trifluorphenyl | -Cl | -CN | | 4,6 | |
| 117 | -CH₂-CH₂-CH(CH₃)-CH₂-CH₂- | * | 2,4,6-Trifluorphenyl | -Cl | -CN | | 4,6 | |
| 118 | -CH₂-CH₂-CH=C(CH₃)-CH₂- | * | 2,4,6-Trifluorphenyl | -Cl | -CN | | 4,34 | |
| 119 | -CH₂-CH₂-CHF-CH₂-CH₂- | * | 2,4,6-Trifluorphenyl | -Cl | -CN | | 3,72 | |
| 120 | -CH₂-CH₂-CH(CF₃)-CH₂-CH₂- | * | 2,4,6-Trifluorphenyl | -Cl | -CN | | 4,26 | |
| 121 | -CH₂-CH₂-CH₂-CH₂-CH₂- | * | 2,4,6-Trifluorphenyl | -Cl | -CN | | 4,23 | |
| 122 | -CH₂-CH₂-O-CH₂-CH₂- | * | 2,4,6-Trifluorphenyl | -Cl | -CN | | 3,16 | |
| 123 | -CH₂-CH₂-S-CH₂-CH₂- | * | 2,4,6-Trifluorphenyl | -Cl | -CN | | 3,79 | |
| 124 | -CH₂-CF₃ | -H | 2,4,6-Trifluorphenyl | -Cl | -CN | | 3,37 | |
| 125 | -C₂H₅ | -C₂H₅ | 2,4-Difluorphenyl | -Cl | -CN | | | |
| 126 | 2,2,2-Trifluor-1-methylethyl | -H | 2,4-Difluorphenyl | -Cl | -CN | S | 3,65 | 123-25 |
| 127 | -C₂H₅ | -H | 2-Fluorphenyl | -Cl | -CN | | 3,06 | |
| 128 | -CH₂-CN | -H | 2-Fluorphenyl | -Cl | -CN | | 2,45 | |
| 129 | -C(CH₃)₂-CF₃ | -H | 2-Fluorphenyl | -Cl | -CN | | 4,01 | |
| 130 | 4-Trifluormethylcyclohexyl | -H | 2-Fluorphenyl | -Cl | -CN | | 4,2 | |
| 131 | -CH₃ | -CH₃ | 2-Fluorphenyl | -Cl | -CN | | 3,12 | |
| 132 | -CH₂-CH₂-CH₂-CH₂- | * | 2-Fluorphenyl | -Cl | -CN | | 3,56 | |
| 133 | -CH₂-CH₂-CH(CF₃)-CH₂-CH₂- | * | 2-Fluorphenyl | -Cl | -CN | | 4,13 | |
| 134 | -CH₂-CH(CH₃)-O-CH(CH₃)-CH₂- | * | 2-Fluorphenyl | -Cl | -CN | | 3,67 | |
| 135 | -CH₂-CH₂-S-CH₂-CH₂- | * | 2-Fluorphenyl | -Cl | -CN | | 3,63 | |
| 136 | 1-Cyclopropylethyl | -H | 2,4,6-Trifluorphenyl | -Cl | -Cl | | 4,66 | |
| 137 | -CH₂-CF₃ | -H | 2-Chlorphenyl | -Cl | -CN | | 3,43 | |
| 138 | i-Butyl | -CH₃ | 2-Chlorphenyl | -Cl | -CN | | 4,51 | |
| 139 | 2-Methoxyethyl | n-Propyl | 2-Chlorphenyl | -Cl | -CN | | 4,23 | |
| 140 | 2-Methoxyethyl | -C₂H₅ | 2-Chlorphenyl | -Cl | -CN | | 4,28 | |
| 141 | -CH(CF₃)-CH₂-CH₂-CH₂- | * | 2-Chlorphenyl | -Cl | -CN | | 4,19 | |
| 142 | -CH₂-CH₂-CH₂-CH(CH₃)-CH₂- | * | 2-Chlorphenyl | -Cl | -CN | | 4,82 | |
| 143 | -CH₂-CH₂-CHF-CH₂-CH₂- | * | 2-Chlorphenyl | -Cl | -CN | | 3,81 | |
| 144 | i-Propyl | -H | 2-Chlor-4-fluorphenyl | -Cl | -CN | | 3,78 | |
| 145 | 2,2,2-Trifluor-1-methylethyl | -H | 2-Chlor-4-fluorphenyl | -Cl | -CN | AS+BR | 3,87 | |
| 146 | 2,2,2-Trifluor-1-methylethyl | -H | 2-Chlor-4-fluorphenyl | -Cl | -CN | AS+BR + BS + AR | 3,92 | |
| 147 | 2,2,2-Trifluor-1-methylethyl | -H | 2-Chlor-4-fluorphenyl | -Cl | -CN | AS+BR | 3,91 | |
| 148 | i-Butyl | -H | 2,4-Difluorphenyl | -Cl | -CN | | 3,87 | |
| 149 | n-Butyl | -H | 2,4-Difluorphenyl | -Cl | -CN | | 3,86 | |
| 150 | -CH₂-C(CH₃)₃ | -H | 2,4-Difluorphenyl | -Cl | -CN | | 4,23 | |
| 151 | 2-Butyl | -H | 2,4-Difluorphenyl | -Cl | -CN | | 3,82 | |
| 152 | -CH₂-CH₂-CF₃ | -H | 2,4-Difluorphenyl | -Cl | -CN | | 3,47 | |
| 153 | n-Propyl | -H | 2,4-Difluorphenyl | -Cl | -CN | | 3,5 | |
| 154 | Cyclopentyl | -H | 2,4-Difluorphenyl | -Cl | -CN | | 3,98 | |
| 155 | -i-Propyl | -H | 2,4-Difluorphenyl | -Cl | -CN | | 3,5 | |
| 156 | Cyclohexyl | -H | 2,4-Difluorphenyl | -Cl | -CN | | 4,26 | |
| 157 | 1-Cyclohexylethyl | -H | 2,4-Difluorphenyl | -Cl | -CN | | 4,96 | |
| 158 | 2-Methoxyethyl | -H | 2,4-Difluorphenyl | -Cl | -CN | | 3,06 | |
| 159 | Cyclopropyl | -H | 2,4-Difluorphenyl | -Cl | -CN | | 3,23 | |
| 160 | Cyclopropylmethyl | -H | 2,4-Difluorphenyl | -Cl | -CN | | 4,35 | |
| 161 | -CH₂-C(CH₃)=CH₂ | -H | 2,4-Difluorphenyl | -Cl | -CN | | 3,51 | |
| 162 | 3-Trifluormethylcyclohexyl | -H | 2,4-Difluorphenyl | -Cl | -CN | | 4,2 | |
| 163 | 2-Trifluormethylcyclohexyl | -H | 2,4-Difluorphenyl | -Cl | -CN | | 4,23 | |
| 164 | 4-Trifluormethylcyclohexyl | -H | 2,4-Difluorphenyl | -Cl | -CN | | 4,21 | |
| 165 | -CH(CH₃)-CH₂-CH(CH₃)₂ | -H | 2,4-Difluorphenyl | -Cl | -CN | | 4,47 | |
| 166 | -CH₂-CH₂-N(CH₃)₂ | -CH₃ | 2,4-Difluorphenyl | -Cl | -CN | | 1,72 | |
| 167 | Propargyl | -CH₃ | 2,4-Difluorphenyl | -Cl | -CN | | 3,35 | |
| 168 | 1,3-Dioxolan-2-ylmethyl | -CH₃ | 2,4-Difluorphenyl | -Cl | -CN | | 3,3 | |
| 169 | -CH₂-CH(OCH₃)₂ | -CH₃ | 2,4-Difluorphenyl | -Cl | -CN | | 3,46 | |
| 170 | -CH₂-C(CH₃)=CH₂ | -CH₃ | 2,4-Difluorphenyl | -Cl | -CN | | 4,16 | |
| 171 | n-Butyl | -CH₃ | 2,4-Difluorphenyl | -Cl | -CN | | 4,36 | |
| 172 | i-Butyl | -H | 2,6-Difluorphenyl | -Cl | -CN | | 3,73 | |
| 173 | -CH₂-C(CH₃)₃ | -H | 2,6-Difluorphenyl | -Cl | -CN | | 4,15 | |
| 174 | 2-Butyl | -H | 2,6-Difluorphenyl | -Cl | -CN | | 3,71 | |
| 175 | -CH₂-CN | -H | 2,6-Difluorphenyl | -Cl | -CN | | 2,49 | |
| 176 | Cyclopentyl | -H | 2,6-Difluorphenyl | -Cl | -CN | | 3,89 | |
| 177 | -i-Propyl | -H | 2,6-Difluorphenyl | -Cl | -CN | | 3,39 | |
| 178 | 2-Methoxyethyl | -H | 2,6-Difluorphenyl | -Cl | -CN | | 2,96 | |
| 179 | Cyclopropyl | -H | 2,6-Difluorphenyl | -Cl | -CN | | 3,13 | |
| 180 | -CH₂-CF₃ | -H | 2,6-Difluorphenyl | -Cl | -CN | | 3,07 | |
| 181 | Cyclopropylmethyl | -H | 2,6-Difluorphenyl | -Cl | -CN | | 3,5 | |
| 182 | -CH₂-C(CH₃)=CH₂ | -H | 2,6-Difluorphenyl | -Cl | -CN | | 3,4 | |
| 183 | -CH(CH₃)-CH₂-CH(CH₃)₂ | -H | 2,6-Difluorphenyl | -Cl | -CN | | 4,39 | |
| 184 | Propargyl | -CH₃ | 2,6-Difluorphenyl | -Cl | -CN | | 3,27 | |
| 185 | -CH₂-COOC₂H₅ | -CH₃ | 2,6-Difluorphenyl | -Cl | -CN | | 3,31 | |
| 186 | 1,3-Dioxolan-2-ylmethyl | -CH₃ | 2,6-Difluorphenyl | -Cl | -CN | | 3,21 | |
| 187 | Allyl | -CH₃ | 2,6-Difluorphenyl | -Cl | -CN | | 3,77 | |
| 188 | i-Butyl | -CH₃ | 2,6-Difluorphenyl | -Cl | -CN | | 4,23 | |
| 189 | 2-Methoxyethyl | -CH₃ | 2,6-Difluorphenyl | -Cl | -CN | | 3,27 | |
| 190 | -CH₂-C(CH₃)=CH₂ | -CH₃ | 2,6-Difluorphenyl | -Cl | -CN | | 4,1 | |
| 191 | Allyl | -C₂H₅ | 2,6-Difluorphenyl | -Cl | -CN | | 4,07 | |
| 192 | (2-Furyl)-methyl | -C₂H₅ | 2,6-Difluorphenyl | -Cl | -CN | | 4,04 | |
| 193 | (2-Tetrahydro-furyl)methyl | -C₂H₅ | 2,6-Difluorphenyl | -Cl | -CN | | 3,84 | |
| 194 | 2-Methoxyethyl | -C₂H₅ | 2,6-Difluorphenyl | -Cl | -CN | | 3,59 | |
| 195 | -CH₂-COOC₂H₅ | -C₂H₅ | 2,6-Difluorphenyl | -Cl | -CN | | 3,61 | |
| 196 | n-Butyl | -C₂H₅ | 2,6-Difluorphenyl | -Cl | -CN | | 4,64 | |
| 197 | -C₂H₅ | -C₂H₅ | 2,6-Difluorphenyl | -Cl | -CN | | 3,88 | |
| 198 | Cyclopropylmethyl | n-Propyl | 2,6-Difluorphenyl | -Cl | -CN | | 4,65 | |
| 199 | (2-Tetrahydrofuryl)methyl | n-Propyl | 2,6-Difluorphenyl | -Cl | -CN | | 4,24 | |
| 200 | 2-Methoxyethyl | n-Propyl | 2,6-Difluorphenyl | -Cl | -CN | | 3,96 | |
| 201 | -CH₂-CH(OH)-CH₂-CH₂- | * | 2,6-Difluorphenyl | -Cl | -CN | | 2,47 | |
| 202 | -CH₂-CH₂-CH₂-CH(CH₃)- | * | 2,6-Difluorphenyl | -Cl | -CN | | 3,92 | |
| 203 | -CH₂-CH₂-CH₂-CH₂- | * | 2,6-Difluorphenyl | -Cl | -CN | | 3,55 | |
| 204 | -CH₂-CH₂-CH₂-CH₂-CH(CH₃)- | * | 2,6-Difluorphenyl | -Cl | -CN | | 4,4 | |
| 205 | -CH₂-CH₂-CH₂-CH(CH₃)-CH₂- | * | 2,6-Difluorphenyl | -Cl | -CN | | 4,46 | |
| 206 | -CH₂-CH₂-CH(CH₃)-CH₂-CH₂- | * | 2,6-Difluorphenyl | -Cl | -CN | | 4,46 | |
| 207 | -CH₂-CH₂-CH=C(CH₃)-CH₂- | * | 2,6-Difluorphenyl | -Cl | -CN | | 4,2 | |
| 208 | -CH₂-CH₂-CH(CF₃)-CH₂-CH₂- | * | 2,6-Difluorphenyl | -Cl | -CN | | 4,13 | |
| 209 | -CH₂-CH₂-CH₂-CH₂-CH₂- | * | 2,6-Difluorphenyl | -Cl | -CN | | 4,07 | |
| 210 | -CH₂-CH₂-S-CH₂-CH₂- | * | 2,6-Difluorphenyl | -Cl | -CN | | 3,65 | |
| 211 | 2-Fluorcyclopropyl | -H | 2,4,6-Trifluorphenyl | -Cl | -CN | | 3,06 | |
| 212 | i-Butyl | -H | 2,4,6-Trifluorphenyl | -Cl | -Cl | | 4,7 | |
| 213 | Allyl | -C₂H₅ | 2,4,6-Trifluorphenyl | -Cl | -Cl | | 5,14 | |
| 214 | 2-Methoxyethyl | -C₂H₅ | 2,4,6-Trifluorphenyl | -Cl | -Cl | | 4,61 | |
| 215 | -CH₂-CH₂-CH₂-CH(CH₃)- | * | 2,4,6-Trifluorphenyl | -Cl | -Cl | | 4,99 | |
| 216 | -CH₂-CH₂-CH₂-CH₂- | * | 2,4,6-Trifluorphenyl | -Cl | -Cl | | 4,56 | |
| 217 | -CH₂-CH₂-CH(CH₃)-CH₂-CH₂- | * | 2,4,6-Trifluorphenyl | -Cl | -Cl | | 5,59 | |
| 218 | -CH₂-CH₂-CH=CH-CH₂- | * | 2,4,6-Trifluorphenyl | -Cl | -Cl | | 4,84 | |
| 219 | -CH₂-CH₂-CH₂-CH(CH₃)-CH₂- | * | 2,4,6-Trifluorphenyl | -Cl | -Cl | | 5,59 | |
| 220 | -CH₂-CH₂-CH₂-CH₂-CH₂- | * | 2,4,6-Trifluorphenyl | -Cl | -Cl | | 5,14 | |
| 221 | -CH₂-CH₂-O-CH₂-CH₂- | * | 2,4,6-Trifluorphenyl | -Cl | -Cl | | 3,94 | |
| 222 | -CH₂-C(CH₃)₃ | -H | 2,4,6-Trifluorphenyl | -Cl | -Cl | | 5,19 | |
| 223 | Cyclopropylmethyl | -H | 2,4,6-Trifluorphenyl | -Cl | -Cl | | 4,41 | |
| 224 | -CH₂-CF₃ | -H | 2,4,6-Trifluorphenyl | -Cl | -Cl | | 4,08 | |
| 225 | -CH₂-C(CH₃)=CH₂ | -H | 2,4,6-Trifluorphenyl | -Cl | -Cl | | 4,32 | |
| 226 | Allyl | -CH₃ | 2,4,6-Trifluorphenyl | -Cl | -Cl | | 4,8 | |
| 227 | i-Butyl | -CH₃ | 2,4,6-Trifluorphenyl | -Cl | -Cl | | 5,31 | |
| 228 | 2-Methoxyethyl | -CH₃ | 2,4,6-Trifluorphenyl | -Cl | -Cl | | 4,23 | |
| 229 | -CH₂-C(CH₃)=CH₂ | -CH₃ | 2,4,6-Trifluorphenyl | -Cl | -Cl | | 5,17 | |
| 230 | -CH₂-CH₂-CF₂-CH₂-CH₂- | * | 2,4,6-Trifluorphenyl | -Cl | -CN | | 3,81 | |
| 231 | -CH₂-CH₂-CF₂-CH₂-CH₂- | * | 2,4,6-Trifluorphenyl | -Cl | -Cl | | 4,61 | |
| 232 | (2,2-Dichlor-cyclopropyl)-methyl | -CH₃ | 2,4,6-Trifluorphenyl | -Cl | -CN | | 4,32 | |
| 233 | (2,2-Dichlor-cyclopropyl)-methyl | -CH₃ | 2,4,6-Trifluorphenyl | -Cl | -Cl | | 5,16 | |
| 234 | 2-Fluorcyclopropyl | -H | 2,4,6-Trifluorphenyl | -Cl | -Cl | | 3,72 | |
| 235 | -C₂H₅ | -H | 2,4-Difluorphenyl | -Cl | -CN | | 3,2 | |
| 236 | -CH₂-CF₃ | -H | 2,4-Difluorphenyl | -Cl | -CN | | 3,34 | |
| 237 | 3,5-bis-Trifluormethylcyclohexyl | -H | 2,4-Difluorphenyl | -Cl | -CN | | 4,41 | |
| 238 | -CH₂-COOC₂H₅ | -CH₃ | 2,4-Difluorphenyl | -Cl | -CN | | 3,49 | |
| 239 | Allyl | -CH₃ | 2,4-Difluorphenyl | -Cl | -CN | | 3,87 | |
| 240 | -CH₂-CH₂-CN | -CH₃ | 2,4-Difluorphenyl | -Cl | -CN | | 2,98 | |
| 241 | -CH₂-CN | -CH₃ | 2,4-Difluorphenyl | -Cl | -CN | | 2,95 | |
| 242 | -CH₂-COOCH₃ | -CH₃ | 2,4-Difluorphenyl | -Cl | -CN | | 3,17 | |
| 243 | (2-Furyl)-methyl | -CH₃ | 2,4-Difluorphenyl | -Cl | -CN | | 3,87 | |
| 244 | i-Butyl | -CH₃ | 2,4-Difluorphenyl | -Cl | -CN | | 4,33 | |
| 245 | -CH₂-CH₂-O-CH=CH₂ | -CH₃ | 2,4-Difluorphenyl | -Cl | -CN | | 2,6 | |
| 246 | 2-Methoxyethyl | -CH₃ | 2,4-Difluorphenyl | -Cl | -CN | | 3,41 | |
| 247 | -CH₃ | -CH₃ | 2,4-Difluorphenyl | -Cl | -CN | | 3,25 | |
| 248 | 1,2-Dimethylpropyl | -H | 2,4,6-Trifluorphenyl | -Cl | -CN | | 4,17 | |
| 249 | 1,2-Dimethylpropyl | -H | 2,4,6-Trifluorphenyl | -Cl | -Cl | | 5,02 | |
| 250 | 1,2-Dimethylpropyl | -H | 2,4,6-Trifluorphenyl | -Cl | -Cl | | 5,02 | |
| 251 | 1,2-Dimethylpropyl | -H | 2,4,6-Trifluorphenyl | -Cl | -Cl | | 5,02 | |
| 252 | 1,2-Dimethylpropyl | -H | 2,4,6-Trifluorphenyl | -Cl | -CN | | 4,16 | |
| 253 | 1,2-Dimethylpropyl | -H | 2,4,6-Trifluorphenyl | -Cl | -CN | | 4,16 | |
| 254 | -O-CH₂-CH₂-CH₂-CH₂- | * | 2,4-Difluorphenyl | -Cl | -CN | | 3,71 | |
| 255 | -O-CH₂-CH₂-CH₂-CH₂- | * | 2-Chlor-4-fluorphenyl | -Cl | -CN | | 4,02 | |
| 256 | -O-CH₂-CH₂-CH₂-CH₂- | * | 2-Chlor-6-fluorphenyl | -Cl | -CN | | 3,85 | |
| 257 | 1,2-Dimethylpropyl | -H | 2-Chlor-4-fluorphenyl | -Cl | -CN | AS + BR | 4,43 | |
| 258 | 1,2-Dimethylpropyl | -H | 2-Chlor-4-fluorphenyl | -Cl | -CN | AR+BS | 4,48 | |
| 259 | i-Butyl | -H | 2-Chlor-4-fluorphenyl | -Cl | -CN | | 4,18 | |
| 260 | -CH₂-C(CH₃)₃ | -H | 2-Chlor-4-fluorphenyl | -Cl | -CN | | 4,61 | |
| 261 | 2-Butyl | -H | 2-Chlor-4-fluorphenyl | -Cl | -CN | | 4,18 | |
| 262 | Cyclopentyl | -H | 2-Chlor-4-fluorphenyl | -Cl | -CN | | 4,32 | |
| 263 | 2-Methoxyethyl | -H | 2-Chlor-4-fluorphenyl | -Cl | -CN | | 3,33 | |
| 264 | Cyclopropylmethyl | -H | 2-Chlor-4-fluorphenyl | -Cl | -CN | | 3,9 | |
| 265 | -CH₂-C(CH₃)=CH₂ | -H | 2-Chlor-4-fluorphenyl | -Cl | -CN | | 3,85 | |
| 266 | i-Butyl | -CH₃ | 2-Chlor-4-fluorphenyl | -Cl | -CN | | 4,67 | |
| 267 | 2-Methoxyethyl | -CH₃ | 2-Chlor-4-fluorphenyl | -Cl | -CN | | 3,72 | |
| 268 | -CH₂-C(CH₃)=CH₂ | -CH₃ | 2-Chlor-4-fluorphenyl | -Cl | -CN | | 4,56 | |
| 269 | -CH₂-C(CH₃)=CH₂ | -C₂H₅ | 2-Chlor-4-fluorphenyl | -Cl | -CN | | 4,87 | |
| 270 | 2-Methoxyethyl | -C₂H₅ | 2-Chlor-4-fluorphenyl | -Cl | -CN | | 3,99 | |
| 271 | -CH₂-CH₂-CH₂-CH(CH₃)- | * | 2-Chlor-4-fluorphenyl | -Cl | -CN | | 4,32 | |
| 272 | -CH₂-CH₂-CH₂-CH₂- | * | 2-Chlor-4-fluorphenyl | -Cl | -CN | | 3,99 | |
| 273 | -CH₂-CH₂CH(CH₃)-CH₂-CH₂- | * | 2-Chlor-4-fluorphenyl | -Cl | -CN | | 4,92 | |
| 274 | -CH₂-CH₂-CH₂-CH₂-CH₂- | * | 2-Chlor-4-fluorphenyl | -Cl | -CN | | 4,51 | |
| 275 | -CH₂-CH₂-O-CH₂-CH₂- | * | 2-Chlor-4-fluorphenyl | -Cl | -CN | | 3,33 | |
| 276 | -CH₂-CF₃ | -H | 2-Chlor-4-fluorphenyl | -Cl | -CN | | | |
| 277 | -CH(CF₃)-CH₂-CH₂-CH₂- | * | 2-chlor-4-fluorphenyl | -Cl | -CN | | | |
| 278 | 1,2-Dimethylpropyl | -H | 2-Chlor-6-fluorphenyl | -H | -Cl | | 4,43 | |
| 279 | -CH₂-C(CH₃)=CH₂ | -C₂H₅ | 2-Chlor-4-fluorphenyl | -H | -Cl | | 5,14 | |
| 280 | -NH-CH₂-CH₂-CH₂-CH₂- | * | 2-Chlor-4-fluorphenyl | -H | -Cl | | 3,57 | |
| 281 | -NH-CH₂-CH₂-CH₂-CH₂- | * | 2-Chlor-6-fluorphenyl | -H | -Cl | | 3,6 | |
| 282 | -CH₂-CH₂-CH(COCH₃)-CH₂-CH₂- | * | 2,4-Difluorphenyl | -Cl | -CN | | 3,31 | |
| 283 | -CH₂-CH=C(C₂H₅)-CH₂-CH₂- | * | 2,4-Difluorphenyl | -Cl | -CN | | 4,76 | |
| 284 | -CH₂-CH₂-CH=C(CH₃)CH₂- | * | 2,4-Difluorphenyl | -Cl | -CN | | 4,33 | |
| 285 | -CH₂-CH₂-CH(COOCH₃)-CH₂-CH₂- | * | 2,4-Difluorphenyl | -Cl | -CN | | 3,61 | |
| 286 | -CH₂-CH₂-CHBr-CH₂-CH₂- | * | 2,4-Difluorphenyl | -Cl | -CN | | 4,21 | |
| 287 | -CH(COOCH₃)-CH₂-CH₂-CH₂-CH₂- | * | 2,4-Difluorphenyl | -Cl | -CN | | 3,85 | |
| 288 | -CH₂-CH₂-CHF-CH₂-CH₂- | * | 2,4-Difluorphenyl | -Cl | -CN | | 3,66 | |
| 289 | 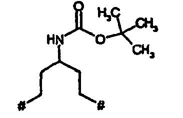 | * | 2,4-Difluorphenyl | -Cl | -CN | | 4 | |
| 290 | -CH₂-CH₂-CH(CF₃)-CH₂-CH₂- | * | 2,4-Difluorphenyl | -Cl | -CN | | 4,2 | |
| 291 | 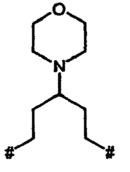 | * | 2,4-Difluorphenyl | -Cl | -CN | | 1,74 | |
| 292 | -CH₂-CH₂-CH(NH-COCH₃)-CH₂-CH₂- | * | 2,4-Difluorphenyl | -Cl | -CN | | 2,51 | |
| 293 | -CH₂-CH₂-N(CH₃)-CH₂-CH₂- | * | 2,4-Difluorphenyl | -Cl | -CN | | 1,47 | |
| 294 | -CH₂-CH(CH₃)-O-CH(CH₃)-CH₂- | * | 2,4-Difluorphenyl | -Cl | -CN | | 3,77 | |
| 295 | -CH₂-CH₂-CH₂-CH₂-CH₂- | * | 2,4-Difluorphenyl | -Cl | -CN | | 4,18 | |
| 296 | -CH₂-CH₂-S-CH₂-CH₂- | * | 2,4-Difluorphenyl | -Cl | -CN | | 3,73 | |
| 297 | 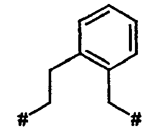 | * | 2,4-Difluorphenyl | -Cl | -CN | | 4,38 | |
| 298 | 1,2-Dimethylpropyl | -H | 2,6-Difluorphenyl | -Cl | -CN | | 4,02 | |
| 299 | -CH₂-CHF₂ | -H | 2-Chlor-6-fluorphenyl | -H | -Cl | | 3,09 | |
| 300 | 2-Methoxyethyl | n-Propyl | 2,4,6-Trifluorphenyl | -Cl | -CN | | 4,1 | |
| 301 | 2,2,2-Trifluor-1-methylethyl | -H | 2,6-Difluorphenyl | -Cl | -CN | R | 3,47 | |
| 302 | 1,2-Dimethylpropyl | -H | 2-Chlor-4-fluorphenyl | -Cl | -CN | BR | 4,44 | |
| 303 | 1,2-Di-methylpropyl | -H | 2-Chlor-4-fluorphenyl | -Cl | -CN | AR | 4,47 | |
| 304 | 1,2-Dimethylpropyl | -H | 2-Chlor-4-fluorphenyl | -Cl | -CN | AR+BR | 4,45 | |
| 305 | 1,2-Dimethylpropyl | -H | 2-Chlor-4-fluorphenyl | -Cl | -CN | AS | 4,46 | |
| 306 | 1,2-Dimethylpropyl | -H | 2-Chlor-4-fluorphenyl | -Cl | -CN | BS | 4,46 | |
| 307 | 1,2-Dimethylpropyl | -H | 2-Chlor-4-fluorphenyl | -Cl | -CN | AS + BS | 4,45 | |
| 308 | -CH₂-CH₂-N(CH₃)₂ | -C₂H₅ | 2,4-Difluorphenyl | -Cl | -CN | | 1,83 | |
| 309 | Allyl | -C₂H₅ | 2,4-Difluorphenyl | -Cl | -CN | | 4,18 | |
| 310 | (2-Furyl)methyl | -C₂H₅ | 2,4-Difluorphenyl | -Cl | -CN | | 4,18 | |
| 311 | (2-Tetrahydrofuryl)methyl | -C₂H₅ | 2,4-Difluorphenyl | -Cl | -CN | | 4,02 | |
| 312 | -CH₂-CH₂-CN | -C₂H₅ | 2,4-Difluorphenyl | -Cl | -CN | | 3,24 | |
| 313 | 2-Methoxyethyl | -C₂H₅ | 2,4-Difluorphenyl | -Cl | -CN | | 3,74 | |
| 314 | -CH₂-COOC₂H₅ | -C₂H₅ | 2,4-Difluorphenyl | -Cl | -CN | | 3,81 | |
| 315 | 3-Aminopropyl | n-Propyl | 2,4-Difluorphenyl | -Cl | -CN | | 1,75 | |
| 316 | (2-Tetrahydrofuryl)methyl | n-Propyl | 2,4-Difluorphenyl | -Cl | -CN | | 4,45 | |
| 317 | 2-Thienylmethyl | n-Propyl | 2,4-Difluorphenyl | -Cl | -CN | | 4,8 | |
| 318 | 2-Methoxyethyl | n-Propyl | 2,4-Difluorphenyl | -Cl | -CN | | 4,13 | |
| 319 | -CH₂-CH₂-NH₂ | -i-Propyl | 2,4-Difluorphenyl | -Cl | -CN | | 1,72 | |
| 320 | -CH₂-COOC₂H₅ | Cyclopropyl | 2,4-Difluorphenyl | -Cl | -CN | | 3,99 | |
| 321 | -CH₂-CH(OH)-CH₂-CH₂- | * | 2,4-Difluorphenyl | -Cl | -CN | | 2,57 | |
| 322 | -CH₂-CH₂-CH₂-CH(CH₃)- | * | 2,4-Difluorphenyl | -Cl | -CN | | 4,05 | |
| 323 | -CH₂-CH₂-CH₂-CH₂- | * | 2,4-Difluorphenyl | -Cl | -CN | | 3,7 | |
| 324 | -CH₂-CH₂-CH(OH)-CH₂-CH₂- | * | 2,4-Difluorphenyl | -Cl | -CN | | 2,63 | |
| 325 | 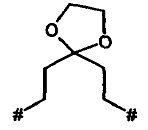 | * | 2,4-Difluorphenyl | -Cl | -CN | | 3,51 | |
| 326 | -CH₂-CH(CH₃)-CH₂-CH(CH₃)-CH₂- | * | 2,4-Difluorphenyl | -Cl | -CN | | 4,98 | |
| 327 | -CH₂-CH₂-CH₂-CH₂-CH(CH₃)- | * | 2,4-Difluorphenyl | -Cl | -CN | | 4,49 | |
| 328 | -CH₂-CH₂-CH₂-CH(CH₃)-CH₂- | * | 2,4-Difluorphenyl | -Cl | -CN | | 4,59 | |
| 329 | -CH₂-CH₂-CH(CH₃)CH₂-CH₂- | * | 2,4-Difluorphenyl | -Cl | -CN | | 4,59 | |
| 330 | -CH₂-CH(OH)-CH₂-CH₂-CH₂- | * | 2,4-Difluorphenyl | -Cl | -CN | | 2,83 | |
| 331 | -CH₂-CH₂-C(CH₃)₂-CH₂-CH₂- | * | 2,4-Difluorphenyl | -Cl | -CN | | 4,83 | |
| 332 | -CH₂-CH₂-S-CH₂-CH₂- | * | 2,4,6-Trifluorphenyl | -Cl | -Cl | | 4,64 | |
| 333 | 1,2-Dimethylpropyl | -H | 2-Chlorphenyl | -Cl | -Cl | B | 5,17 | |
| 334 | 1,2-Dimethylpropyl | -H | 2-Chlorphenyl | -Cl | -Cl | A | 5,18 | |
| 335 | i-Butyl | -CH₃ | 2-Chlorphenyl | -Cl | -Cl | | 5,38 | |
| 336 | 1,2-Dimethylpropyl | -H | 2-Chlor-6-fluorphenyl | -Cl | -CN | | 4,25 | |
| 337 | 1,2-Dimethylpropyl | -H | 2-Chlor-6-fluorphenyl | -Cl | -CN | | 4,26 | |
| 338 | -CH₂-CH₂-O-CH₂-CH₂- | * | 2-Chlorphenyl | -Cl | -Cl | | 3,86 | |
| 339 | -CH₂-CH₂-S-CH₂-CH₂ | * | 2-Chlorphenyl | -Cl | -Cl | | 4,67 | |
| 340 | -CH₂-CH₂-CF₂-CH₂-CH₂ | * | 2-Chlorphenyl | -Cl | -Cl | | 4,67 | |
| 341 | 2,2,2-Trifluor-1-methylethyl | -H | 2-Chlorphenyl | -Cl | -Cl | B | 4,48 | |
| 342 | 2,2,2-Trifluor-1-methylethyl | -H | 2-Chlorphenyl | -Cl | -Cl | A | 4,52 | |
| 343 | -CH₂-CH₂-O-CH₂-CH₂- | * | 2-Chlor-4-fluorphenyl | -Cl | -Cl | | 4,01 | |
| 344 | -CH₂-CH₂-S-CH₂-CH₂ | * | 2-Chlor-4-fluorphenyl | -Cl | -Cl | | 4,79 | |
| 345 | -CH₂-CH₂-CF₂-CH₂-CH₂- | * | 2-Chlor-4-fluorphenyl | -Cl | -Cl | | 4,79 | |
| 346 | Cyclopropylmethyl | -H | 2-Chlor-4-fluorphenyl | -Cl | -Cl | | 4,57 | |
| 347 | -CH₂-CF₃ | -H | 2-Chlor-4-fluorphenyl | -Cl | -Cl | | 4,2 | |
| 348 | i-Butyl | -CH₃ | 2-Trifluormethylphenyl | -Cl | -CN | | 4,48 | |
| 349 | -CH₂-C(CH₃)₃ | -H | 2-Trifluormethylphenyl | -Cl | -CN | | 4,43 | |
| 350 | -CH₂-CH₂-CH(CH₃)-CH₂-CH₂ | * | 2-Trifluormethylphenyl | -Cl | -CN | | 4,71 | |
| 351 | 2,2,2-Trifluor-1-methylethyl | -H | 2-Chlor-4-fluorphenyl | -Cl | -Cl | B | 4,63 | |
| 352 | 2,2,2-Trifluor-1-methylethyl | -H | 2-Chlor-4-fluorphenyl | -Cl | -Cl | A | 4,62 | |
| 353 | 1,2-Dimethylpropyl | -H | 2-Chlor-4-fluorphenyl | -Cl | -Cl | B | 5,26 | |
| 354 | 1,2-Dimethylpropyl | -H | 2-Chlor-4-fluorphenyl | -Cl | -Cl | A | 5,25 | |
| 355 | -NH-CH₂-CH₂-CH₂-CH₂- | * | 2-Chlor-6-fluorphenyl | -Cl | -CN | | 3,66 | Paste |
| 356 | -NH₂ | i-Butyl | 2-Chlor-6-fluorphenyl | -Cl | -CN | | 3,9 | Öl |
| 357 | -CH₂-CF₃ | -H | 2-Trifluormethylphenyl | -Cl | -CN | | 3,44 | |
| 358 | 1,2-Dimethylpropyl | -H | 2-Trifluormethylphenyl | -Cl | -Cl | A | 5,04 | |
| 359 | 1,2-Dimethylpropyl | -H | 2-Trifluormethylphenyl | -Cl | -Cl | B | 5,05 | |
| 360 | -CH₂-C(CH₃)₃ | -H | 2-Chlorphenyl | -Cl | -Cl | | 5,34 | |
| 361 | -CH₂-C(CH₃)₃ | -H | 2-Chlor-4-fluorphenyl | -Cl | -Cl | | 5,4 | |
| 362 | -CH₂-CH₂-S-CH₂-CH₂- | * | 2-Chlor-6-fluorphenyl | -Cl | -Cl | | 4,79 | |
| 363 | -CH₂-CH₂-S-CH₂-CH₂- | * | 2-Trifluormethylphenyl | -Cl | -Cl | | 4,68 | |
| 364 | -NH-CH₂-CH₂-CH₂-CH₂- | * | 2-Trifluormethylphenyl | -Cl | -Cl | | 4,41 | |
| 365 | -CH₂-CH₂-O-CH₂-CH2- | * | 2-Trifluormethylphenyl | -Cl | -Cl | | 3,9 | |
| 366 | 2,2,2-Trifluor-1-methylethyl | -H | 2-Trifluormethylphenyl | -Cl | -Cl | B | 4,49 | |
| 367 | 2,2,2-Trifluor-1-methylethyl | -H | 2-Trifluormethylphenyl | -Cl | -Cl | A | 4,46 | |
| 368 | -NH-CH₂-H₂-CH₂-CH₂- | * | 2-Chlor-4-fluorphenyl | -Cl | -CN | | 3,78 | 209-11 |
| 369 | 2,2,2-Trifluor-1-methylethyl | -H | 2-Trifluonnethylphenyl | -Cl | -CN | A | 3,75 | |
| 370 | 2,2,2-Trifluor-1-methylethyl | -H | 2-Trifluormethylphenyl | -Cl | -CN | B | 3,79 | |
| 371 | 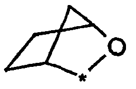 | * | 2-Trifluormethylphenyl | -Cl | -Cl | | 4,17 | |
| 372 | -NH₂ | i-Butyl | 2-Chlor-4-fluorphenyl | -Cl | -CN | | 4,09 | Paste |
| 373 | -CH₂-CH₂-CH(CH₃)-CH₂-CH₂- | * | 2-Chlor-6-fluorphenyl | -Cl | -Cl | | 5,77 | |
| 374 | -NH-CH₂-CH₂-CH₂-CH₂ | * | 2-Chlor-6-fluorphenyl | -Cl | -Cl | | 4,4 | |
| 375 | -NH₂ | i-Butyl | 2-Chlor-6-fluorphenyl | -H | -Cl | | 4,11 | Öl |
| 376 | 1,2-Dimethylpropyl | -H | 2-Trifluormethylphenyl | -Cl | -CN | | 4,26 | |
| 377 | -NH₂ | i-Butyl | 2-Trifluormethylphenyl | -Cl | -CN | | 3,96 | 128-30 |
| 378 | 2,2,2-Trifluor-1-methylethyl | -H | 2-Trifluormethylphenyl | -Cl | -CN | A | 3,79 | |
| 379 | 2,2,2-Trifluor-1-methylethyl | -H | 2-Trifluormethylphenyl | -Cl | -CN | B | 3,79 | |
| 380 | 2,2,2-Trifluor-1-methylethyl | -H | 2,4,6-Trifluorphenyl | -Cl | -Cl | | 4,42 | |
| 381 | -CH₂-CH₂-CH₂-NH- | * | 2-Chlor-6-fluorphenyl | -Cl | -CN | | 3,23 | |
| 382 | -CH(CH₃)-C(CH₃)₃ | -H | 2-Chlor-4-fluorphenyl | -Cl | -CN | | 4,78 | 180-5 |
| 383 | -CH(CH₃)-C(CH₃)₃ | -H | 2-Chlor-6-flfuorphenyl | -Cl | -CN | | 4,6 | |
| 384 | -CH(CH₃)-C(CH₃)₃ | -H | 2,4,6-Trifluorphenyl | -Cl | -CN | | 4,51 | |
| 385 | -CH₂-CH₂-O-CH₂-CH₂ | * | 2-Chlor-5-nitrophenyl | -Cl | -NO₂ | | 2,91 | |
| 386 | 1,2,2-Trimethylpropyl | -H | 2-Chlor-6-fluorphenyl | -Cl | -CN | | 4,59 | 146-8 |
| 387 | 1,2,2-Trimethylpropyl | -H | 2-Chlor-6-fluorphenyl | -Cl | -CN | | 4,66 | 145-8 |
| 388 | -CH(CH₃-C(CH₃)₃ | -H | 2-Trifluormethylphenyl | -Cl | -CN | | 4,57 | |
| 389 | -CH(CH₃)-C(CH₃)₃ | -H | 2-Chlor-4-fluorphenyl | -Cl | -Cl | | 5,63 | |
| 390 | 1,2,2-Trimethylpropyl | -H | 2-Chlor-4-fluorphenyl | -Cl | -CN | | 4,8 | Paste |
| 391 | 1,2,2-Trimethylpropyl | -H | 2-Chlor-4-fluorphenyl | -Cl | -CN | | 4,83 | Paste |
| 392 | -CH₂-CH₂-O-CH₂-CH₂- | * | 2-Chlorphenyl | -Cl | -Br | | 3,97 | |
| 393 | -CH(CH₃)-C(CH₃)₃ | -H | 2-Chlor-4-fluorphenyl | -Cl | -CSNH₂ | | 4,74 | Paste |
| 394 | 1,2,2-Trimethylpropyl | -H | 2,4,6-Trifluorphenyl | -Cl | -CN | | 4,55 | |
| 395 | i-Butyl | -H | 2-Chlor-4-methoxyphenyl | -Cl | -CN | | 4,01 | |
| 396 | -CH(CH₃)-C(CH₃)₃ | -H | 2-Chlor-4-methoxyphenyl | -Cl | -CN | | 4,67 | |
| 397 | -CH₂-C(CH₃)₃ | -H | 2-Chlor-4-methoxyphenyl | -Cl | -CN | | 4,45 | |
| 398 | 1,2-Dimethylpropyl | -H | 2-Chlorphenyl | -Cl | -Br | | 5,31 | |
| 399 | -CH₂-CH₂-O-CH₂-CH₂ | * | 2-Chlorphenyl | -Cl | I | | 4,13 | |
| 400 | 1,2-Dimethylpropyl | -H | 2-Chlorphenyl | -Cl | I | | 5,43 | |
| 401 | -CH(CH₃)-C(CH₃)₃ | -H | 2-Chlor-4-fluorphenyl | -Cl | -CHO | | 4,43 | |
| 402 | -CH(CH₃)-C(CH₃)₃ | -H | 2-Chlor-4-fluorphenyl | -Cl | -CH=N-O-CH₃ | A + B | 5,48 | Paste |
| 403 | -CH(CH₃)-C(CH₃)₃ | -H | 2-Chlor-4-fluorphenyl | -Cl | -CH=N-O-CH₃ | A | 5,5 | Paste |
| 404 | -CH(CH₃)-C(CH₃)₃ | -H | 2-Chlor-4-fluorphenyl | -Cl | -CH=N-O-CH₃ | B | 5,57 | Paste |
| 405 | 1,2-Dimethylpropyl | -H | 2-Chlor-4-methoxyphenyl | -Cl | -CN | | 4,33 | |
| 406 | -CH₂-CH₂-CH₂-CH₂-CH(CH₃)- | * | 2-Chlor-4-methoxyphenyl | -Cl | -CN | | 4,65 | |
| 407 | 1,2,2-Trimethylpropyl | -H | 2,4,6-Trifluorphenyl | -Cl | Cyclopropyl | | 5,88 | |
| 408 | -CH₂-CF₃ | -H | 2-Chlor-4-methylphenyl | -Cl | -CN | | 3,47 | |
| 409 | 1,2-Dimethylpropyl | -H | 2-Chlorphenyl | -Cl | -CHO | | 3,98 | |
| 410 | i-Butyl | -CH₃ | 2-Chlor-4-methoxypyhenyl | -Cl | -CN | | 4,5 | |
| 411 | -CH₂-CH₂-O-CH₂-CH₂- | * | 2-Chlor-4-methoxypyhenyl | -Cl | -CN | | 3,26 | |
| 412 | -CH₂-CH₂-CH₂-CH₂-CH(CH₃)- | * | 2-Chlor-4-methoxypyhenyl | -Cl | -CN | | 4,18 | |
| 413 | -CH₂-CH₂-CH(CN)-CH₂-CH₂ | * | 2-Chlor-4-fluorphenyl | -Cl | -Cl | | 4,06 | |
| 414 | 1,2-Dimethylpropyl | -H | 2,4,6-Trifluorphenyl | -Cl | Cyclopropyl | | 5,5 | |
| 415 | -CH(CH₃-C(CH₃)₃ | -H | 2-Chlor-6-fluorphenyl | -Cl | -CSNH₂ | | 4,52 | |
| 416 | 1,2-Dimethylpropyl | -H | 2-Chlorphenyl | -Cl | Cyclopropyl | | 5,61 | |
| 417 | 1,2-Dimethylpropyl | -H | 2,4,6-Trifluorphenyl | -Cl | -Br | | 5,13 | |
| 418 | -CH₂-CH₂-CH(CH₃)-CH₂-CH₂ | * | 2,4,6-Trifluorphenyl | -Cl | -Br | | 5,65 | |
| 419 | -CH₂-CH₂-O-CH₂-CH₂- | * | 2,4,6-Trifluorphenyl | -Cl | -Br | | 3,97 | |
| 420 | 1,2,2-Trimethylpropyl | -H | 2-Chlorphenyl | -Cl | Cyclopropyl | | 5,97 | |
| 421 | 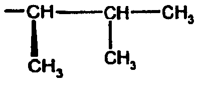 | -H | 2-Chlor-4-fluorphenyl | -Cl | -CH₃ | | 4,79 | |
| 422 | 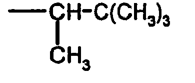 | -H | 2-Chlor-4-fluorphenyl | -Cl | -CHO | | 4,43 | |
| 423 | 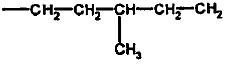 | * | 2-Chlorphenyl | -Cl | -CHO | | 4,42 | |
| 424 | 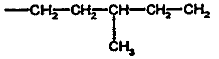 | * | 2-Chlor-4-fluorphenyl | -Cl | -CHO | | 4,53 | |
| 425 | 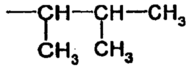 | H | 2-Chlor-phenyl | -Cl | -CHO | | 3,98 | |
| 426 | -CH₂-CH₂-O-CH₂-CH₂-CH₂- | * | 2,4,6-Trifluorphenyl | -Cl | -CHO | | 2,78 | |
| 427 | 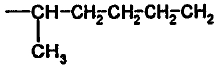 | * | 2,4,6-Trifluorphenyl | -Cl | -CHO | | 4,24 | |
| 428 | 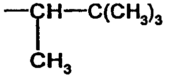 | H | 2-Chlor-6-fluorphenyl | -Cl | -CHO | | 4,25 | |
| 429 | -NH-CH₂-CH₂-CH₂-CH₂ | * | 2,4,6-Trifluorphenyl | -Cl | -CHO | | 3,28 | |
| 430 | 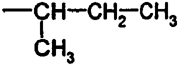 | H | 2,4,6-Trifluorphenyl | -Cl | -CHO | | 3,52 | |
| 431 | 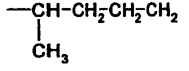 | * | 2,4,6-Trifluorphenyl | -Cl | -CHO | | 3,74 | |
| 432 | -CH₂-CH₂-S-CH₂-CH₂- | ***** | 2,4,6-Trifluorphenyl | -Cl | -CHO | | 3,45 | |
| 433 | 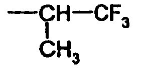 | H | 2,4,6-Trifluorphenyl | -Cl | -CHO | | 3,39 | |
| 434 | -CH₂-CH₂-O-CH₂-CH₂- | * | 2-Chlor-4-fluorphenyl | -Cl | -CHO | | 2,94 | |
| 435 | -CH₂-CH₂-S-CH₂-CH₂- | * | 2-Chlor-phenyl | -Cl | -CHO | | 3,51 | |
| 436 | 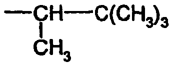 | H | 2,4,6-Trifluorphenyl | -Cl | -CHO | | 3,16 | |
| 437 | 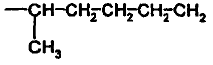 | * | 2-Chlorphenyl | -Cl | -CHO | | 3,80 | |
| 438 | -CH₂-CH₂-S-CH₂-CH₂- | * | 2-Chlor-4-fluor-phenyl | -Cl | -CHO | | 3,65 | |
| 439 | 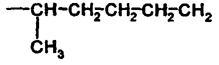 | * | 2-Chlor-4-fluorphenyl | -Cl | -CHO | | 4,45 | |
| 440 | 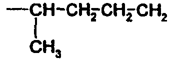 | * | 2-Chlor-4-fluorphenyl | -Cl | -CHO | | 3,95 | |

| | | | | | | | | |
|---|---|---|---|---|---|---|---|---|
| # steht für die Anknüpfungsstelle Die Bestimmung der logP-Werte erfolgte gemäß EEC-Directive 79/831 Annex V. A8 durch HPLC (Gradientenmethode, Acetonitril/0,1 % wässrige Phosphorsäure) *) bedeutet, dass R¹ und R² gemeinsam mit dem Stickstoffatom, an das sie gebunden sind, einen heterocyclischen Ring bilden. **) Die Produkte wurden teilweise als Stereoisomere isoliert. "S", bzw. "R" bedeutet S- bzw. R-Konfiguration am Chiralitätszentrum; "AS" bedeutet eine eindeutige aber unbekannte Konfiguration am Atropiezentrum und S-Konfiguration am Chiralitätszentrum. BS bedeutet die jeweils andere eindeutige aber unbekannte Konfiguration am Atropiezentrum und S-Konfiguration am Chiralitätszentrum. "AR" und "BR" bedeuten wiederum die jeweiligen komplementären Konfigurationen am Atropiezentrum gepaart mit der R-Konfiguration am Chiralitätszentrum. Demnach sind bei gleichen Substituenten "AR" und "BS", sowie "AS" und "BR" jeweils Enantiomerenpaare. | | | | | | | | |

### Herstellung der Verbindung gemäß Beispiel 401

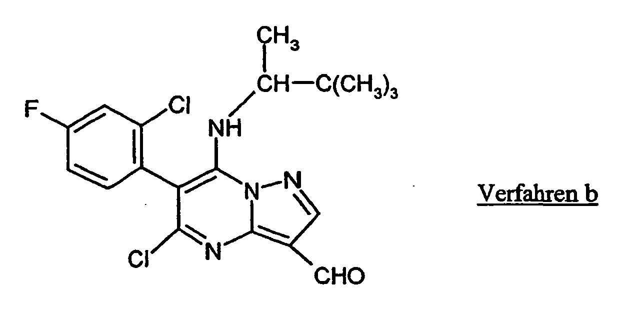

In eine Lösung von 300 mg (0,738 mmol) 3-Cyano-5-chlor-6-(2-chlor-4-fluorphenyl)-7-(1,2,2-trimethyl-propyl-amino)-pyrazolo[1,5a]pyrimidin in 13,2 g Dichlormethan wird unter Argonatmosphäre und unter Rühren bei -50°C langsam eine 1-molare Lösung von 649 mg (0,812 mmol) Di-isobutyl-aluminiumhydrid in Toluol eingetropft. Nach beendeter Zugabe wird noch 30 Minuten bei -50°C gerührt. Anschließend lässt man die Temperatur des Reaktionsgemisches auf 0°C ansteigen, versetzt dann mit gesättigter, wässriger Ammoniumchlorid-Lösung und rührt 2 Stunden bei 0°C. Man versetzt mit 1-N Salzsäure, trennt die organische Phase ab und extrahiert die wässrige Phase noch dreimal mit Dichlormethan. Die vereinigten organischen Phasen werden nacheinander mit gesättigter, wässriger Natriumhydrogencarbonat-Lösung und mit gesättigter, wässriger Kochsalz-Lösung gewaschen, über Natriumsulfat getrocknet und dann unter vermindertem Druck eingeengt. Man erhält auf diese Weise 300 mg (99 % der Theorie) an 3-Formyl-5-chlor-6-(2-chlor-4-fluor-phenyl)-7-(1,2,2-trimethyl-propylamino)-pyrazolo[1,5a]pyrimidin.
HPLC: logP = 4,43.

### Herstellung der Verbindung gemäß Beispiel 402

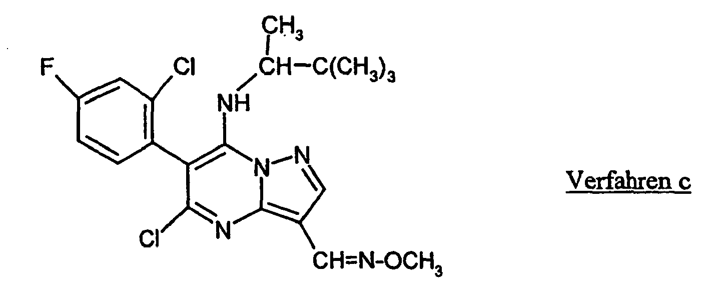

Eine Lösung von 300 mg (0,733 mmol) 3-Formyl-5-chlor-6-(2-chlor-4-fluor-phenyl)-7-(1,2,2-trimethylpropylamino)-pyrazlo[1,5a]pyrimidin in 20 ml Ethanol wird bei Raumtemperatur mit 73 mg (0,880 mmol) Methoxyamin-Hydrochlorid und 1,0 g des unter der Bezeichnung Amberlyst A-21 im Handel befindlichen, schwach basischen Ionenaustauschers versetzt und 18 Stunden bei Raumtemperatur geschüttelt. Danach wird das Reaktionsgemisch filtriert und das Filtrat unter vermindertem Druck eingeengt. Man erhält 220 mg an 3-Methoximino-5-chlor-6-(2-chlor-4-fluor-phenyl)-7-(1,2,2-trimethyl-propylamino)-pyrazolo[1,5a]pyrimidin in Form eines Atropisomerengemisches.
HPLC: logP = 5,48.

### Herstellung von Vorprodukten der Formel (II)

### Beispiel 441

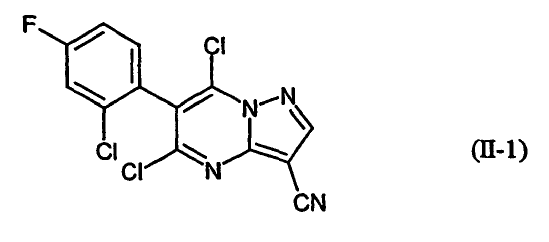

### Verfahren (g):

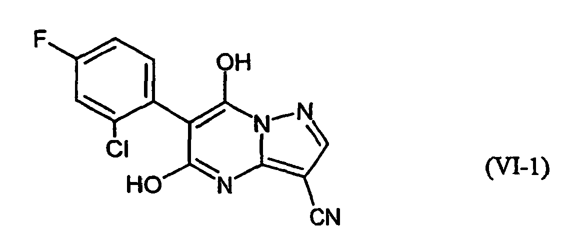

48 g (0,184 Mol) 2-Chlor-4-fluor-phenylmalonsäuredimethylester werden mit 19,91 g (0,184 Mol) 4-Cyano-5-aminopyrazol und mit 37,55 g (0,203 Mol) Tri-n-butylamin vermischt und 6 Stunden bei 180°C gerührt. Das bei der Reaktion entstehende Methanol wird abdestilliert. Anschließend wird das Reaktionsgemisch auf Raumtemperatur abgekühlt. Bei 95°C und 1 mbar werden flüchtige Komponenten abdestilliert. Man erhält als Rückstand 6-(2-Chlor-4-fluorphenyl)-5,7-dihydroxypyrazolo[1,5-a]pyrimidin-3-carbonitril in Form eines Rohproduktes, das ohne zusätzliche Reinigung für die weitere Synthese verwendet wird.

### Verfahren (e)

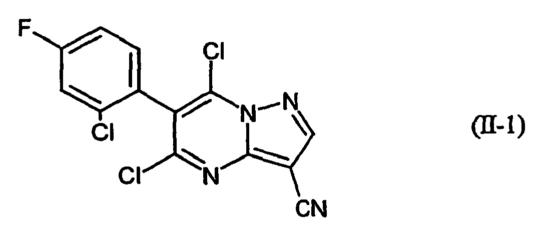

Das zuvor erhaltene 6-(2-Chlor-4-fluor-phenyl)-5,7-dihydroxy-pyrazolo[1,5-a]pyrimidin-3-carbonitril wird im Rohzustand in 367,3 g (2,395 Mol) Phosphoroxychlorid gelöst. Man gibt bei Raumtemperatur 31,95 g (0,153 Mol) Phosphorpentachlorid in Portionen dazu. Dann kocht man die Mischung 12 Stunden unter Rückfluss. Die flüchtigen Komponenten werden unter vermindertem Druck abdestilliert, der Rückstand wird mit Dichlormethan versetzt und mit Wasser gewaschen. Die organische Phase wird über Natriumsulfat getrocknet und unter vermindertem Druck eingeengt. Der Rückstand wird mit 3 Teilen Cyclohexan und 1 Teil Essigsäureethylester als Laufmittel an Kieselgel chromatographiert. Man erhält 21 g 95,7 %iges 3-Cyano-5,7-dichlor-6-(2-chlor-4-fluorphenyl)-pyrazolo[1,5-a]pyrimidin.
HPLC: logP = 3,48
¹H-NMR (DMSO-d6, Tetramethylsilan): δ = 7.44-7.52 (1H); 7.62-7.66 (1H); 7.71-7.77 (1H); 9.03 (1H) ppm.

### Beispiel 442

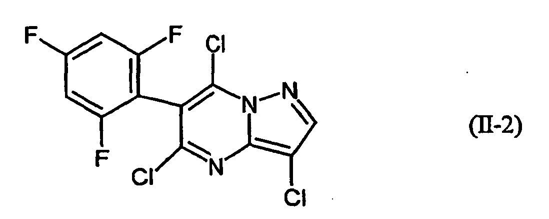

### Verfahren (e)

26 g (82,4 mMol) 3-Chlor-6-(2,4,6-trifluorphenyl)-pyrazolo[1,5-a]pyrimidin-5,7-diol und 8,6 g (41,2 mMol) Phosphorpentachlorid werden in 126,3 g Phosphoroxychlorid eine Stunde bei 110°C gerührt. Nach dem Abkühlen wird die Reaktionsmischung unter Eiskühlung mit Wasser und Dichlormethan verrührt. Die organische Phase wird abgetrennt, getrocknet und unter vermindertem Druck eingeengt. Der Rückstand wird mit Methyl-t-butylether/Petrolether (1:9) an Kieselgel chromatografiert. Man erhält 5 g (16,4 % der Theorie) an 3,5,7-Trichlor-6-(2,4,6-trifluorphenyl)pyrazolo[1,5-a]-pyrimidin.
HPLC: logP = 3,97

### Beispiel 443

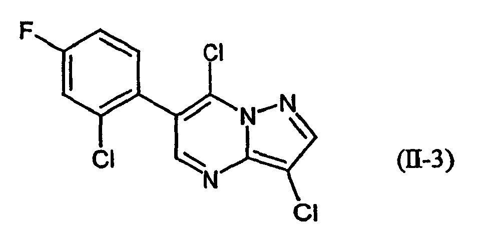

### Verfahren (d):

14,2 g (11,9 mMol) 25 %iges 3-Chlor-6-(2-chlor-4-fluorphenyl)-pyrazolo[1,5-a]pyrimidin-7-ol und 1,24 g (5,9 mMol) Phosphorpentachlorid werden in 16,3 g Phosphoroxychlorid eine Stunde bei 110°C und dann 4 Stunden ohne weitere Wärmezufuhr gerührt. Nach dem Abkühlen wird die Reaktionsmischung unter Eiskühlung mit Wasser und Dichlormethan verrührt. Die organische Phase wird abgetrennt, getrocknet und unter vermindertem Druck eingeengt. Der Rückstand wird mit n-Hexan/Essigsäureethylester (3:1 bis 1:1) an Kieselgel chromatografiert. Man erhält 2,1 g (54 % der Theorie) an 3,7-Dichlor-6-(2-chlor-4-fluorphenyl)pyrazolo[1,5-a]-pyrimidin.
HPLC: logP = 3,56

Nach den zuvor angegebenen Methoden werden auch die in der folgenden Tabelle 2 aufgeführten Verbindungen der Formel 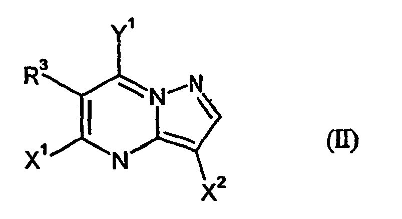 hergestellt.

**Tabelle 2**

| Bsp. Nr. | X¹ | Y¹ | R³ | X² | logP | Fp.: (°C) |
|---|---|---|---|---|---|---|
| 444 | -Cl | -Cl | 2-Chlor-6-fluorphenyl | -CN | 3,31 | |
| 445 | -Cl | -Cl | 2-Chlor-4-fluorphenyl | -Cl | | |
| 446 | -Cl | -Cl | 2,4-Difluorphenyl | -CN | 3,16 | 136-38 |
| 447 | -Cl | -Cl | 2,6-Dichlorphenyl | -CN | 3,59 | |
| 448 | -Cl | -Cl | 2,4,6-Trifluorphenyl | -CN | 3,2 | |
| 449 | -H | -Cl | 2-Chlor-6-fluorphenyl | -CN | | |
| 450 | H | -Cl | 2-Chlor-6-fluorphenyl | -Cl | | |
| 451 | -Cl | -Cl | 2,4,6-Trifluorphenyl | 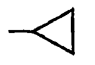 | 4,38 | |
| 452 | -Cl | -Cl | 2-Chlorphenyl | 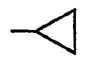 | | |

Die Bestimmung der logP-Werte erfolgte gemäß EEC-Directive 79/831 Annex V. A8 durch HPLC (Gradientenmethode, Acetonitril/0,1 % wässrige Phosphorsäure)

### Herstellung von Vorprodukten der Formeln (V) und (VI):

### Beispiel 453

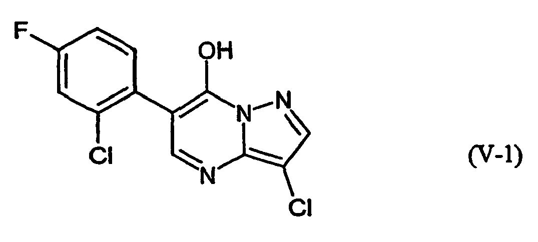

### Verfahren (f)

11,3 g (43,85 mMol) 2-(2-Chlor-4-fluorphenyl)-3-(dimethylamino)-2-acrylsäure-methyl und 5,2 g (43,85 mMol) 4-Chlor-1H-pyrazol-5-amin werden in 11,5 ml Tri-n-butylamin 6 Stunden bei 180°C gerührt, wobei entstehendes Methanol und Dimethylamin abdestilliert werden. Anschließend wird die Mischung unter vermindertem Druck weiter eingeengt. Man erhält 14,2 g (27 % der Theorie) 25%iges 3-Chlor-6-(2-chlor-4-fluorphenyl)pyrazolo[1,5-a]pyrirnidin-7-ol.

### Beispiel 454

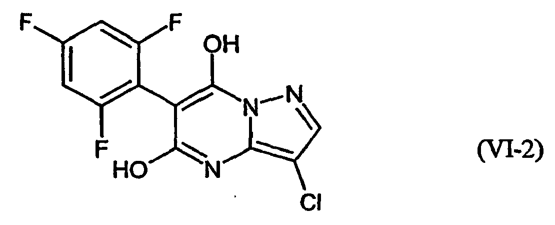

### Verfahren (g)

11,15 g (42,5 mMol) 2-(2,4,6-Trifluorphenyl)-malonsäuredimethylester und 5 g (42,5 mMol) 4-Chlor-1H-pyrazol-5-amin werden in 11,5 ml Tri-n-butylamin 3 Stunden bei 180°C gerührt, wobei entstehendes Methanol abdestilliert wird. Das Produkt wird abdekantiert. Man erhält 21 g (76 % der Theorie) 49 %iges 3-Chlor-6-(2,4,6-trifluorphenyl)-pyrazolo[1,5-a]pyrimidin-5,7-diol.

Nach den zuvor angegebenen Methoden werden auch die in der folgenden Tabelle 3 aufgeführten Verbindungen der Formel 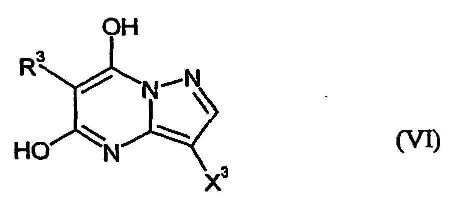 hergestellt.

**Tabelle 3**

| Bsp.-Nr. | R³ | X³ | log P |
|---|---|---|---|
| 455 | 2,4,6-Trifluorphenyl | 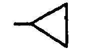 | |
| 456 | 2-Chlorphenyl | 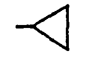 | |
| 457 | 2-Chlor-4-fluor-phenyl | -CH₃ | |

### Herstellung von Aminen der Formel (III)

### Beispiel 458

### Verfahren (h) erste Stufe:

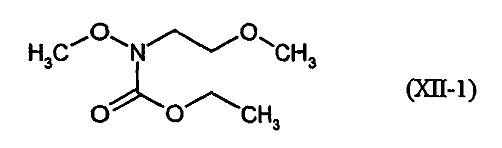

1 000 mg N-Methoxy-carbaminsäureethylester werden in 10,0 ml Dimethylformamid vorgelegt und portionsweise mit 403 mg Natriumhydrid versetzt, wobei die Temperatur durch Kühlung auf 30°C eingestellt wurde. Die Reaktionsmischung wird für 2 Stunden bei 30°C gerührt und anschließend mit 3 500 mg 2-Bromethyl-methylether versetzt. Die Reaktionsmischung wird für 18 Stunden bei 20°C bis 25°C gerührt und anschließend in 20 ml Wasser eingerührt. Die erhaltene Reaktionsmischung wird unter vermindertem Druck zur Trockne eingeengt und viermal mit je 30 ml Dichlormethan extrahiert. Die organischen Extrakte werden über Natriumsulfat getrocknet, filtriert und unter vermindertem Druck zur Trockne eingeengt.

Man erhält 1200 mg (N-Methoxy-N-methoxyethyl)carbaminsäure-ethylester (Reinheit 77,6 %, Ausbeute 62,6 %).

### Verfahren (h), zweite Stufe:

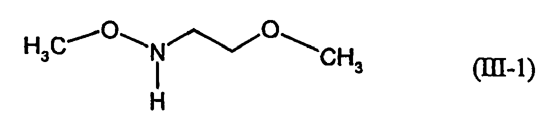

200 mg (N-Methoxy-N-methoxyethyl)-carbaminsäureethylester werden in 4,0 ml wässrigem Ethanol (59 %ig) vorgelegt, mit 240,6 mg Kaliumhydroxid versetzt und für 18 Stunden bei 40°C gerührt. Die Reaktionsmischung wird dann in 50 ml Wasser eingerührt, dreimal mit je 20 ml Diethylether und dreimal mit je 20 ml Dichlormethan extrahiert. Die vereinigten organischen Phasen werden zweimal mit je 20 ml Wasser gewaschen, getrocknet und bei 20°C unter vermindertem Druck auf ein Volumen von 20 ml eingeengt.

Die erhaltene Lösung wird unter Eiskühlung mit 2 ml Salzsäure versetzt, 1 Stunde bei Raumtemperatur gerührt und bei 20°C unter vermindertem Druck zur Trockne eingeengt.

Das erhaltene Produkt wird dreimal mit je 15 ml Methanol digeriert und anschließend bei 20°C unter vermindertem Druck zur Trockne eingeengt.

Man erhält 140 mg N-Methoxy-N-methoxyethylamin-hydrochlorid (Ausbeute 87,6 %).

### Beispiel 459

### Verfahren (i), erste Stufe:

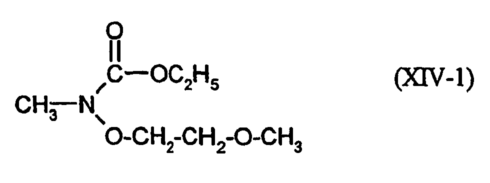

Ein Gemisch aus 1000 mg N-Hydroxy-N-methyl-carbaminsäure-ethylester, 1166 mg 2-Bromethyl-methylether wird unter Rühren auf Rückflusstemperatur erhitzt und dann tropfenweise mit einer Lösung von 493 mg Kaliumhydroxid in 5 ml Ethanol versetzt. Man kocht das Reaktionsgemisch 10 Stunden unter Rückfluss und arbeitet dann auf, indem man das Reaktionsgemisch filtriert und das Filtrat unter vermindertem Druck einengt. Der verbleibende Rückstand wird mit einem Gemisch aus Wasser und Essigsäureethylester versetzt Die organische Phase wird abgetrennt, mit gesättigter, wässriger Ammoniumchlorid-Lösung und dann mit Wasser gewaschen. Anschließend wird die organische Phase über Natriumsulfat getrocknet und unter vermindertem Druck eingeengt. Man erhält auf diese Weise 0,7 g eines Produktes, das gemäß Gaschromatogramm zu 83 % aus (N-Methyl-N-methoxyethoxy)-carbaminsäure-ethylester besteht. Die Ausbeute errechnet sich danach zu 39 % der Theorie.

### Verfahren (i), zweite Stufe:

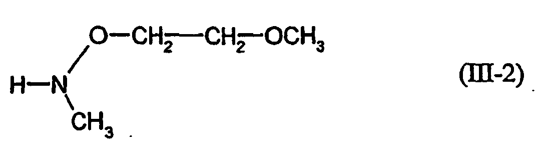

Ein Gemisch aus 200 mg (N-Methyl-N-methoxyethoxy)-carbaminsäure-ethylester, 4 ml Ethanol und 4 ml Wasser wird mit 240,6 mg pulverisiertem Kaliumhydroxid versetzt und 2 Stunden bei 40°C gerührt. Das Reaktionsgemisch wird danach in 50 ml Wasser eingerührt, dann dreimal mit je 20 ml Diethylether und anschließend dreimal mit je 20 ml Methylenchlorid extrahiert. Die vereinigten organischen Phasen werden zweimal mit je 20 ml Wasser gewaschen, über Natriumsulfat getrocknet und bei Raumtemperatur unter vermindertem Druck auf ein Volumen von 20 ml eingeengt. Die erhaltene Lösung wird unter Eiskühlung mit 1 ml etherischer Salzsäure versetzt. Die sich abscheidenden Kristalle werden abfiltriert und getrocknet. Man erhält auf diese Weise 190 mg an N-Methyl-N-methoxyethoxy-amin-hydrochlorid.

### Beispiel 460

### Verfahren (i), erste Stufe:

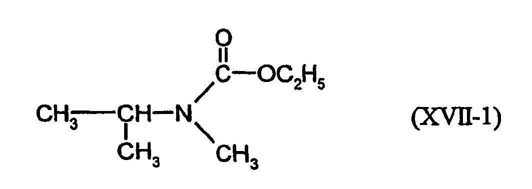

Ein Gemisch aus 2000 mg N-(2,2,2-Trifluor-1-methyl-ethyl)-carbaminsäure-ethylester und 20 ml Tetrahydrofuran wird bei Raumtemperatur unter Rühren mit 475 mg Natriumhydrid versetzt. Danach wird unter Rühren bei Raumtemperatur eine Lösung von 4600 mg Iodmethan in 10 ml Tetrahydrofuran hinzugetropft. Das Reaktionsgemisch wird 16 Stunden bei 50°C gerührt und dann mit Wasser versetzt. Man extrahiert dreimal mit je 20 ml Methylenchlorid, trocknet die vereinigten organischen Phasen über Natriumsulfat und engt unter vermindertem Druck ein. Man erhält 1000 mg eines Produktes, das gemäß Gaschromatogramm zu 75 % aus N-(2,2,2-Trifluor-1-methyl-ethyl)-N-methyl-carbaminsäure-ethylester besteht. Die Ausbeute errechnet sich danach zu 34,86 %.

### Verfahren (j), zweite Stufe:

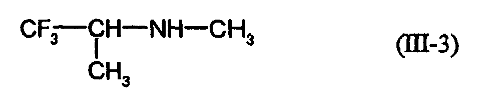

Ein Gemisch aus 1000 mg N-(2,2,2-Trifluor-1-methyl-ethyl)-N-methyl-carbaminsäure-ethylester, 20 ml Ethanol und 20 ml Wasser wird mit 1070 mg pulverisiertem Kaliumhydroxid versetzt und 66 Stunden bei 40°C gerührt. Danach wird das Reaktionsgemisch mit Wasser verdünnt und dreimal mit je 20 ml eines Gemisches extrahiert, das zu gleichen Teilen aus Methylenchlorid und Diethylether besteht. Die vereinigten organischen Phasen werden über Natriumsulfat getrocknet und dann bei Raumtemperatur unter leicht vermindertem Druck eingeengt. Die erhaltene Lösung wird unter Eiskühlung mit etherischer Salzsäure versetzt und 60 Stunden bei Raumtemperatur gerührt. Nach dem Einengen unter vermindertem Druck erhält man 280 mg an N-(2,2,2-Trifluor-1-methyl-ethyl)-N-methylamin-hydrochlorid. Die Ausbeute errechnet sich danach zu 34 % der Theorie.

### Beispiel 461

### Verfahren (k):

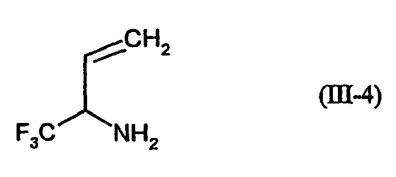

600 mg N-(1-Trifluormethyl-2-propen)-carbaminsäurebenzylester werden in 8,0 ml 16 %iger Salzsäure für 1,5 Stunden unter Rückfluss erhitzt. Nach dem Abkühlen auf 20°C wird zweimal mit je 20 ml Diethylether extrahiert.

Die verbleibende wässrige Phase wird unter vermindertem Druck zur Trockne eingeengt und dreimal mit je 10 ml Methanol versetzt. Nach Entfernen des Methanols unter vermindertem Druck werden 310 mg an (1-Trifluormethyl-prop-2-en)-amin-hydrochlorid isoliert. Die Ausbeute errechnet sich danach zu 82,9% der Theorie.

Nach den zuvor angegebenen Methoden lassen sich auch die in den folgenden Tabellen angegebenen Carbamate herstellen.

**Tabelle 4**

| | | | |
|---|---|---|---|
| 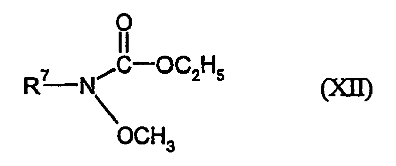 | | | |

| Beispiel-Nr. | Verb.-Nr. | R⁷ | logP |
|---|---|---|---|
| 462 | XII-2 | 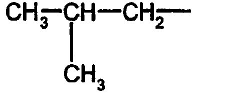 | 2,38 |
| 463 | XII-3 | 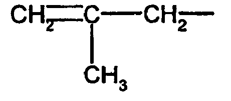 | 2,06 |

**Tabelle 5**

| | | | |
|---|---|---|---|
| 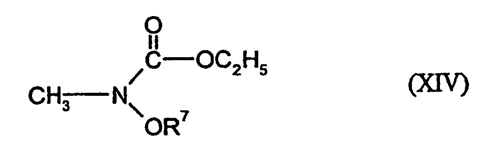 | | | |

| Beispiel-Nr. | Verb.-Nr. | R⁷ | Physikalische Konst. |
|---|---|---|---|
| 464 | XIV-2 | 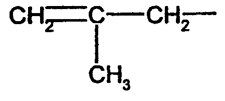 | |

**Tabelle 6**

| | | | |
|---|---|---|---|
| 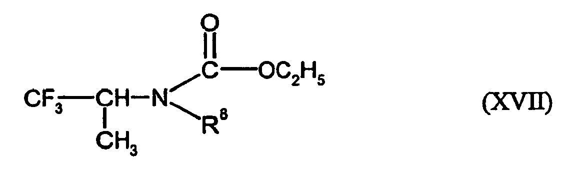 | | | |

| Beispiel-Nr. | Verb.-Nr. | R⁸ | Physikalische Konst. |
|---|---|---|---|
| 465 | XVII-2 | -C₂H₅ | ¹H-NMR (400 MHz, CD₃CN): |
| | | | δ (ppm) = 1,13 (t, CH₃CH₂N), 1,21 (t, CH₃CHCF₃), 1,23 (t, CH₃CH₂O), 3,20 (m, CH₂N, CHCF₃), 4,1 (q, CH₃CH₂O). |

Nach den zuvor angegebenen Methoden lassen sich auch die in der folgenden Tabelle aufgeführten Amine herstellen.

**Tabelle 7**

| | | | | |
|---|---|---|---|---|
| 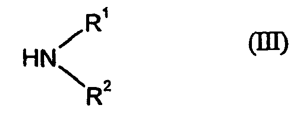 | | | | |

| Beispiel-Nr. | Verb.-Nr. | R¹ | R² | Physikal. Konst |
|---|---|---|---|---|
| 466 | III-5 | 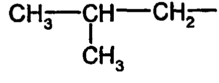 | -OCH₃ | ¹H-NMR (400 MHz, CD₃CN): |
| | | | | δ (ppm) = 1,03 (d, CH₃)₂CH), 3,06 (d, CH₂), 3,28 (b, (CH₃)₂CH), 4,01 (s, OCH₃) |
| 467 | III-6 | 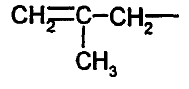 | -OCH₃ | ¹H-NMR (400 MHz, DMSO): |
| | | | | δ (ppm) = 1,76 (s, CH₃(CCH₂)CH₂), 3,29 (b, NH, CH₃(CCH₂)CH₂, OCH₃), 7,89, 5,02 (2 s, CH₃(CCH₂)CH₂). |
| 468 | III-7 | 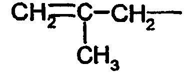 | | |
| 469 | III-8 | 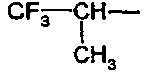 | -C₂H₅ | ¹H-NMR (400 MHz, DMSO): |
| | | | | δ (ppm) = 1,06 (m, CH₃CH₂N, CH₃CHCF₃), 3,20 (m, CH₂N), 4,1 (m, CHCF₃). |

Die in den Beispielen 466 bis 469 aufgeführten Amine wurden jeweils in Form ihrer Hydrochloride isoliert und charakterisiert.

### Herstellung eines Aminopyrazols

### Beispiel 470

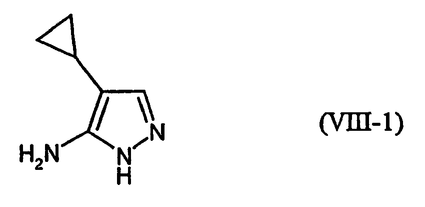

a) Ein Gemisch aus 16,223 g (200 mmol) Cyclopropylacetonitril und 15,556 g (210 mmol) Ethylformiat wird unter Argonatmosphäre und unter Rühren bei Raumtemperatur innerhalb von einer Stunde in 400 ml Diethylether eingetropft. Man fügt 4,598 g (200 mmol) Natrium hinzu und rührt 4 Tage lang bei Raumtemperatur. Wenn sich das metallische Natrium gelöst hat, wird auf 10°C abgekühlt und innerhalb von 30 Minuten mit 12,01 g (200 mmol) Essigsäure versetzt, wobei das Reaktionsgemisch auf Temperaturen zwischen 10°C und 15°C gehalten wird. Man rührt 15 Minuten nach, saugt das Reaktionsgemisch dann ab und wäscht mit 30 ml kaltem Diethylether nach. Das Filtrat wird unter vermindertem Druck eingeengt. Man erhält auf diese Weise 22,0 g an 1-Formyl-1-cyclopropyl-acetonitril in Form eines Rohproduktes, das ohne vorherige Reinigung für die weitere Synthese verwendet wird.
b) Ein Gemisch aus 8,670 g (0,173 mol) Hydrazinhydrat und 3,12 ml Essigsäure wird unter Rühren bei Raumtemperatur in eine Lösung von 21,825 g (0,200 mol) 1-Formyl-1-cyclopropyl-acetonitril in 20 ml Ethanol gegeben. Das Reaktionsgemisch wird 4 Stunden unter Rückfluß erhitzt und dann aufgearbeitet, indem man unter vermindertem Druck einengt. Man erhält 13,7 g (55,6 % der Theorie) an 4-Cyclopropyl-1H-pyrazol-5-amin.

### Beispiel 471

Nach den zuvor angegebenen Methoden wird auch die Verbindung der Formel

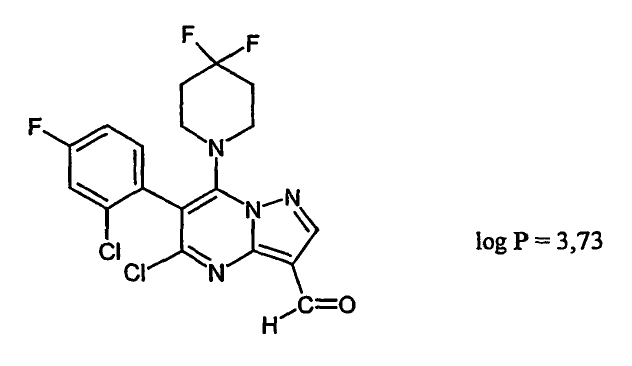

### Verwendungsbeispiele

### Beispiel A

### Venturia - Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers Venturia inaequalis inokuliert und verbleiben dann 1 Tag bei ca. 20°C und 100% relativer Luftfeuchtigkeit in einer Inkubationskabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0% ein Wirkungsgrad, der demjenigen der Kontrolle entspricht, während ein Wirkungsgrad von 100% bedeutet, dass kein Befall beobachtet wird.

Wirkstoffe, Aufwandmengen an Wirkstoffen und Versuchsergebnisse gehen aus der folgenden Tabelle hervor.

**Tabelle A**

| Venturia- Test (Apfel) / protektiv | | |
|---|---|---|
| **Wirkstoff** | **Aufwandmenge in g/ha** | **Wirkungsgrad in %** |
| Erfindungsgemäß: | | |
| 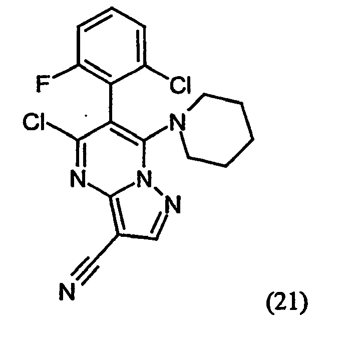 | 100 | |
| 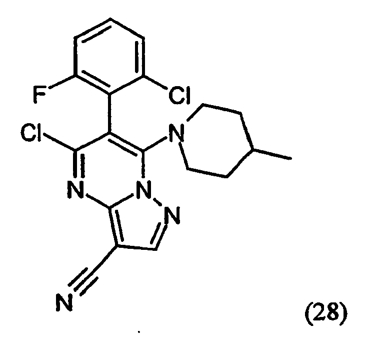 | 100 | 97 |
| 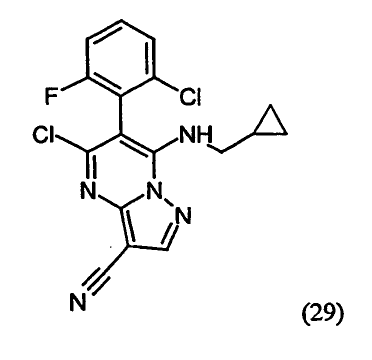 | 100 | 100 |
| 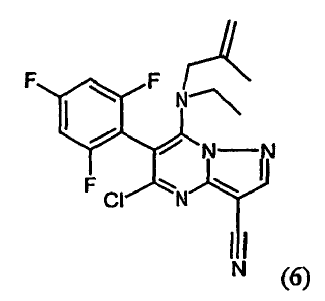 | 100 | 100 |
| 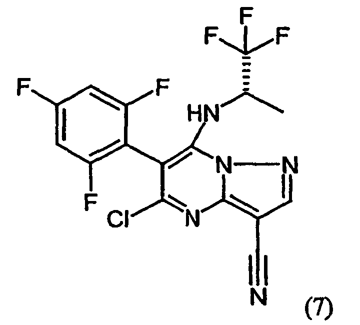 | 100 | 100 |
| 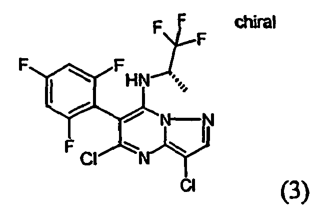 | 100 | 98 |
| 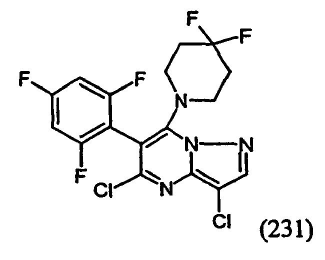 | 100 | 100 |
| 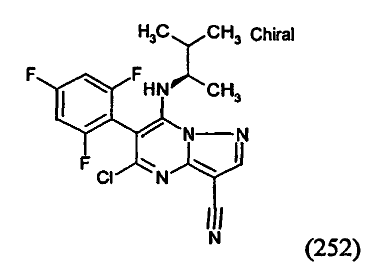 | 100 | 100 |
| 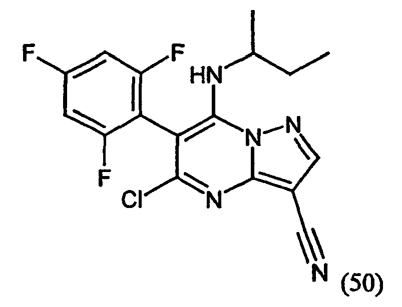 | 100 | 100 |
| 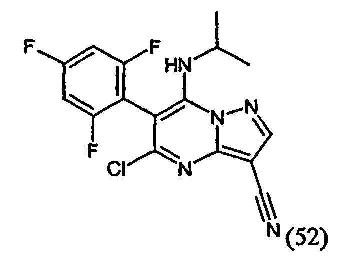 | 100 | 98 |
| 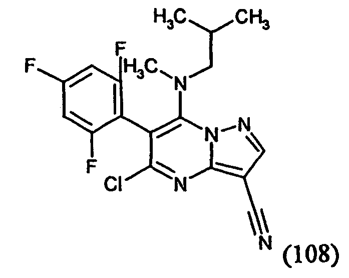 | 100 | 100 |
| 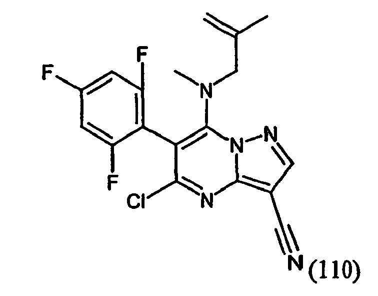 | 100 | 100 |
| 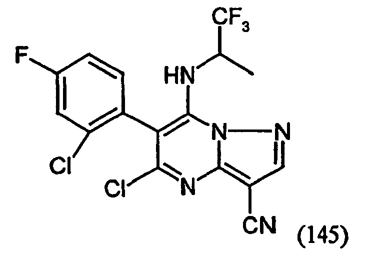 | 100 | 93 |
| 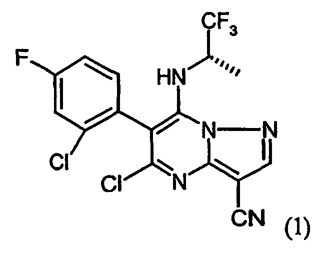 | 100 | 100 |
| 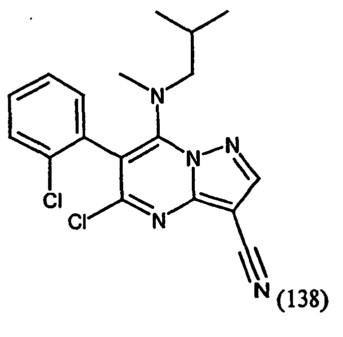 | 100 | 100 |
| 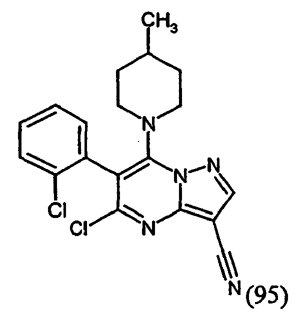 | 100 | 99 |
| 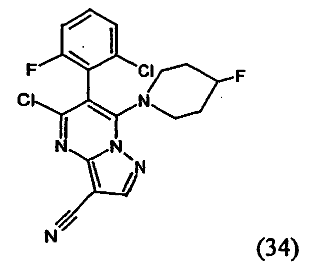 | 100 | 98 |
| 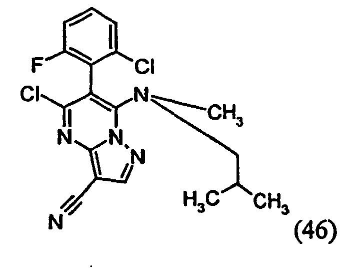 | 100 | 99 |
| 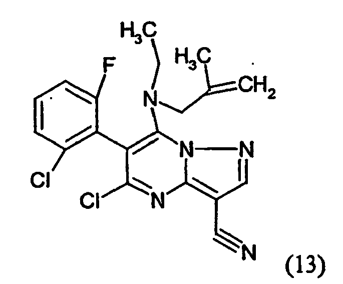 | 100 | 100 |
| 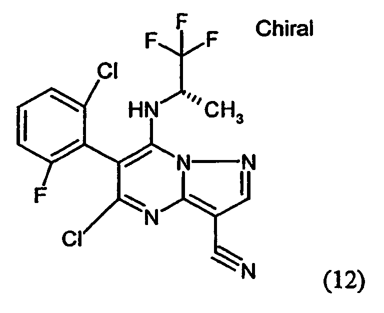 | 100 | 100 |

### Beispiel B

### Botrytis - Test (Bohne) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten Kammer bei ca. 20°C und 100% relativer Luftfeuchtigkeit aufgestellt.

2 Tage nach der Inokulation wird die Größe der Befallsflecken auf den Blättern ausgewertet. Dabei bedeutet 0% ein Wirkungsgrad, der demjenigen der Kontrolle entspricht, während ein Wirkungsgrad von 100% bedeutet, dass kein Befall beobachtet wird.

Wirkstoffe, Aufwandmengen an Wirkstoffen und Versuchsergebnisse gehen aus der folgenden Tabelle hervor.

**Tabelle B**

| Botrytis - Test (Bohne) / protektiv | | |
|---|---|---|
| **Wirkstoff** | **Aufwandmenge an Wirkstoff in g/ha** | **Wirkungsgrad in %** |
| Erfindungsgemäß: | | |
| 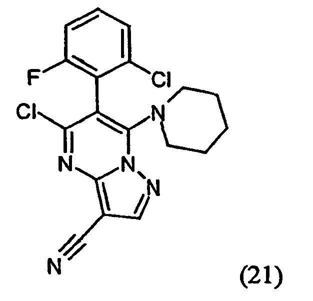 | 500 | 93 |
| 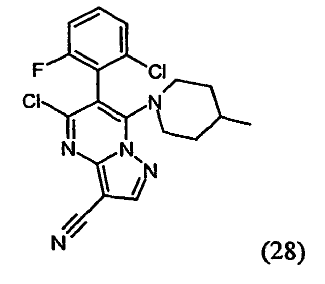 | 500 | 96 |
| 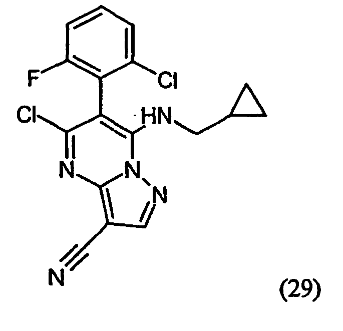 | 500 | 99 |
| 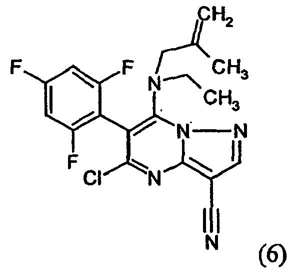 | 500 | 100 |
| 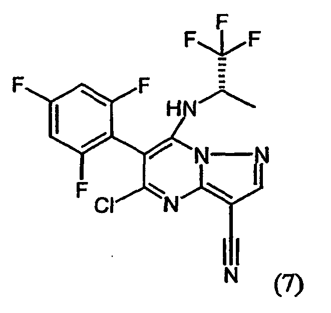 | 500 | 99 |
| 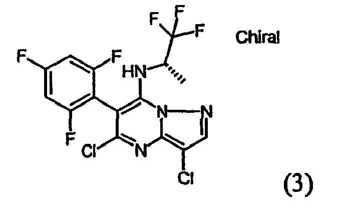 | 500 | 100 |
| 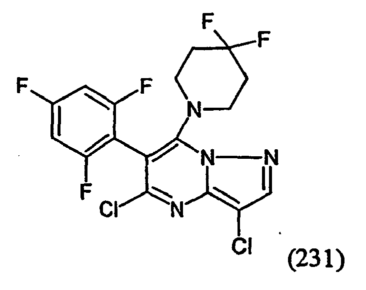 | 500 | 95 |
| 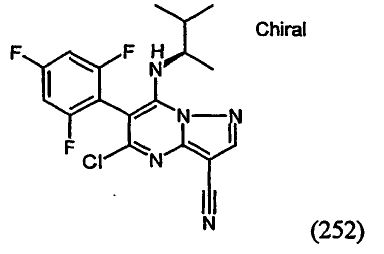 | 500 | 96 |
| 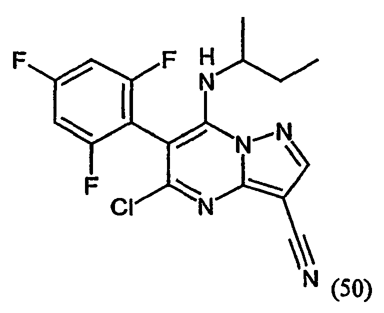 | 500 | 97 |
| 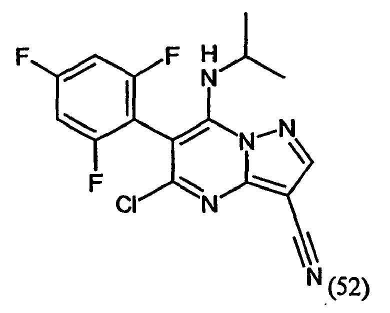 | 500 | 93 |
| 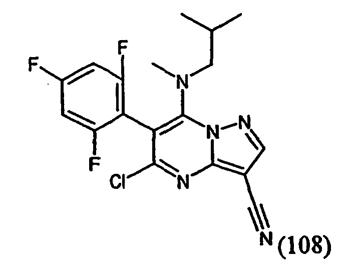 | 500 | 100 |
| 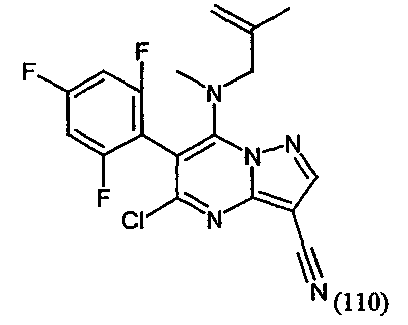 | 500 | 100 |
| 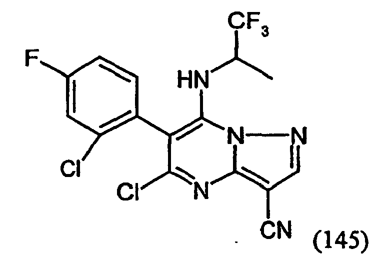 | 500 | 99 |
| 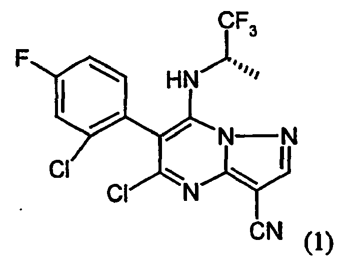 | 500 | 100 |
| 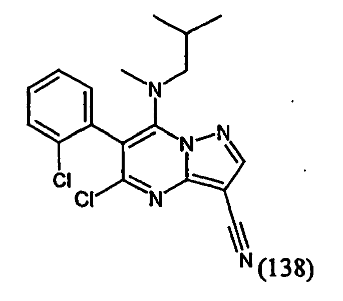 | 500 | 100 |
| 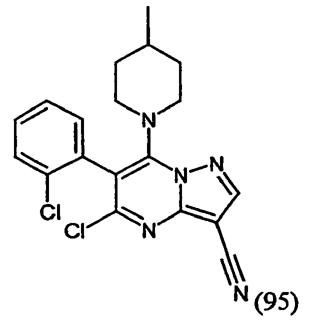 | 500 | 95 |
| 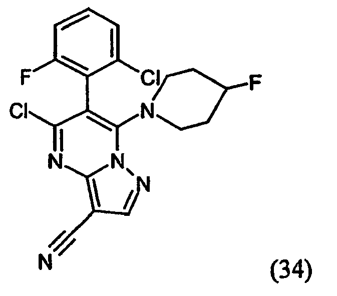 | 500 | 94 |
| 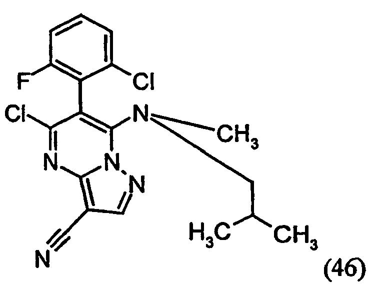 | 500 | 99 |
| 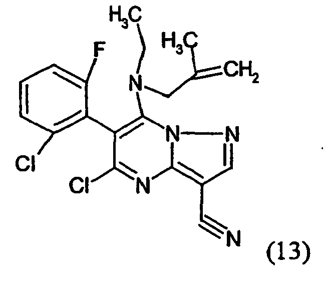 | 500 | 100 |
| 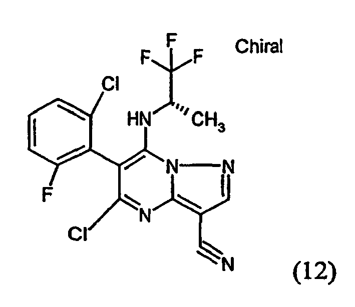 | 500 | 100 |

### Beispiel C

### Alternaria-Test (Tomate) protektiv

| | | |
|---|---|---|
| Lösungsmittel: | 49 Gewichtsteile | N, N - Dimethylformamid |
| Emulgator: | 1 Gewichtsteil | Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Tomatenpflanzen mit der Wirkstoffzubereitung in der angegebenen Aufwandmenge. 1 Tag nach der Behandlung werden die Pflanzen mit einer Sporensuspension von Alternaria solani inokuliert und stehen dann 24h bei 100% rel. Feuchte und 20°C. Anschließend stehen die Pflanzen bei 96% rel. Luftfeuchtigkeit und einer Temperatur von 20°C.

7 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen an Wirkstoffen und Versuchsergebnisse gehen aus der folgenden Tabelle hervor.

**Tabelle C**

| Alternaria-Test (Tomate) / protektiv | | |
|---|---|---|
| **Wirkstoff** | **Aufwandmenge an Wirkstoff in g/ha** | **Wirkungsgrad in %** |
| Erfindungsgemäß: | | |
| 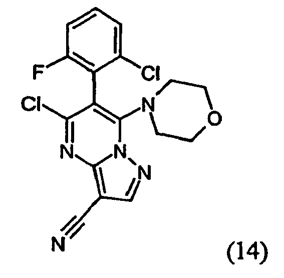 | 750 | 95 |
| 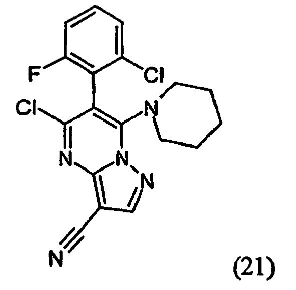 | 750 | 95 |
| 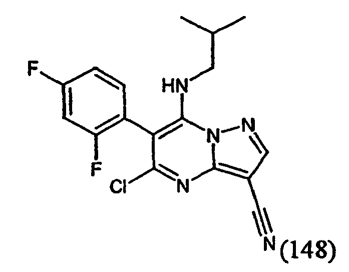 | 750 | 95 |
| 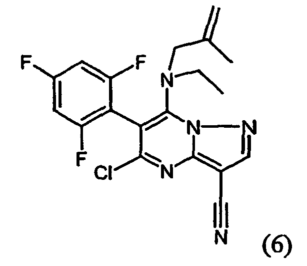 | 750 | 95 |
| 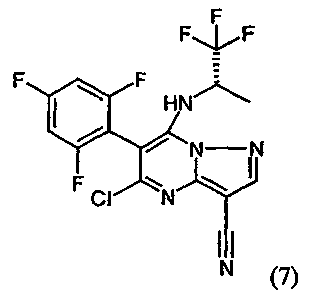 | 750 | 95 |
| 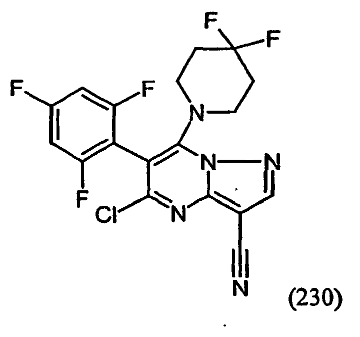 | 750 | 90 |

### Beispiel D

### Fusarium nivale (var. majus)-Test (Weizen) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer Konidiensuspension von Fusarium nivale (var. majus) besprüht.

Die Pflanzen werden in einem Gewächshaus unter lichtdurchlässigen Inkubationshauben bei einer Temperatur von ca. 15°C und einer relativen Luftfeuchtigkeit von ca. 100 % aufgestellt

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen an Wirkstoffen und Versuchsergebnisse gehen aus der folgenden Tabelle hervor.

**Tabelle D**

| Fusarium nivale (var. majus)-Test (Weizen) / protektiv | | |
|---|---|---|
| **Wirkstoff** | **Aufwandmenge an Wirkstoff in g/ha** | **Wirkungsgrad in %** |
| Erfindungsgemäß: | | |
| 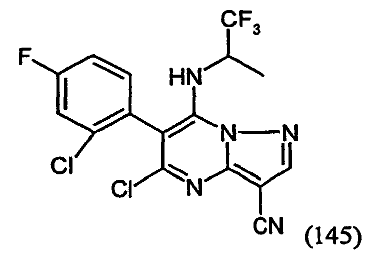 | 500 | 88 |
| 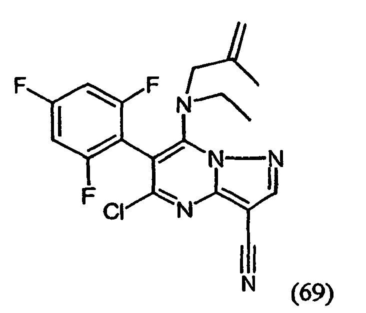 | 500 | 100 |
| 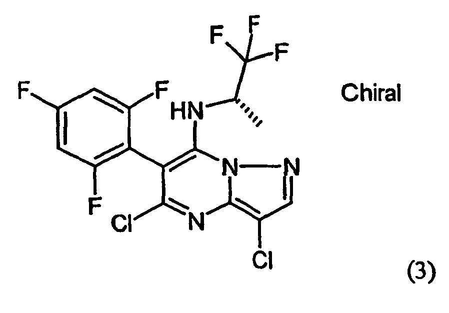 | 500 | 80 |

### Beispiel E

### Pyricularia-Test (Reis) / protektiv

| | |
|---|---|
| Lösungsmittel: | 25 Gewichtsteile N,N-Dimethylacetamid |
| Emulgator: | 0,6 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung in der angegebenen Aufwandmenge. Nach dem Antrocknen des Spritzbelages werden die Pflanzen mit einer wässrigen Sporensuspension von Pyricularia oryzae inokuliert. Anschließend werden die Pflanzen in einem Gewächshaus bei 100 % relativer Luftfeuchtigkeit und 25°C aufgestellt.

6 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

Wirkstoffe, Aufwandmengen an Wirkstoffen und Versuchsergebnisse gehen aus der folgenden Tabelle hervor.

**Tabelle E**

| Pyricularia-Test (Reis) / protektiv | | |
|---|---|---|
| **Wirkstoff** | **Aufwandmenge an Wirkstoff in g/ha** | **Wirkungsgrad in %** |
| Erfindungsgemäß: | | |
| 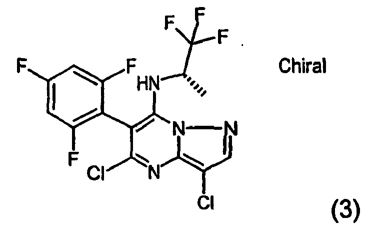 | 500 | 88 |
| 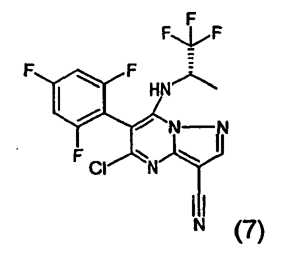 | 500 | 88 |
| 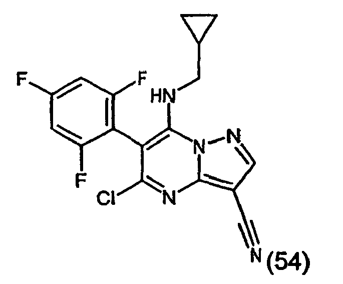 | 500 | 86 |
| 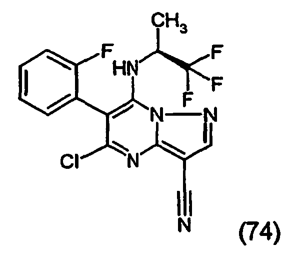 | 500 | 75 |

### Beispiel F

### Plutella-Test

| | |
|---|---|
| Lösungsmittel: | 100 Gewichtsteile Aceton |
| | 1900 Gewichtsteile Methanol |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Methanol auf die gewünschten Konzentrationen.

Auf eine genormte Menge Kunstfutter wird eine angegebene Menge Wirkstoffzubereitung der gewünschten Konzentration pipettiert Nachdem das Methanol verdunstet ist, werden ca. 200-300 Eier der Kohlschabe (Plutella xylostella) auf das Futter gegeben.

Nach der gewünschten Zeit wird die Abtötung der Eier bzw. Larven in % bestimmt. Dabei bedeutet 100 %, dass alle Tiere abgetötet wurden; 0 % bedeutet, dass keine Tiere abgetötet wurden.

Wirkstoffe, Wirkstoffkonzentrationen und Versuchsergebnisse gehen aus der folgenden Tabelle hervor.

**Tabelle F**

| pflanzenschädigende Insekten | | |
|---|---|---|
| Plutella -Test | | |
| **Wirkstoff** | **Wirkstoffkonzentration in ppm** | **Abtötungsgrad in % nach 7^{d}** |
| Erfindungsgemäß: | | |
| 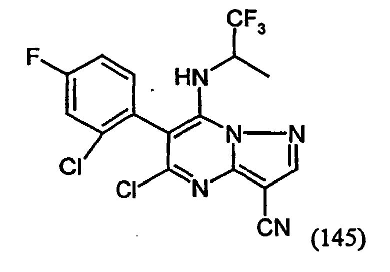 | 1000 | 100 |

## Patentansprüche

1. Pyrazolopyrimidine der Formel 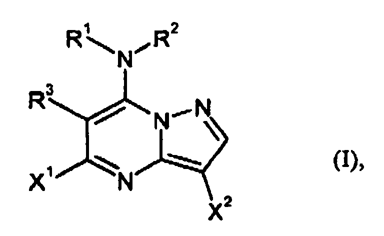 in welcher
R¹ für Hydroxy, Amino, für gegebenenfalls durch Halogen, Cyano, Hydroxy, Amino, Phenyl, Heterocyclyl, Alkoxy mit 1 bis 4 Kohlenstoffatomen, Alkoxycarbonyl mit 1 bis 4 Kohlenstoffatomen im Alkoxyteil, Alkylamino mit 1 bis 4 Kohlenstoffatomen, Dialkylamino mit 2 bis 8 Kohlenstoffatomen, Cycloalkyl mit 3 bis 6 Kohlenstoffatomen, Halogencycloalkyl mit 3 bis 6 Kohlenstoffatomen und 1 bis 5 Halogenatomen, Alkylthio mit 1 bis 4 Kohlenstoffatomen, Oxo, Hydroxyimino und/oder Alkoximino mit 1 bis 4 Kohlenstoffatomen substituiertes Alkyl mit 1 bis 6 Kohlenstoffatomen,
für gegebenenfalls durch Halogen, Cycloalkyl, Cyano, Phenyl und/oder Heterocyclyl substituiertes Alkenyl mit 2 bis 6 Kohlenstoffatomen,
für gegebenenfalls durch Halogen, Cycloalkyl, Cyano, Phenyl und/oder Heterocyclyl substituiertes Alkinyl mit 2 bis 6 Kohlenstoffatomen,
für gegebenenfalls durch Halogen, Cycloalkyl, Cyano, Halogenalkyl mit 1 bis 2 Kohlenstoffatomen und 1 bis 5 Halogenatomen, Phenyl und/oder Heterocyclyl substituiertes Cycloalkyl mit 3 bis 7 Kohlenstoffatomen,
für gegebenenfalls durch Halogen, Cycloalkyl, Cyano, Phenyl und/oder Heterocyclyl substituiertes Alkoxy mit 1 bis 7 Kohlenstoffatomen,
für gegebenenfalls durch Halogen, Cycloalkyl, Cyano, Phenyl und/oder Heterocyclyl substituiertes Alkenyloxy mit 2 bis 6 Kohlenstoffatomen,
für gegebenenfalls durch Halogen, Cycloalkyl, Cyano, Phenyl und/oder Heterocyclyl substituiertes Alkinyloxy mit 2 bis 6 Kohlenstoffatomen,
für gegebenenfalls durch Halogen, Cycloalkyl, Cyano, Phenyl und/oder Heterocyclyl substituiertes Cycloalkyloxy mit 3 bis 7 Kohlenstoffatomen,
für gegebenenfalls durch Halogen, Cycloalkyl, Cyano, Phenyl und/oder Heterocyclyl substituiertes Alkylamino mit 1 bis 7 Kohlenstoffatomen,
für gegebenenfalls durch Halogen, Cycloalkyl, Cyano, Phenyl und/oder Heterocyclyl substituiertes Dialkylamino mit 1 bis 7 Kohlenstoffatomen in jedem der Alkylreste,
für gegebenenfalls durch Halogen, Cycloalkyl, Cyano, Phenyl und/oder Heterocyclyl substituiertes Alkenylamino mit 2 bis 6 Kohlenstoffatomen,
für gegebenenfalls durch Halogen, Cycloalkyl, Cyano, Phenyl und/oder Heterocyclyl substituiertes Alkinylamino mit 2 bis 6 Kohlenstoffatomen,
für gegebenenfalls durch Halogen, Cycloalkyl, Cyano, Phenyl und/oder Heterocyclyl substituiertes Cycloalkylamino mit 3 bis 7 Kohlenstoffatomen,
für gegebenenfalls durch Halogen, Cycloalkyl, Cyano, Phenyl und/oder Heterocyclyl substituiertes N-Cycloalkyl-N-alkyl-amino mit 3 bis 7 Kohlenstoffatomen im Cycloalkylteil und 1 bis 7 Kohlenstoffatomen in Alkylteil,
für gegebenenfalls durch Halogen, Cycloalkyl, Cyano, Phenyl und/oder Heterocyclyl substituiertes Alkylidenamino mit 2 bis 6 Kohlenstoffatomen,
oder
für gegebenenfalls durch Halogen, Alkyl, Cycloalkyl, Cyano, Phenyl und/oder Heterocyclyl substituiertes Heterocyclyl mit 5 oder 6 Ringgliedern steht,
wobei die zuvor genannten Heterocyclyl-Reste einfach bis dreifach, gleichartig oder verschieden substituiert sein können durch
Halogen, Hydroxy, Phenyl, 1,2-Dioxyethylen, Alkyl mit 1 bis 4 Kohlenstoffatomen, Halogenalkyl mit 1 oder 2 Kohlenstoffatomen und 1 bis 5 Halogenatomen, Alkoxy mit 1 bis 4 Kohlenstoffatomen, Alkylthio mit 1 bis 4 Kohlenstoffatomen, Halogenalkoxy mit 1 oder 2 Kohlenstoffatomen und 1 bis 5 Halogenatomen, Halogenalkylthio mit 1 oder 2 Kohlenstoffatomen und 1 bis 5 Halogenatomen und wobei die zuvor genannten Heterocyclylreste gesättigt oder teilweise ungesättigt sind,
und wobei die zuvor genannten Phenyl-Reste einfach bis dreifach, gleichartig oder verschieden substituiert sein können durch
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
in 2,3-Position verknüpftes 1,3-Propandiyl, 1,4-Butandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen.
R² für Wasserstoff,
für gegebenenfalls durch Halogen, Cycloalkyl mit 3 bis 6 Kohlenstoffatomen Alkoxy mit 1 bis 4 Kohlenstoffatomen, Alkylthio mit 1 bis 4 Kohlenstoffatomen, Oxo, Hydroximino und/oder Alkoximino mit 1 bis 4 Kohlenstoffatomen substituiertes Alkyl mit 1 bis 4 Kohlenstoffatomen,
für gegebenenfalls durch Halogen und/oder Cycloalkyl mit 3 bis 6 Kohlenstoffatomen substituiertes Alkenyl mit 2 bis 4 Kohlenstoffatomen,
für gegebenenfalls durch Halogen und/oder Cycloalkyl mit 3 bis 6 Kohlenstoffatomen substituiertes Alkinyl mit 2 bis 4 Kohlenstoffatomen oder
für gegebenenfalls durch Halogen und/oder Cycloalkyl mit 3 bis 6 Kohlenstoffatomen substituiertes Cycloalkyl mit 3 bis 6 Kohlenstoffatomen steht;
oder
R¹ und R² gemeinsam mit dem Stickstoffatom, an das sie gebunden sind, für einen 3- bis 6-gliedrigen heterocyclischen Ring stehen, der gesättigt oder teilweise gesättigt ist, der neben dem bereits erwähnten Stickstoffatom noch ein weiteres Heteroatom aus der Reihe Stickstoff, Sauerstoff und Schwefel enthalten kann und der einfach bis dreifach, gleichartig oder verschieden substituiert sein kann durch
Halogen, Hydroxy, Cyano, Morpholinyl, Amino, einen annelierten Phenylring, eine Methylen- oder Ethylenbrücke,
Alkyl mit 1 bis 4 Kohlenstoffatomen,
Halogenalkyl mit 1 bis 4 Kohlenstoffatomen und 1 bis 9 gleichen oder verschiedenen Halogenatomen,
Alkylcarbonylamino mit 1 bis 4 Kohlenstoffatomen im Alkylteil,
Dialkylamino mit 2 bis 8 Kohlenstoffatomen,
Alkoxycarbonylamino mit 1 bis 4 Kohlenstoffatomen im Alkoxyteil,
Di(alkoxycarbonyl)amino mit 2 bis 8 Kohlenstoffatomen in den Alkoxyteilen,
Hydroxyalkyl mit 1 bis 4 Kohlenstoffatomen,
Alkoxycarbonyl mit 1 bis 4 Kohlenstoffatomen im Alkoxyteil und/oder
Alkylcarbonyl mit 1 bis 4 Kohlenstoffatomen im Alkylteil.
R³ für Phenyl steht, das einfach bis vierfach, gleichartig oder verschieden substituiert sein kann durch
Halogen, Cyano, Nitro, Amino, Hydroxy, Formyl, Carboxy, Carbamoyl, Thiocarbamoyl;
jeweils geradkettiges oder verzweigtes Alkyl, Alkoxy, Alkylthio, Alkylsulfinyl oder Alkylsulfonyl mit jeweils 1 bis 6 Kohlenstoffatomen;
jeweils geradkettiges oder verzweigtes Alkenyl oder Alkenyloxy mit jeweils 2 bis 6 Kohlenstoffatomen;
jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen;
jeweils geradkettiges oder verzweigtes Halogenalkenyl oder Halogenalkenyloxy mit jeweils 2 bis 6 Kohlenstoffatomen und 1 bis 11 gleichen oder verschiedenen Halogenatomen;
jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen;
Cycloalkyl mit 3 bis 6 Kohlenstoffatomen,
in 2,3-Position verknüpftes 1,3-Propandiyl, 1,4-Butandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Halogen, Alkyl mit 1 bis 4 Kohlenstoffatomen und/oder Halogenalkyl mit 1 bis 4 Kohlenstoffatomen und 1 bis 9 gleichen oder verschiedenen Halogenatomen.
X¹ für Wasserstoff, Fluor, Chlor oder Brom steht.
X² für Cyano, Fluor, Chlor, Brom, Iod, Nitro, Formyl, Halogenalkyl mit 1 bis 4 Kohlenstoffatomen und 1 bis 9 Fluor, Chlor und/oder Bromatomen, Alkyl mit 1 bis 4 Kohlenstoffatomen, Cycloalkyl mit 3 bis 6 Kohlenstoffatomen, Thiocarbamoyl, Alkoxycarbonyl mit 1 bis 4 Kohlenstoffatomen im Alkoxyteil, Alkylcarbonyl mit 1 bis 4 Kohlenstoffatomen im Alkylteil, Hydroximinoalkyl mit 1 bis 4 Kohlenstoffatomen im Alkylteil oder für Alkoxyiminoalkyl mit 1 bis 4 Kohlenstoffatomen im Alkoxyteil und 1 bis 4 Kohlenstoffatomen im Alkylteil steht,
sowie Säure-Additionssalze von denjenigen Verbindungen der Formel (I), in denen
R¹ für Amino steht.

2. Pyrazolopyrimidine der Formel 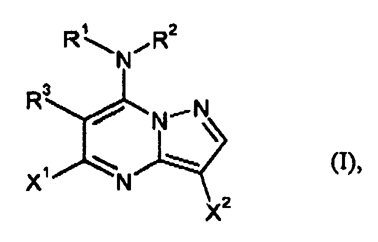 in welcher
R¹ für Hydroxy, Amino, Methyl, Ethyl, n-Propyl, i-Propyl, n-Butyl, i-Butyl, s-Butyl, t-Butyl, n-Pentyl, i-Pentyl, 1,2-Dimethyl-propyl, 2,2-Dimethylpropyl, 1,2,2-Trimethylpropyl, oder
R¹ für Methoxymethyl, 2-Methoxy-ethyl, Methylthiomethyl, 2-Methylthio-ethyl, Hydroximinomethyl, Methoximinomethyl, Acetylmethyl, 2-Hydroximino-propyl, 2-Methoximino-propyl, Allyl, 2-Methyl-prop-2-enyl, Propargyl, 2,2,2-Trifluorethyl, 1-(Trifluormethyl)-ethyl, 3,3,3-Trifluorpropyl, Cyclopropylmethyl, Cyclopropyloxy, Cyclobutyloxy, Cyclopentyloxy, Cyclohexyloxy, Difluormethoxy, Trifluormethoxy, Difluorchlormethoxy, Trifluorethoxy, Methylamino, Ethylamino, n- oder i-Propylamino, n-, i-, s- oder t-Butylamino, Dimethylamino, Diethylamino, Trifluorethylamino, Cyclohexylmethylamino, 2-Cyanoethylamino, Allylamino, 1-Cyclopropylethylamino, Cyclopropylamino, Cyclobutylamino, Cyclopentylamino, Cyclohexylamino, 1-Methylethylidenamino,
für jeweils gegebenenfalls einfach oder zweifach, gleichartig oder verschieden durch Fluor, Chlor und/oder Methyl substituiertes Cyclopropyl, Cyclobutyl, Cyclopentyl, Cyclohexyl, Pyrrolidinyl, Piperidinyl, Morpholinyl, Thiamorpholinyl, Piperazinyl oder
für gegebenenfalls substituiertes Pyridylmethyloxy oder Thiazolylmethoxy,
oder
R¹ für (2,2-Dichlorcyclopropyl)methyl, (2-Furyl)methyl, (2-Tetrahydrofuryl)methyl, (2-Tetrahydropyranyl)methyl, 1,3-Dioxolan-2-ylmethyl, 1-Cyclopropylethyl, Benzyloxy, 2,4-Dichlorbenzyloxy, 2,6-Dichlorbenzyloxy, 2-Chlorbenzyloxy, 2-Fluorcyclopropyl, 2-Hexahydropyranyloxy, 2-Thienylmethyl, 2-Trifluormethylcyclohexyl, 3-(Dimethylamino)-propyl, 3,5-bis-Trifluormethylcyclehexyl, 3,5-Dichlorbenzyloxy, 3-Aminopropyl, 3-Chlorbenzyloxy, 3-Trifluormethylbenzyloxy, 3-Trifluormethylcyclohexyl, 4-Trifluormethylcyclohexyl, 4-Chlorbenzyloxy, 4-Fluorbenzyloxy, 4-Trifluormethylbenzyloxy, -C(CH₃)₂-CF₃, -C(CH₃)₂-CH₂-COCH₃, -CH(CH₂OH)-COOCH₃, -CH(CH₃)-CH(O-CH₃)₂, -CH(CH₃)-CH=CH₂, -CH(CH₃)-CH₂-CH(CH₃)₂, -CH(CH₃)-CH₂-O-CH₃, -CH(CH₃)-CH₂-OH, -CH(CH₃)-COOCH₃, -CH(CH₃)-COO-t-butyl, -CH₂-C(CH₃) =CH₂, -CH₂-CH(OCH₃)₂, -CH₂-CH₂-CF₃, -CH₂-CH₂-Cl, -CH₂-CH₂-CN, -CH₂-CH₂-N(CH₃)₂, -CH₂-CH₂-N(CH₃)₂, -CH₂-CH₂-NH₂, -CH₂-CHF₂, -CH₂-CN, -CH₂-COOC₂H₅, -CH₂-COOCH₃, i-Butoxy, -NH-CH₂-CF₂-CHF₂, -NH-CH₂-CF₃, -NH-CH₂-CH(CH₃)₂, Methoxy, Ethoxy, i-Propoxy, t-Butoxy oder -O-CH(CH₃)-CH₂-CH₃ steht.
wobei die zuvor genannten Thiazolyl- und Pyridyl-Reste im Falle von Thiazolyl einfach oder zweifach und im Falle von Pyridyl einfach bis dreifach, jeweils gleichartig oder verschieden substituiert sein können durch Fluor, Chlor, Brom, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, n- oder i-Propylthio, Difluormethoxy, Trifluormethoxy, Difluorchlormethoxy, Trifluorethoxy, Difluormethylthio, Difluorchlormethylthio, Dichlorfluormethylthio, Trifluormethylthio und/oder Phenyl,
und wobei die zuvor genannten Benzyloxy-Reste im Phenylteil einfach bis dreifach, gleichartig oder verschieden substituiert sein können durch
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfnyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
in 2,3-Position verknüpftes 1,3-Propandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl und/oder Trifluormethyl.
R² für Wasserstoff, Methyl, Ethyl, n- oder i-Propyl, n-, i-, s- oder t-Butyl, Methoxymethyl, 2-Methoxy-ethyl, Methylthiomethyl, 2-Methylthio-ethyl, Hydroximinomethyl, Methoximinomethyl, Acetylmethyl, 2-Hydroxyimino-propyl, 2-Methoxyimino-propyl, Allyl, Propargyl, 2,2,2-Trifluorethyl, 1-(1,1,1-Trifluormethyl)ethyl, Cyclopropylmethyl, Cyclobutylmethyl, Cyclopentylmethyl oder Cyclohexylmethyl steh, oder
R¹ und R² gemeinsam mit dem Stickstoffatom, an welches sie gebunden sind, für 1-Pyrrolinyl, 3-Pyrrolinyl, Pyrrolidinyl, Dihydropyridinyl, Piperidinyl, Pyrazolinyl, Pyrazolidinyl, Imidazolinyl, Imidiazolidinyl, 1,2-Diazinan-yl, 1,3-Diazinan-yl, Piperazinyl, Oxazolinyl, Oxazolidinyl, Isoxazolyl, Isoxazolidinyl, Tetrahydropyridazinyl, Dihydrooxazinyl, Morpholinyl, Thiazolinyl, Thiazolidinyl oder Thiomorpholinyl stehen, wobei die genannten Heterocyclen substituiert sein können durch
Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Methylamino, Ethylamino, n- oder i-Propylamino, Dimethylamino, Diethylamino, Acetyl, Propionyl, Acetyloxa, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl, Cyclohexyl,
durch einen annelierten Phenylring oder
durch eine Methandiyl- oder Ethandiyl-Brücke,
oder
R¹ und R² gemeinsam für eine Gruppierung der Formel stehen 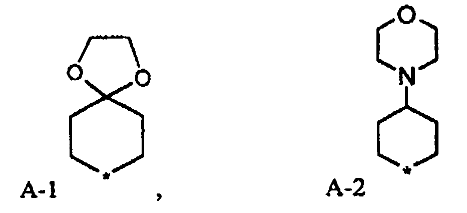 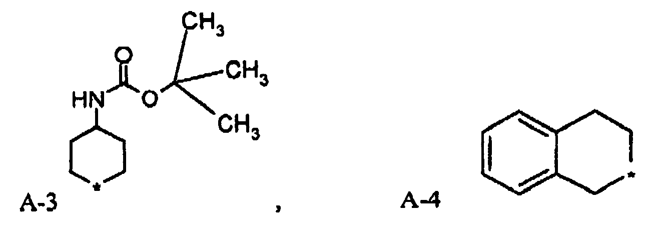 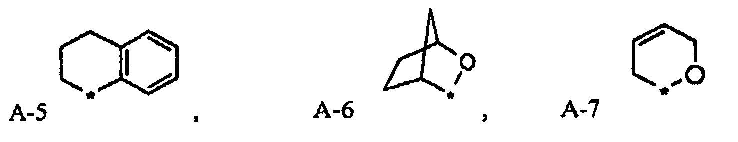 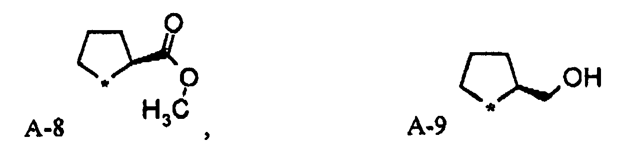 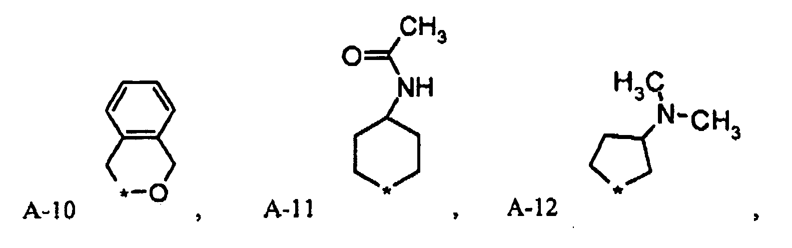 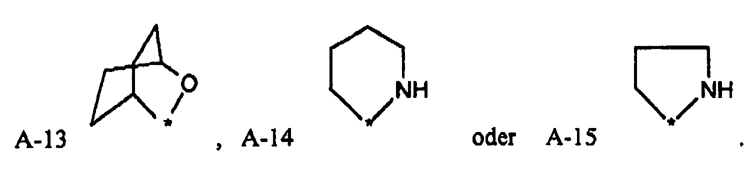
In diesen Gruppierungen ist die mit dem Stickstoffatom verbundene Stelle jeweils durch * bezeichnet.
R³ für Phenyl steht, das einfach bis dreifach, gleichartig oder verschieden substituiert sein kann durch
Fluor, Chlor, Brom, Cyano, Nitro, Formyl, Methyl, Ethyl, n- oder i-Propyl, n-, i-, s- oder t-Butyl, Allyl, Propargyl, Methoxy, Ethoxy, n- oder i-Propoxy, Methylthio, Ethylthio, n- oder i-Propylthio, Methylsulfinyl, Ethylsulfinyl, Methylsulfonyl, Ethylsulfonyl, Allyloxy, Propargyloxy, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Trichlorethinyloxy, Trifluorethinyloxy, Chlorallyloxy, Iodpropargyloxy, Methylamino, Ethylamino, n- oder i-Propylamino, Dimethylamino, Diethylamino, Acetyl, Propionyl, Acetyloxy, Methoxycarbonyl, Ethoxycarbonyl, Hydroximinomethyl, Hydroximinoethyl, Methoximinomethyl, Ethoximinomethyl, Methoximinoethyl, Ethoximinoethyl, Cyclopropyl, Cyclobutyl, Cyclopentyl oder Cyclohexyl,
in 2,3-Position verknüpftes 1,3-Propandiyl, Methylendioxy (-O-CH₂-O-) oder 1,2-Ethylendioxy (-O-CH₂-CH₂-O-), wobei diese Reste einfach oder mehrfach, gleichartig oder verschieden substituiert sein können durch Fluor, Chlor, Methyl, Ethyl, n-Propyl, i-Propyl und/oder Trifluormethyl.
X¹ für Wasserstoff, Fluor oder Chlor steht
X² für Cyano, Fluor, Chlor, Brom, Iod, Formyl, Trifluormethyl, Methoxycarbonyl, Methylcarbonyl, Hydroximinomethyl, Methoximinomethyl, Thiocarbamoyl, Nitro, Methyl, Ethyl oder Cyclopropyl steht.

3. Pyrazolopyrimidine der Formel (I) gemäss einem oder mehreren der Ansprüche 1 oder 2, wobei
R³ für 2,4-, 2,5- oder 2,6-disubstituiertes Phenyl, oder für 2-substituiertes Phenyl oder für 2,4,6-trisubstituiertes Phenyl steht;

4. Pyrazolopyrimidine der Formel (I) gemäss einem oder mehreren der Ansprüche 1, 2 oder 3, wobei
R¹ für Amino, Hydroxy, Methyl, Ethyl, n-Propyl, i-Propyl, n-Butyl, i-Butyl, s-Butyl, t-Butyl, 1,2-Dimethyl-propyl, 1,2,2-Trimethylpropyl, 2,2-Dimethyl-propyl, Trifluormethyl, 2,2,2-Trifluormethyl, 2,2-Difluorethyl, 2,2,2-Trifluor-1-methyl-ethyl, 3,3,3-Trifluor-propyl, 2,2,2-Trifluor-1,1-dimethyl-ethyl, 3-Methyl-butyl, Allyl, 2-Methyl-prop-2-enyl, 2-Methoxyethyl, 2,2-Dimethoxy-ethyl, Cyclopropyl, Cyclopentyl, Cyclohexyl, 2-Fluorcyclopropyl, 2-Trifluormethyl-cyclohexyl, 3-Trifluormethyl-cyclohexyl, 4-Trifluormethyl-cyclohexyl, 3,5-Di(trifluormethyl)cyclohexyl, Cyclopropyl-methyl, Dichlorcyclopropyl-methyl, 1-Cyclohexyl-ethyl, 2-Furyl-methyl, 2-Tetrahydrofuryl-methyl, 2-Thienyl-methyl, 1,3-Dioxolan-2-yl-methyl, Propargyl, Methoxycarbonylmethyl, Ethoxycarbonylmethyl, 2-Amino-ethyl, 3-Amino-propyl, 2-Dimethylamino-ethyl, Cyanomethyl, 2-Cyano-ethyl, 2-Vinyloxy-ethyl, Pyrrolidinyl, Piperidinyl, Morpholinyl, Thiomorpholinyl oder Piperazinyl steht,
R² für Wasserstoff, Methyl, Ethyl, n- oder i-Propyl, n-, i-, s- oder t-Butyl, Allyl, Propargyl, 2,2,2-Trifluorethyl, 1-(1,1,1-Trifluormethyl)ethyl, Cyclopropylmethyl, Cyclobutylmethyl, Cyclopentylmethyl, Cyclohexylmethyl oder Cyclopropyl steht oder
R¹ und R² gemeinsam mit dem Stickstoffatom, an das sie gebunden sind, für jeweils gegebenenfalls einfach oder zweifach, gleichartig oder verschieden durch Fluor, Chlor, Brom, Cyano, Hydroxy, Methyl, Ethyl, Trifluormethyl, Methylcarbonyl, Methylcarbonylamino oder Methoxycarbonyl substituiertes Pyrrolidinyl, Piperidinyl, Morpholinyl, Thiomorpholinyl, Piperazinyl, 5-Methyl-3,6-dihydro-1(2H)-pyridinyl, 5-Ethyl-3,6-dihydro-1-(2H)-pyridinyl oder Tetrahydro-1-(2H)-pyridazinyl stehen oder für eine Gruppierung der Formel 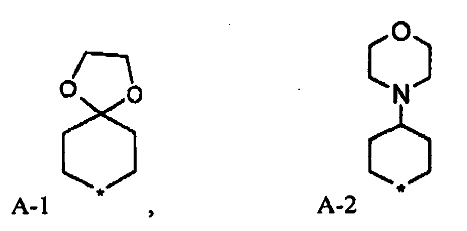 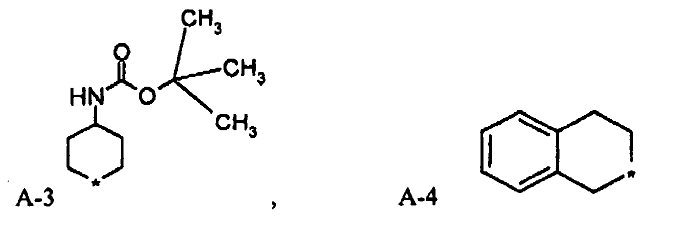 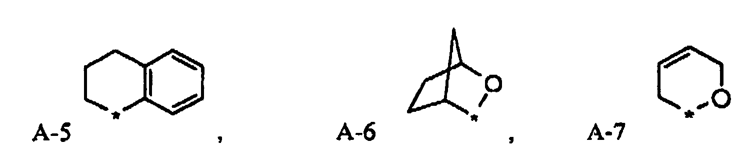 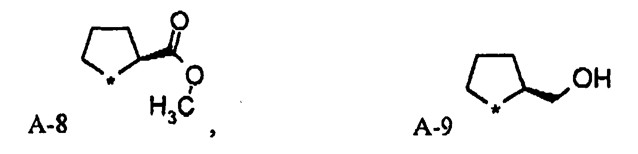 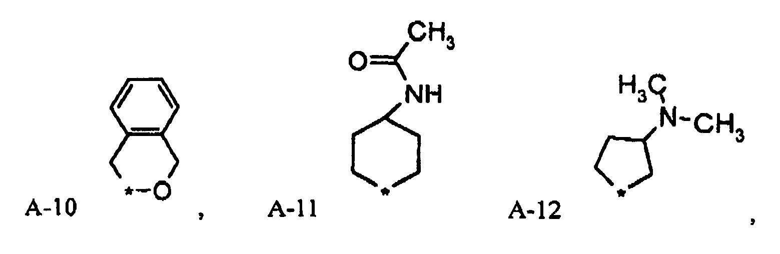 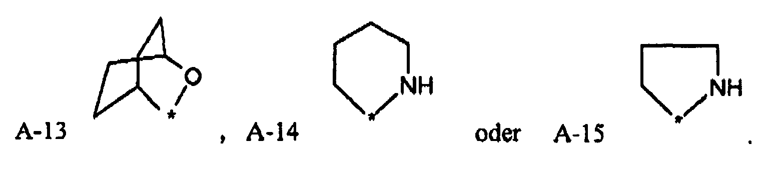 stehen,
R³ für einfach bis dreifach in 2-, 4- und/oder 6-Position durch Fluor und/oder Chlor substituiertes Phenyl steht,
oder
R³ für 2-Trifluormethylphenyl, 2-Chlor-5-nitro-phenyl oder 2-Chlor-4-methoxyphenyl steht,
X¹ für Wasserstoff oder Chlor steht und
X² für Fluor, Chlor, Brom, Iod, Cyano, Nitro, Methyl, Cyclopropyl, Formyl, Thiocarbamoyl oder Methoximinomethyl steht.

5. Verfahren zur Herstellung von Pyrazolopyrimidinen der Formel (I) gemäß einem oder mehreren der Ansprüche 1 bis 4, **dadurch gekennzeichnet, dass** man
a) Halogen-pyrazolopyrimidine der Formel 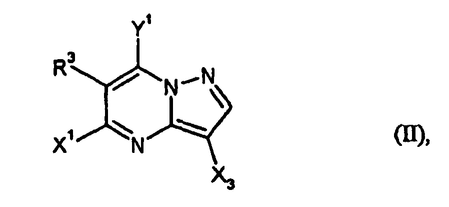 in welcher
R³ und X¹ die oben angegebenen Bedeutungen haben,
X³ für Halogen, Cyano, Nitro, Alkyl, Halogenalkyl, Cycloalkyl, Thiocarbamoyl, Alkoxycarbonyl oder Alkylcarbonyl steht und
Y¹ für Halogen steht,
mit Aminen der Formel 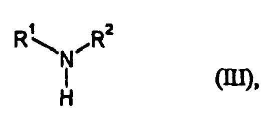 in welcher
R¹ und R² die oben angegebenen Bedeutungen haben,
gegebenenfalls in Gegenwart eines Verdünnungsmittels, gegebenenfalls in Gegenwart eines Katalysators und gegebenenfalls in Gegenwart eines Säureakzeptors, umsetzt,
oder
b) Pyrazolopyrimidine der Formel 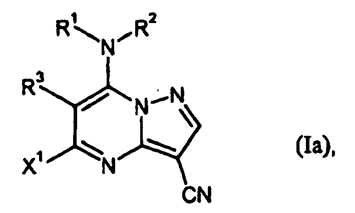 in welcher
R¹, R², R³ und X¹ die oben angegebenen Bedeutungen haben,
mit Diisobutyl-aluminiumhydrid, in Gegenwart von wässriger Ammoniumchlorid-Lösung sowie in Gegenwart eines organischen Verdünnungsmittels umsetzt,
oder
c) Pyrazolopyrimidine der Formel 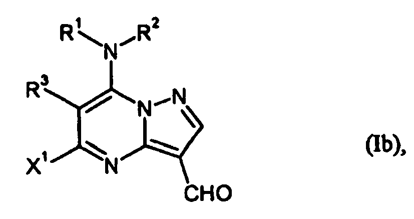 in welcher
R¹, R², R³ und X¹ die oben angegebenen Bedeutungen haben,
mit Amino-Verbindungen der Formel
H₂N-OR⁴ (IV),
in welcher
R⁴ für Wasserstoff oder Alkyl steht,
in Gegenwart eines Verdünnungsmittels und gegebenenfalls in Gegenwart eines Katalysators umsetzt, wobei die Amino-Verbindungen der Formel (IV) auch in Form ihrer Säureadditions-Salze eingesetzt werden können,
und gegebenenfalls an die so erhaltenen Verbindungen der Formel (I), in denen
R¹ für Amino steht,
eine Säure addiert.

6. Mittel zur Bekämpfung von Schadorganismen, **gekennzeichnet durch** einen Gehalt an mindestens einem Pyrazolopyrimidin der Formel (I) gemäß einem oder mehreren der Ansprüche 1 bis 4 bzw. an einem Saureadditions-Salz davon neben Streckmitteln und/oder oberflächenaktiven Stoffen.

7. Verwendung von Pyrazolopyrimidinen der Formel (I) gemäß einem oder mehreren der Ansprüche 1 bis 4 bzw. von deren Säureadditions-Salzen zur Bekämpfung von Schadorganismen im Pflanzen - und Materialschutz

8. Verfahren zur Bekämpfung von Schadorganismen, **dadurch gekennzeichnet, dass** man Pyrazolopyrimidine der Formel (I) gemäß einem oder mehreren der Ansprüche 1 bis 4 bzw. deren Säureadditions-Salze auf die Schadorganismen und/oder deren Lebensraum ausbringt.

9. Verfahren zur Herstellung von Mitteln zur Bekämpfung von Schadorganismen, **dadurch gekennzeichnet, dass** man Pyrazolopyrimidine der Formel (I) gemäß einem oder mehreren der Ansprüche 1 bis 4 bzw. deren Säureadditions-Salze mit Streckmitteln und/oder oberflächenaktiven Stoffen vermischt.

10. Mittel zur Bekämpfung von Schädlingen, **gekennzeichnet durch** einen Gehalt an mindestens einem Pyrazolopyrimidin der Formel (1) gemäß einem oder mehreren der Ansprüche 1 bis 4 bzw. an einem Säureadditions-Salz davon neben Streckmitteln und/oder oberflächenaktiven Stoffen.

11. Verwendung von Pyrazolopyrimidinen der Formel (I) gemäß einem oder mehreren der Ansprüche 1 bis 4 bzw. von deren Säureadditions-Salzen zur Bekämpfung von Schädlingen.

12. Verfahren zur Bekämpfung von Schädlingen, **dadurch gekennzeichnet, dass** man Pyrazolopyrimidine der Formel (I) gemäß einem oder mehreren der Ansprüche 1 bis 4 bzw. deren Säureadditions-Salze auf Schädlinge und/oder deren Lebensraum ausbringt.

13. Verfahren zur Herstellung von Mitteln zur Bekämpfung von Schädlingen, **dadurch gekennzeichnet, dass** man Pyrazolopyrimidine der Formel (I) gemäß einem oder mehreren der Ansprüche 1 bis 4 bzw. deren Säureadditions-Salze mit Streckmitteln und/oder oberflächenaktiven Stoffen vermischt.

14. Halogen-pyrazolopyrimidine der Formel 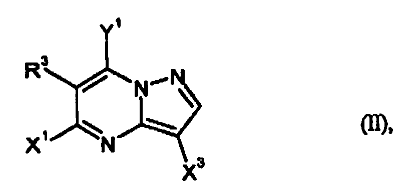 in welcher
R³ die im Anspruch 1 angegebene Bedeutung hat
X¹ für Wasserstoff oder Halogen steht und
X³ für Halogen, Cyano, Nitro, Alkyl, Thiocarbamoyl, Cycloalkyl, Halogenalkyl, Alkoxycarbonyl oder Alkylcarbonyl steht und
Y¹ für Halogen steht.

15. Verfahren zur Herstellung von Halogen-pyrazolopyrimidinen der Formel (II) gemäß Anspruch 14, **dadurch gekennzeichnet, dass** man
d) Hydroxy-pyrazolopyrimidine der Formel 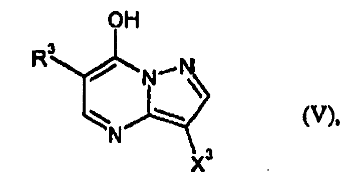 in welcher
R³ und X³ die oben angegebenen Bedeutungen haben,
mit Halogenierungsmittein, gegebenenfalls in Gegenwart eines Verdünnungsmittels umsetzt,
oder
e) Dihydroxy-pyrazolopyrimidine der Formel 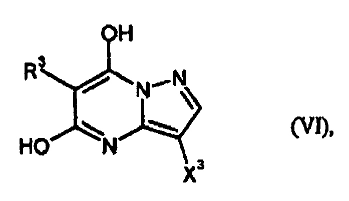 in welcher
R³ und X³ die oben angegebenen Bedeutungen haben,
mit Halogenierungsmitteln, gegebenenfalls in Gegenwart eines Verdünnungsmittels umsetzt.

16. Hydroxy-pyrazolopyrimidine der Formel 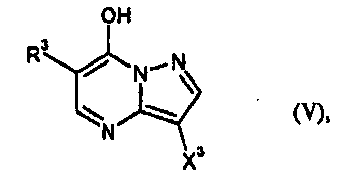 in welcher
R³ die im Anspruch 1 angegebene Bedeutung hat
X3 für Halogen, Cyano, Nitro, Alkyl. Thiocarbamoyl, Cycloalkyl, Halogenalkyl, Alkoxycarbonyl oder Alkylcarbonyl steht

17. Verfahren zur Herstellung von Hydroxy-pyrazolopyrimidinen der Formel (V) gemäß Anspruch 16, **dadurch gekennzeichnet, dass** man
f) Acrylsäureester der Formel 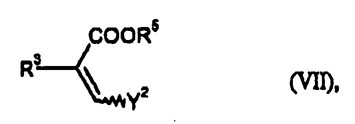 in welcher
R³ die oben angegebene Bedeutung hat,
R⁵ für Alkyl steht und
Y² für Alkoxy oder Dialkylamino steht,
mit Aminopyrazolen der Formel 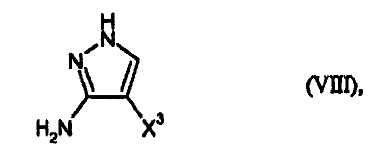 in welcher
X³ die oben angegebene Bedeutung hat,
gegebenenfalls in Gegenwart eines Verdünnungsmittels und gegebenenfalls in Gegenwart einer Base umsetzt.

18. Dihydroxy-pyrazolopyrimidine der Formel 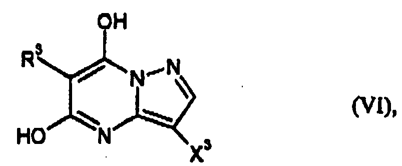 in welcher
R³ die im Anspruch 1 angegebene Bedeutung hat
X³ für Halogen, Cyano, Nitro, Alkyl, Thiocarbamoyl, Cycloalkyl, Halogenalkyl, Alkoxycarbonyl oder Alkylcarbonyl steht.

19. Verfahren zur Herstellung von Dihydroxy-pyrazolopyrimidinen der Formel (VI) gemäß Anspruch 18, **dadurch gekennzeichnet dass** man
g) Malonester der Formel 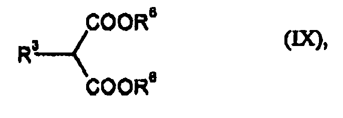
in welcher
R³ die oben angegebenen Bedeutung hat und
R6 für Alkyl steht,
mit Aminopyrazolen der Formel 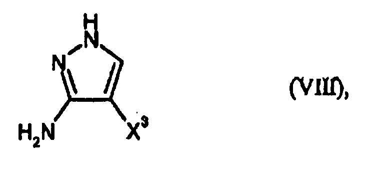 in welcher
X³ die oben angegebene Bedeutung hat,
gegebenenfalls in Gegenwart eines Verdünnungsmittels und gegebenenfalls in Gegenwart einer starken Base umsetzt.

## Claims

1. Pyrazolopyrimidines of the formula 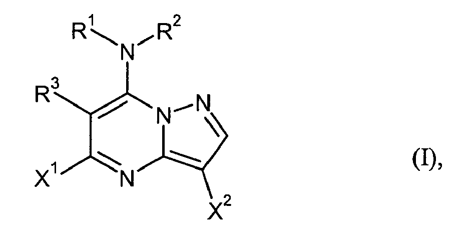 in which
R¹ represents hydroxyl, amino, represents alkyl having 1 to 6 carbon atoms which is optionally substituted by halogen, cyano, hydroxyl, amino, phenyl, heterocyclyl, alkoxy having 1 to 4 carbon atoms, alkoxycarbonyl having 1 to 4 carbon atoms in the alkoxy moiety, alkylamino having 1 to 4 carbon atoms, dialkylamino having 2 to 8 carbon atoms, cycloalkyl having 3 to 6 carbon atoms, halocycloalkyl having 3 to 6 carbon atoms and 1 to 5 halogen atoms, alkylthio having 1 to 4 carbon atoms, oxo, hydroxyimino and/or alkoximino having 1 to 4 carbon atoms,
represents optionally halogen-, cycloalkyl-, cyano-, phenyl- and/or heterocyclyl-substituted alkenyl having 2 to 6 carbon atoms,
represents optionally halogen-, cycloalkyl-, cyano-, phenyl- and/or heterocyclyl-substituted alkynyl having 2 to 6 carbon atoms,
represents optionally cycloalkyl having 3 to 7 carbon atoms which is optionally substituted by halogen, cycloalkyl, cyano, haloalkyl having 1 to 2 carbon atoms and 1 to 5 halogen atoms, phenyl and/or heterocyclyl,
represents optionally halogen-, cycloalkyl-, cyano-, phenyl- and/or heterocyclyl-substituted alkoxy having 1 to 7 carbon atoms,
represents optionally halogen-, cycloalkyl-, cyano-, phenyl- and/or heterocyclyl-substituted alkenyloxy having 2 to 6 carbon atoms,
represents optionally halogen-, cycloalkyl-, cyano-, phenyl- and/or heterocyclyl-substituted alkynyloxy having 2 to 6 carbon atoms,
represents optionally halogen-, cycloalkyl-, cyano-, phenyl- and/or heterocyclyl-substituted cycloalkyloxy having 3 to 7 carbon atoms,
represents optionally halogen-, cycloalkyl-, cyano-, phenyl- and/or heterocyclyl-substituted alkylamino having 1 to 7 carbon atoms,
represents optionally halogen-, cycloalkyl-, cyano-, phenyl- and/or heterocyclyl-substituted dialkylamino having 1 to 7 carbon atoms in each of the alkyl radicals,
represents optionally halogen-, cycloalkyl-, cyano-, phenyl- and/or heterocyclyl-substituted alkenylamino having 2 to 6 carbon atoms,
represents optionally halogen-, cycloalkyl-, cyano-, phenyl- and/or heterocyclyl-substituted alkynylamino having 2 to 6 carbon atoms,
represents optionally halogen-, cycloalkyl-, cyano-, phenyl- and/or heterocyclyl-substituted cycloalkylamino having 3 to 7 carbon atoms,
represents optionally halogen-, cycloalkyl-, cyano-, phenyl- and/or heterocyclyl-substituted N-cycloalkyl-N-alkylamino having 3 to 7 carbon atoms in the cycloalkyl moiety and 1 to 7 carbon atoms in the alkyl moiety,
represents optionally halogen-, cycloalkyl-, cyano-, phenyl- and/or heterocyclyl-substituted alkylideneamino having 2 to 6 carbon atoms,
or
represents optionally halogen-, alkyl-, cycloalkyl-, cyano-, phenyl- and/or heterocyclyl-substituted heterocyclyl having 5 or 6 ring members,
where the heterocyclyl radicals mentioned above may be mono- to trisubstituted by identical or different substituents from the group consisting of
halogen, hydroxyl, phenyl, 1,2-dioxyethylene, alkyl having 1 to 4 carbon atoms, haloalkyl having 1 or 2 carbon atoms and 1 to 5 halogen atoms, alkoxy having 1 to 4 carbon atoms, alkylthio having 1 to 4 carbon atoms, haloalkoxy having 1 or 2 carbon atoms and 1 to 5 halogen atoms, haloalkylthio having 1 or 2 carbon atoms and 1 to 5 halogen atoms, where the heterocyclyl radicals mentioned above are saturated or partially unsaturated,
and where the phenyl radicals mentioned above may be mono- to trisubstituted by identical or different substituents from the group consisting of
halogen, cyano, nitro, amino, hydroxyl, formyl, carboxyl, carbamoyl, thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched haloalkyl, haloalkoxy, haloalkylthio, haloalkylsulphinyl or haloalkylsulphonyl having in each case 1 to 6 carbon atoms and 1 to 13 identical or different halogen atoms;
in each case straight-chain or branched haloalkenyl or haloalkenyloxy having in each case 2 to 6 carbon atoms and 1 to 13 identical or different halogen atoms;
in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
cycloalkyl having 3 to 6 carbon atoms;
1,3-propanediyl attached in the 2,3-position, 1,4-butanediyl, methylenedioxy (-O-CH₂-O-) or 1,2-ethylenedioxy (-O-CH₂-CH₂-O-), where these radicals may be mono- or polysubstituted by identical or different substituents from the group consisting of halogen, alkyl having 1 to 4 carbon atoms and/or haloalkyl having 1 to 4 carbon atoms and 1 to 9 identical or different halogen atoms.
R² represents hydrogen,
represents alkyl having 1 to 4 carbon atoms which is optionally substituted by halogen, cycloalkyl having 3 to 6 carbon atoms, alkoxy having 1 to 4 carbon atoms, alkylthio having 1 to 4 carbon atoms, oxo, hydroximino and/or alkoximino having 1 to 4 carbon atoms,
represents alkenyl having 2 to 4 carbon atoms which is optionally substituted by halogen and/or cycloalkyl having 3 to 6 carbon atoms,
represents alkynyl having 2 to 4 carbon atoms which is optionally substituted by halogen and/or cycloalkyl having 3 to 6 carbon atoms or
represents cycloalkyl having 3 to 6 carbon atoms which is optionally substituted by halogen and/or cycloalkyl having 3 to 6 carbon atoms;
or
R¹ and R² together with the nitrogen atom to which they are attached represent a 3- to 6-membered heterocyclic ring which is saturated or partially saturated, which, in addition to the nitrogen atom already mentioned, may contain a further heteroatom from the group consisting of nitrogen, oxygen and sulphur and which may be mono- to trisubstituted by identical or different substituents from the group consisting of
halogen, hydroxyl, cyano, morpholinyl, amino, a fused phenyl ring, a methylene or ethylene bridge,
alkyl having 1 to 4 carbon atoms,
haloalkyl having 1 to 4 carbon atoms and 1 to 9 identical or different halogen atoms,
alkylcarbonylamino having 1 to 4 carbon atoms in the alkyl moiety,
dialkylamino having 2 to 8 carbon atoms,
alkoxycarbonylamino having 1 to 4 carbon atoms in the alkoxy moiety,
di(alkoxycarbonyl)amino having 2 to 8 carbon atoms in the alkoxy moieties,
hydroxyalkyl having 1 to 4 carbon atoms,
alkoxycarbonyl having 1 to 4 carbon atoms in the alkoxy moiety and/or
alkylcarbonyl having 1 to 4 carbon atoms in the alkyl moiety.
R³ represents phenyl which may be mono- to tetrasubstituted by identical or different substituents from the group consisting of
halogen, cyano, nitro, amino, hydroxyl, formyl, carboxyl, carbamoyl, thiocarbamoyl;
in each case straight-chain or branched alkyl, alkoxy, alkylthio, alkylsulphinyl or alkylsulphonyl having in each case 1 to 6 carbon atoms;
in each case straight-chain or branched alkenyl or alkenyloxy having in each case 2 to 6 carbon atoms;
in each case straight-chain or branched haloalkyl, haloalkoxy, haloalkylthio, haloalkylsulphinyl or haloalkylsulphonyl having in each case 1 to 6 carbon atoms and 1 to 13 identical or different halogen atoms;
in each case straight-chain or branched haloalkenyl or haloalkenyloxy having in each case 2 to 6 carbon atoms and 1 to 11 identical or different halogen atoms;
in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl having in each case 1 to 6 carbon atoms in the individual alkyl moieties;
cycloalkyl having 3 to 6 carbon atoms,
1,3-propanediyl attached in the 2,3-position, 1,4-butanediyl, methylenedioxy (-O-CH₂-O-) or 1,2-ethylenedioxy (-O-CH₂-CH₂-O-), where these radicals may be mono- or polysubstituted by identical or different substituents from the group consisting of halogen, alkyl having 1 to 4 carbon atoms and/or haloalkyl having 1 to 4 carbon atoms and 1 to 9 identical or different halogen atoms.
X¹ represents hydrogen, fluorine, chlorine or bromine.
X² represents cyano, fluorine, chlorine, bromine, iodine, nitro, formyl, haloalkyl having 1 to 4 carbon atoms and 1 to 9 fluorine, chlorine and/or bromine atoms, alkyl having 1 to 4 carbon atoms, cycloalkyl having 3 to 6 carbon atoms, thiocarbamoyl, alkoxycarbonyl having 1 to 4 carbon atoms in the alkoxy moiety, alkylcarbonyl having 1 to 4 carbon atoms in the alkyl moiety, hydroximinoalkyl having 1 to 4 carbon atoms in the alkyl moiety or represents alkoxyiminoalkyl having 1 to 4 carbon atoms in the alkoxy moiety and 1 to 4 carbon atoms in the alkyl moiety,
and acid addition salts of those compounds of the formula (I),
in which
R¹ represents amino.

2. Pyrazolopyrimidines of the formula 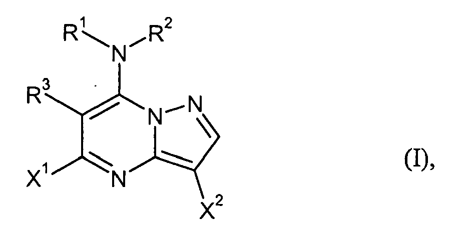 in which
R¹ represents hydroxyl, amino, methyl, ethyl, n-propyl, i-propyl, n-butyl, i-butyl, s-butyl, t-butyl, n-pentyl, i-pentyl, 1,2-dimethylpropyl, 2,2-dimethylpropyl, 1,2,2-trimethylpropyl, or
R¹ represents methoxymethyl, 2-methoxyethyl, methylthiomethyl, 2-methylthioethyl, hydroximinomethyl, methoximinomethyl, acetylmethyl, 2-hydroximinopropyl, 2-methoximinopropyl, allyl, 2-methylprop-2-enyl, propargyl, 2,2,2-trifluoroethyl, 1-(trifluoromethyl)ethyl, 3,3,3-trifluoropropyl, cyclopropylmethyl, cyclopropyloxy, cyclobutyloxy, cyclopentyloxy, cyclohexyloxy, difluoromethoxy, trifluoromethoxy, difluorochloromethoxy, trifluoroethoxy,
methylamino, ethylamino, n- or i-propylamino, n-, i-, s- or t-butylamino, dimethylamino, diethylamino, trifluoroethylamino, cyclohexylmethylamino, 2-cyanoethylamino, allylamino, 1-cyclopropylethylamino, cyclopropylamino, cyclobutylamino, cyclopentylamino, cyclohexylamino, 1-methylethylideneamino,
represents cyclopropyl, cyclobutyl, cyclopentyl, cyclohexyl, pyrrolidinyl, piperidinyl, morpholinyl, thiamorpholinyl, piperazinyl, each of which is optionally mono- or disubstituted by identical or different substituents from the group consisting of fluorine, chlorine and/or methyl, or
represents optionally substituted pyridylmethyloxy or thiazolylmethoxy,
or
R¹ represents (2,2-dichlorocyclopropyl)methyl, (2-furyl)methyl, (2-tetrahydrofuryl)methyl, (2-tetrahydropyranyl)methyl, 1,3-dioxolan-2-ylmethyl, 1-cyclopropylethyl, benzyloxy, 2,4-dichlorobenzyloxy, 2,6-dichlorobenzyloxy, 2-chlorobenzyloxy, 2-fluorocyclopropyl, 2-hexahydropyranyloxy, 2-thienylmethyl, 2-trifluoromethylcyclohexyl, 3-(dimethylamino)propyl, 3,5-bistrifluoromethylcyclohexyl, 3,5-dichlorobenzyloxy, 3-aminopropyl, 3-chlorobenzyloxy, 3-trifluoromethylbenzyloxy, 3-trifluoromethylcyclohexyl, 4-trifluoromethylcyclohexyl, 4-chlorobenzyloxy, 4-fluorobenzyloxy, 4-trifluoromethylbenzyloxy, -C(CH₃)₂-CF₃, -C(CH₃)₂-CH₂-COCH₃, -CH(CH₂OH)-COOCH₃, -CH(CH₃)-CH(O-CH₃)₂, -CH(CH₃)-CH=CH₂, -CH(CH₃)-CH₂-CH(CH₃)₂, -CH(CH₃)-CH₂-O-CH₃, -CH(CH₃)-CH₂-OH, -CH(CH₃)-COOCH₃, -CH(CH₃)-COO-t-butyl, -CH₂-C(CH₃)=CH₂, -CH₂-CH(OCH₃)₂, -CH₂-CH₂-CF₃, -CH₂-CH₂-Cl, -CH₂-CH₂-CN, -CH₂-CH₂-N(CH₃)₂, -CH₂-CH₂-N(CH₃)₂, -CH₂-CH₂-NH₂, -CH₂-CHF₂, -CH₂-CN, -CH₂-COOC₂H₅, -CH₂-COOCH₃, i-butoxy, -NH-CH₂-CF₂-CHF₂, -NH-CH₂-CF₃, -NH-CH₂-CH(CH₃)₂, methoxy, ethoxy, i-propoxy, t-butoxy or -O-CH(CH₃)-CH₂-CH₃,
where the abovementioned thiazolyl and pyridyl radicals may be substituted, in the case of thiazolyl mono- or disubstituted and in the case of pyridyl mono- to trisubstituted, in each case by identical or different substituents from the group consisting of fluorine, chlorine, bromine, methyl, ethyl, n- or i-propyl, n-, i-, s- or t-butyl, methoxy, ethoxy, n- or i-propoxy, n-, i-, s- or t-butoxy, methylthio, ethylthio, n- or i-propylthio, difluoromethoxy, trifluoromethoxy, difluorochloromethoxy, trifluoroethoxy, difluoromethylthio, difluorochloromethylthio, dichlorofluoromethylthio, trifluoromethylthio and/or phenyl,
and where the benzyloxy radicals mentioned above may be mono- to trisubstituted in the phenyl moiety by identical or different substituents from the group consisting of
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl, ethoximinoethyl, cyclopropyl, cyclobutyl, cyclopentyl or cyclohexyl,
1,3-propanediyl attached in the 2,3-position, methylenedioxy (-O-CH₂-O-) or 1,2-ethylenedioxy (-O-CH₂-CH₂-O), where these radicals may be mono- or polysubstituted by identical or different substituents from the group consisting of fluorine, chlorine, methyl, ethyl, n-propyl, i-propyl and/or trifluoromethyl.
R² represents hydrogen, methyl, ethyl, n- or i-propyl, n-, i-, s- or t-butyl, methoxymethyl, 2-methoxyethyl, methylthiomethyl, 2-methylthioethyl, hydroximinomethyl, methoximinomethyl, acetylmethyl, 2-hydroxyiminopropyl, 2-methoxyiminopropyl, allyl, propargyl, 2,2,2-trifluoroethyl, 1-(1,1,1-trifluoromethyl)ethyl, cyclopropylmethyl, cyclobutylmethyl, cyclopentylmethyl or cyclohexylmethyl, or
R¹ and R² together with the nitrogen atom to which they are attached represent 1-pyrrolinyl, 3-pyrrolinyl, pyrrolidinyl, dihydropyridinyl, piperidinyl, pyrazolinyl, pyrazolidinyl, imidazolinyl, imidazolidinyl, 1,2-diazinanyl, 1,3-diazinanyl, piperazinyl, oxazolinyl, oxazolidinyl, isoxazolyl, isoxazolidinyl, tetrahydropyridazinyl, dihydrooxazinyl, morpholinyl, thiazolinyl, thiazolidinyl or thiomorpholinyl, where the heterocycles mentioned may be substituted by
fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, methylamino, ethylamino, n- or i-propylamino, dimethylamino, diethylamino, acetyl, propionyl, acetyloxa, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl, ethoximinoethyl, cyclopropyl, cyclobutyl, cyclopentyl, cyclohexyl,
by a fused phenyl ring or
by a methanediyl or ethanediyl bridge,
or
R¹ and R² together represent a grouping of the formula 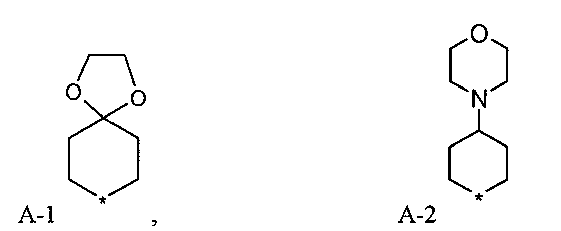 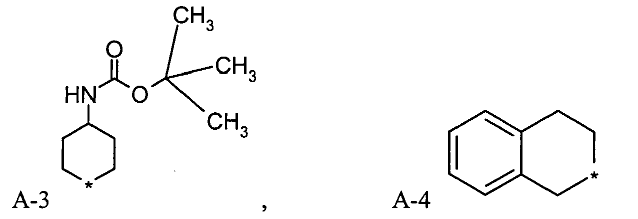 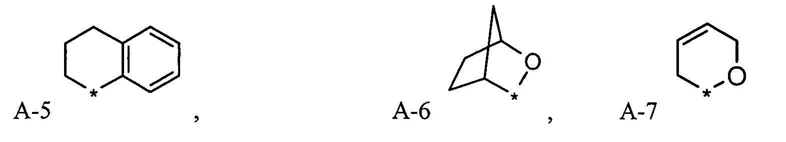 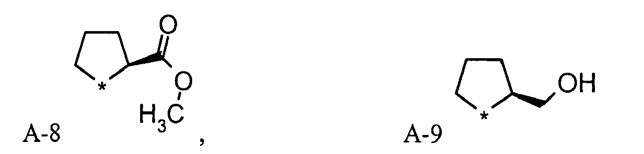 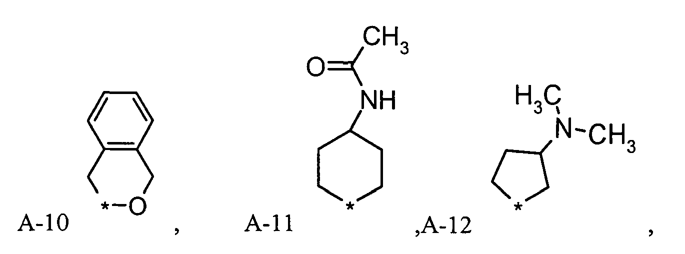 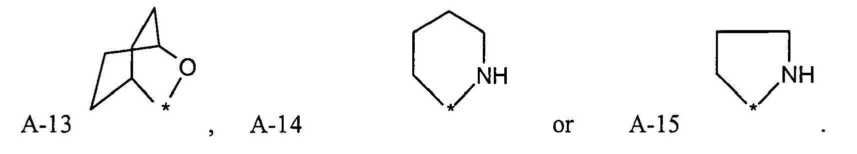
In these groups, the position attached to the nitrogen atom is in each case marked by *.
R³ represents phenyl which is mono- to trisubstituted by identical or different substituents from the group consisting of
fluorine, chlorine, bromine, cyano, nitro, formyl, methyl, ethyl, n- or i-propyl, n-, i-, s- or t-butyl, allyl, propargyl, methoxy, ethoxy, n- or i-propoxy, methylthio, ethylthio, n- or i-propylthio, methylsulphinyl, ethylsulphinyl, methylsulphonyl, ethylsulphonyl, allyloxy, propargyloxy, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, trichloroethynyloxy, trifluoroethynyloxy, chloroallyloxy, iodopropargyloxy, methylamino, ethylamino, n- or i-propylamino, dimethylamino, diethylamino, acetyl, propionyl, acetyloxy, methoxycarbonyl, ethoxycarbonyl, hydroximinomethyl, hydroximinoethyl, methoximinomethyl, ethoximinomethyl, methoximinoethyl, ethoximinoethyl, cyclopropyl, cyclobutyl, cyclopentyl or cyclohexyl,
1,3-propanediyl attached in the 2,3-position, methylenedioxy (-O-CH₂-O-) or 1,2-ethylenedioxy (-O-CH₂-CH₂-O-), where these radicals may be mono- or polysubstituted by identical or different radicals from the group consisting of fluorine, chlorine, methyl, ethyl, n-propyl, i-propyl and/or trifluoromethyl.
X¹ represents hydrogen, fluorine or chlorine.
X² represents cyano, fluorine, chlorine, bromine, iodine, formyl, trifluoromethyl, methoxycarbonyl, methylcarbonyl, hydroximinomethyl or methoximinomethyl, thiocarbamoyl, nitro, methyl, ethyl or cyclopropyl.

3. Pyrazolopyrimidines of the formula (I) according to one or more of Claims 1 or 2, wherein
R³ represents 2,4-, or 2,6-disubstituted phenyl, or represents 2-substituted phenyl or represents 2,4,6-trisubstituted phenyl.

4. Pyrazolopyrimidines of the formula (I) according to one or more of Claims 1, 2 or 3, wherein
R¹ represents amino, hydroxyl, methyl, ethyl, n-propyl, i-propyl, n-butyl, i-butyl, s-butyl, t-butyl, 1,2-dimethylpropyl, 1,2,2-trimethylpropyl, 2,2-dimethylpropyl, trifluoromethyl, 2,2,2-trifluoromethyl, 2,2-difluoroethyl, 2,2,2-trifluoro-1-methylethyl, 3,3,3-trifluoropropyl, 2,2,2-trifluoro-1,1-dimethylethyl, 3-methylbutyl, allyl, 2-methylprop-2-enyl, 2-methoxyethyl, 2,2-dimethoxyethyl, cyclopropyl, cyclopentyl, cyclohexyl, 2-fluorocyclopropyl, 2-trifluoromethylcyclohexyl, 3-trifluoromethylcyclohexyl, 4-trifluoromethylcyclohexyl, 3,5-di(trifluoromethyl)cyclohexyl, cyclopropylmethyl, dichlorocyclopropylmethyl, 1-cyclohexylethyl, 2-furylmethyl, 2-tetrahydrofurylmethyl, 2-thienylmethyl, 1,3-dioxolan-2-ylmethyl, propargyl, methoxycarbonylmethyl, ethoxycarbonylmethyl, 2-aminoethyl, 3-aminopropyl, 2-dimethylaminoethyl, cyanomethyl, 2-cyanoethyl, 2-vinyloxyethyl, pyrrolidinyl, piperidinyl, morpholinyl, thiomorpholinyl or piperazinyl,
R² represents hydrogen, methyl, ethyl, n- or i-propyl, n-, i-, s- or t-butyl, allyl, propargyl, 2,2,2-trifluoroethyl, 1-(1,1,1-trifluoromethyl)ethyl, cyclopropylmethyl, cyclobutylmethyl, cyclopentylmethyl, cyclohexylmethyl or cyclopropyl or
R¹ and R² together with the nitrogen atom to which they are attached represent pyrrolidinyl, piperidinyl, morpholinyl, thiomorpholinyl, piperazinyl, 5-methyl-3,6-dihydro-1(2H)-pyridinyl, 5-ethyl-3,6-dihydro-1-(2H)-pyridinyl, or tetrahydro-1-(2H)-pyridazinyl, each of which is optionally mono- or disubstituted by identical or different substituents from the group consisting of fluorine, chlorine, bromine, cyano, hydroxyl, methyl, ethyl, trifluoromethyl, methylcarbonyl, methylcarbonylamino or methoxycarbonyl, or represent a grouping of the formula 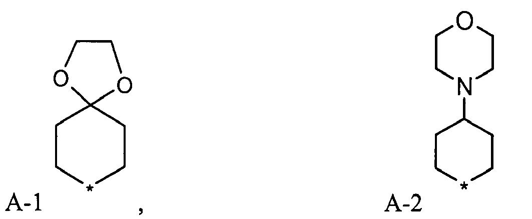 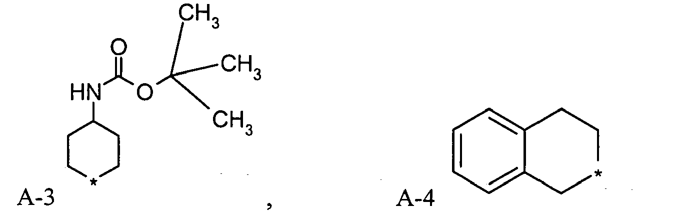 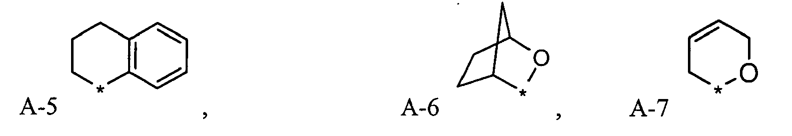 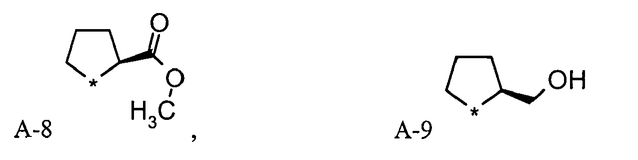 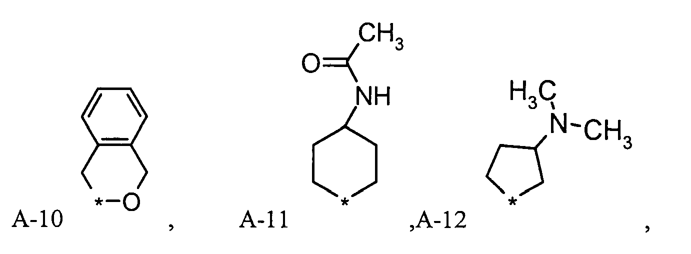 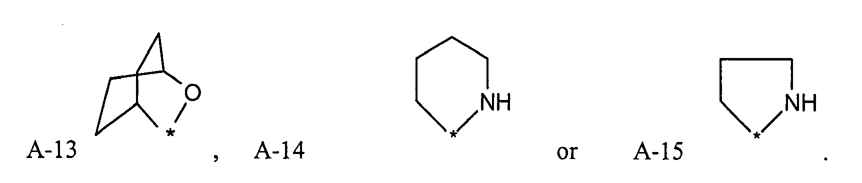
R³ represents phenyl which is mono- to trisubstituted in positions 2, 4 and/or 6 by fluorine and/or chlorine,
or
R³ represents 2-trifluoromethylphenyl, 2-chloro-5-nitrophenyl or 2-chloro-4-methoxyphenyl,
X¹ represents hydrogen or chlorine and
X² represents fluorine, chlorine, bromine, iodine, cyano, nitro, methyl, cyclopropyl, formyl, thiocarbamoyl or methoximinomethyl.

5. Process for preparing pyrazolopyrimidines of the formula (I) according to one or more of Claims 1 to 4, **characterized in that**
a) halopyrazolopyrimidines of the formula 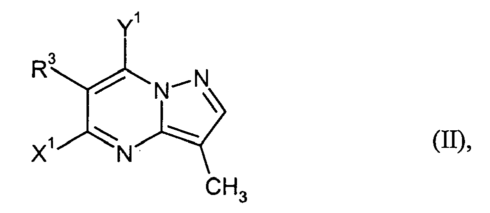 in which
R³ and X¹ are as defined above,
X³ represents halogen, cyano, nitro, alkyl, haloalkyl, cycloalkyl, thiocarbamoyl, alkoxycarbonyl or alkylcarbonyl and
Y¹ represents halogen
are reacted with amines of the formula 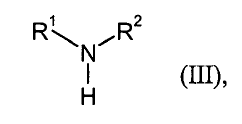 in which
R¹ and R² are as defined above,
if appropriate in the presence of a diluent, if appropriate in the presence of a catalyst and if appropriate in the presence of an acid acceptor,
or
b) pyrazolopyrimidines of the formula 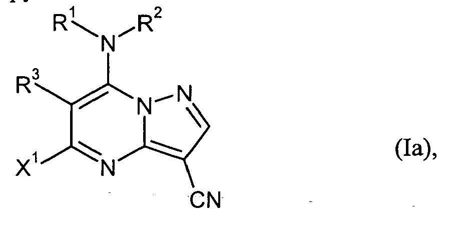 in which
R¹, R², R³ and X¹ are as defined above,
are reacted with diisobutylaluminium hydride in the presence of aqueous ammonium chloride solution and in the presence of an organic diluent,
or
c) pyrazolopyrimidines of the formula 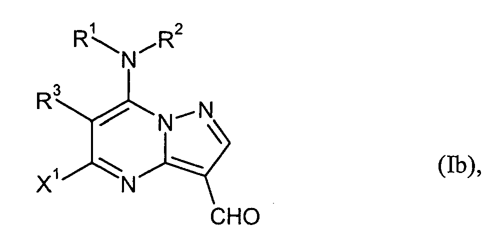 in which
R¹, R², R³ and X¹ are as defined above,
are reacted with amino compounds of the formula
H₂N-OR⁴ (IV),
in which
R⁴ represents hydrogen or alkyl,
in the presence of a diluent and if appropriate in the presence of a catalyst, where the amino compounds of the formula (IV) can also be employed in the form of their acid addition salts,
and, if appropriate, an acid is added to the resulting compounds of the formula (I), in which
R¹ represents amino.

6. Compositions for controlling harmful organisms, **characterized in that** they comprise at least one pyrazolopyrimidine of the formula (I) according to one or more of Claims 1 to 4 or an acid addition salt thereof, in addition to extenders and/or surfactants.

7. Use of pyrazolopyrimidines of the formula (I) according to one or more of Claims 1 to 4 or of acid addition salts thereof for controlling harmful organisms in plant and material protection.

8. Method for controlling harmful organisms, **characterized in that** pyrazolopyrimidines of the formula (I) according to one or more of Claims 1 to 4 or acid addition salts thereof are applied to the harmful organisms and/or their habitat.

9. Process for preparing compositions for controlling harmful organisms, **characterized in that** pyrazolopyrimidines of the formula (I) according to one or more of Claims 1 to 4 or acid addition salts thereof are mixed with extenders and/or surfactants.

10. Compositions for controlling pests, **characterized in that** they comprise at least one pyrazolopyrimidine of the formula (I) according to one or more of Claims 1 to 4 or an acid addition salt thereof in addition to extenders and/or surfactants.

11. Use of pyrazolopyrimidines of the formula (I) according to one or more of Claims 1 to 4 or of acid addition salts thereof for controlling pests.

12. Method for controlling pests, **characterized in that** pyrazolopyrimidines of the formula (I) according to one or more of Claims 1 to 4 or acid addition salts thereof are applied to pests and/or their habitat.

13. Process for preparing compositions for controlling pests, **characterized in that** pyrazolopyrimidines of the formula (I) according to one or more of Claims 1 to 4 or acid addition salts thereof are mixed with extenders and/or surfactants.

14. Halopyrazolopyrimidines of the formula 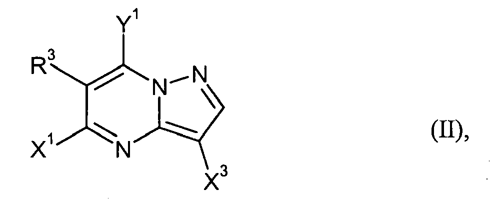 in which
R³ is as defined in Claim 1,
X¹ represents hydrogen or halogen, and
X³ represents halogen, cyano, nitro, alkyl, thiocarbamoyl, cycloalkyl, haloalkyl, alkoxycarbonyl or alkylcarbonyl and
Y¹ represents halogen.

15. Process for preparing halopyrazolopyrimidines of the formula (II) according to Claim 14, **characterized in that**
d) hydroxypyrazolopyrimidines of the formula 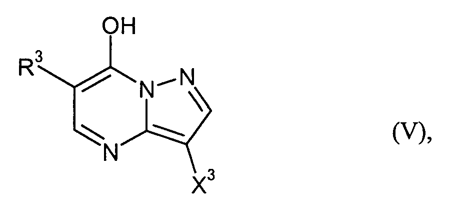 in which
R³ and X³ are as defined above
are reacted with halogenating agents, if appropriate in the presence of a diluent,
or
e) dihydroxypyrazolopyrimidines of the formula 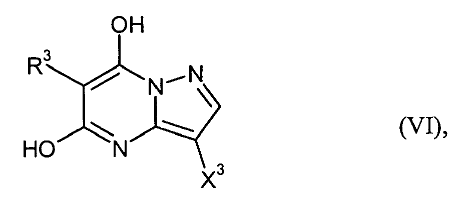 in which
R³ and X³ are as defined above,
are reacted with halogenating agents, if appropriate in the presence of a diluent.

16. Hydroxypyrazolopyrimidines of the formula 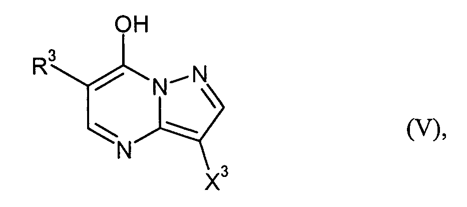 in which
R³ is as defined in Claim 1
X³ represents halogen, cyano, nitro, alkyl, thiocarbamoyl, cycloalkyl, haloalkyl, alkoxycarbonyl or alkylcarbonyl.

17. Process for preparing hydroxypyrazolopyrimidines of the formula (V) according to Claim 16, **characterized in that**
f) acrylic acid esters of the formula 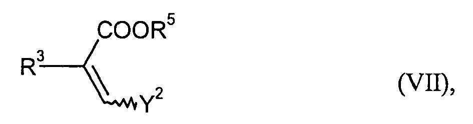 in which
R³ is as defined above,
R⁵ represents alkyl and
Y² represents alkoxy or dialkylamino
are reacted with aminopyrazoles of the formula 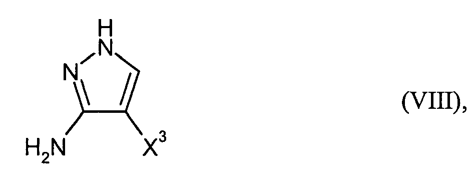 in which
X³ is as defined above,
if appropriate in the presence of a diluent and if appropriate in the presence of a base.

18. Dihydroxypyrazolopyrimidines of the formula 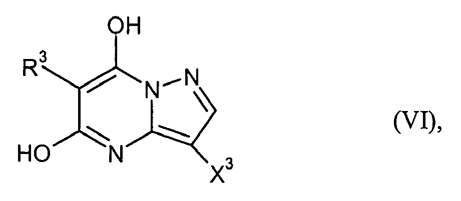 in which
R³ is as defined in Claim 1
X³ represents halogen, cyano, nitro, alkyl, thiocarbamoyl, cycloalkyl, haloalkyl, alkoxycarbonyl or alkylcarbonyl.

19. Process for preparing dihydroxypyrazolopyrimidines of the formula (VI) according to Claim 18, **characterized in that**
g) malonic esters of the formula 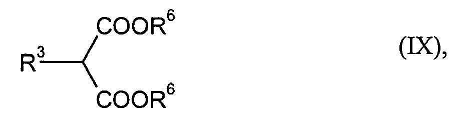
in which
R³ is as defined above and
R⁶ represents alkyl,
are reacted with aminopyrazoles of the formula 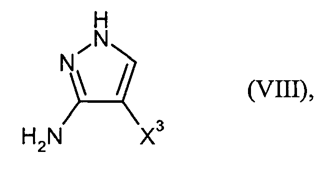 in which
X³ is as defined above,
if appropriate in the presence of a diluent and if appropriate in the presence of a strong base.

## Revendications

1. Pyrazolopyrimidines de la formule : 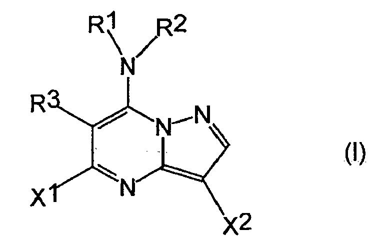 dans laquelle :
R¹ représente:
le radical hydroxy, amino, un radical alcoyle ayant 1 à 6 atomes de carbone, le cas échéant substitué par un ou des atomes d'halogène, un ou des radicaux cyano, hydroxy, amino, phényle, hétérocyclyle, alcoxy ayant 1 à 4 atomes de carbone, alcoxycarbonyle ayant 1 à 4 atomes de carbone dans la partie alcoxy, alcoylamino ayant 1 à 4 atomes de carbone, dialcoylamino ayant 2 à 8 atomes de carbone, cycloalcoyle ayant 3 à 6 atomes de carbone, halogénocycloalcoyle ayant 3 à 6 atomes de carbone et 1 à 5 atomes d'halogène, alcoylthio ayant 1 à 4 atomes de carbone, oxo, hydroxyimino et/ou alcoxyimino ayant 1 à 4 atomes de carbone,
un radical alcényle ayant 2 à 6 atomes de carbone, le cas échéant substitué par un ou des atomes d'halogène, un ou des radicaux cycloalcoyle, cyano, phényle et/ou hétérocyclyle,
un radical alcynyle ayant 2 à 6 atomes de carbone, le cas échéant substitué par un ou des atomes d'halogène, un ou des radicaux cycloalcoyle, cyano, phényle et/ou hétérocyclyle,
un radical cycloalcoyle ayant 3 à 7 atomes de carbone, le cas échéant substitué par un ou des atomes d'halogène, un ou des radicaux cycloalcoyle, cyano, halogénoalcoyle ayant 1 à 2 atomes de carbone et 1 à 5 atomes d'halogène, phényle et/ou hétérocyclyle,
un radical alcoxy ayant 1 à 7 atomes de carbone, le cas échéant substitué par un ou des atomes d'halogène, un ou des radicaux cycloalcoyle, cyano, phényle et/ou hétérocyclyle,
un radical alcényloxy ayant 2 à 6 atomes de carbone, le cas échéant substitué par un ou des atomes d'halogène, un ou des radicaux cycloalcoyle, cyano, phényle et/ou hétérocyclyle,
un radical alcynyloxy ayant 2 à 6 atomes de carbone, le cas échéant substitué par un ou des atomes d'halogène, un ou des radicaux cycloalcoyle, cyano, phényle et/ou hétérocyclyle,
un radical cycloalcoyloxy ayant 3 à 7 atomes de carbone, le cas échéant substitué par un ou des atomes d'halogène, un ou des radicaux cycloalcoyle, cyano, phényle et/ou hétérocyclyle,
un radical alcoylamino ayant 1 à 7 atomes de carbone, le cas échéant substitué par un ou des atomes d'halogène, un ou des radicaaux cycloalcoyle, cyano, phényle et/ou hétérocyclyle,
un radical dialcoylamino ayant 1 à 7 atomes de carbone dans chaque reste alcoyle, le cas échéant substitué par un ou des atomes d'halogène, un ou des radicaux cycloalcoyle, cyano, phényle et/ou hétérocyclyle,
un radical alcénylamino ayant 2 à 6 atomes de carbone, le cas échéant substitué par un ou des atomes d'halogène, un ou des radicaux cycloalcoyle, cyano, phényle et/ou hétérocyclyle,
un radical alcynylamino ayant 2 à 6 atomes de carbone, le cas échéant substitué par un ou des atomes d'halogène, un ou des radicaux cycloalcoyle, cyano, phényle et/ou hétérocyclyle,
un radical cycloalcoylamino ayant 3 à 7 atomes de carbone, le cas échéant substitué par un ou des atomes d'halogène, un ou des radicaux cycloalcoyle, cyano, phényle et/ou hétérocyclyle,
un radical N-cycloalcoyl-N-alcoylamino ayant 3 à 7 atomes de carbone dans la partie cycloalcoyle et 1 à 7 atomes de carbone dans la partie alcoyle, le cas échéant substitué par un ou des atomes d'halogène, un ou des radicaux cycloalcoyle, cyano, phényle et/ou hétérocyclyle,
un radical alcoylydèneamino ayant 2 à 6 atomes de carbone, le cas échéant substitué par un ou dés atomes d*'*halogène, un ou des radicaux cycloalcoyle, cyano, phényle et/ou hétérocyclyle, ou
un radical hétérocyclyle ayant 5 ou 6 membres cycliques, le cas échéant substitué par un ou des atomes d'halogène, un ou des radicaux cycloalcoyle, cyano, phényle et/ou hétérocyclyle,
où les restes hétérocyclyle cités peuvent être substitués une à trois fois, de manière identique ou différente, par
un atome d'halogène, un radical hydroxy, phényle, 1,2-dioxyéthylène, alcoyle ayant 1 à 4 atomes de carbone, halogénoalcoyle ayant 1 ou 2 atomes de carbone et 1 à 5 atomes d'halogène, alcoxy ayant 1 à 4 atomes de carbone, alcoylthio ayant 1 à 4 atomes de carbone, halogénoalcoxy ayant 1 ou 2 atomes de carbone et 1 à 5 atomes d'halogène, halogénoalcoylthio ayant 1 ou 2 atomes de carbone et 1 à 5 atomes d'halogène, et où les restes hétérocycliques cités sont saturés ou partiellement insaturés,
et où les restes phényle cité peuvent être substitués une à trois fois, de manière identique ou différente, par
un atome d*'*halogène, un radical cyano, nitro, amino, hydroxy, formyle, carboxy, carbamoyle, thiocarbamoyle ;
un radical alcoyle, alcoxy, alcoylthio, alcoylsulfinyle ou alcoylsulfonyle chaque fois linéaire ou ramifié, ayant chaque fois, 1 à 6 atomes de carbone ;
un radical alcényle ou alcényloxy, chaque fois linéaire ou ramifié, ayant chaque fois, 2 à 6 atomes de carbone;
un radical halogénoalcoyle, halogénoalcoxy, halogénoalcoylthio, halogénoalcoylsulfinyle ou halogénoalcoylsulfonyle chaque fois linéaire ou ramifié, ayant chaque fois, 1 à 6 atomes de carbone et 1 à 13 atomes d'halogène identiques ou différents;
un radical halogénoalcényle ou halogénoalcényloxy, chaque fois linéaire ou ramifié, ayant chaque fois, 2 à 6 atomes de carbone et 1 à 13 atomes d'halogène identiques ou différents ;
un radical alcoylamino, dialcoylamino, alcoylcarbonyle, alcoylcarbonyloxy, alcoxycarbonyle, alcoylsulfonyloxy, hydroximinoalcoyle ou alcoximinoalcoyle chaque fois linéaire ou ramifié, ayant chaque fois, 1 à 6 atomes de carbone dans la partie alcoyle ;
un radical cycloalcoyle ayant 3 à 6 atomes de carbone ;
un radical 1,4-butandiyle, 1,3-propandiyle reliés en positions 2,3, méthylènedioxy (-O-CH₂-O-) ou 1,2-éthylènedioxy (-O-CH₂-CH₂-O-), où ces restes peuvent être substitués une ou plusieurs fois, de manière identique ou différente, par un atome d'halogène, un radical alcoyle ayant 1 à 4 atomes de carbone et/ou halogénoalcoyle ayant 1 à 4 atomes de carbone et 1 à 9 atomes d'halogène identiques ou différents;
R² représente:
l'atome d'hydrogène,
un radical alcoyle ayant 1 à 4 atomes de carbone, le cas échéant substitué par un ou des atomes d'halogène, un ou des radicaux cycloalcoyle ayant 3 à 6 atomes de carbone, alcoxy ayant 1 à 4 atomes de carbone, alcoylthio ayant 1 à 4 atomes de carbone dans oxo, hydroxyimino et/ou alcoxyimino ayant 1 à 4 atomes de carbone,
un radical alcényle ayant 2 à 4 atomes de carbone, le cas échéant substitué par un ou des atomes d'halogène et/ou un ou des radicaux cycloalcoyle ayant 3 à 6 atomes de carbone,
un radical alcynyle ayant 2 à 6 atomes de carbone, le cas échéant substitué par un ou des atomes d'halogène et/ou un ou des radicaux cycloalcoyle ayant 3 à 6 atomes de carbone,
un radical cycloalcoyle ayant 3 à 6 atomes de carbone, le cas échéant substitué par un ou des atomes d'halogène et/ou un ou des radicaux cycloalcoyle ayant 3 à 6 atomes de carbone, ou
R¹ et R², avec l'atome d'azote sur lequel ils sont liés, représentent un hétérocycle ayant 3 à 6 membres, qui est saturé ou partiellement insaturé, qui en plus de l'atome d'azote déjà mentionné, peut contenir encore un autre hétéroatome de la série des atomes d'azote, d'oxygène et de soufre, et qui peut être substitué une à trois fois, de manière identique ou différente, par
un atome d'halogène, un radical hydroxy, cyano, morpholinyle, amino, un cycle phényle condensé, un pont méthylène ou éthylène,
un radical alcoyle ayant 1 à 4 atomes de carbone,
un radical halogénoalcoyle ayant 1 à 4 atomes de carbone et 1 à 9 atomes d'halogène identiques ou différents,
un radical alcoylcarbonylamino ayant 1 à 4 atomes de carbone dans la partie alcoyle,
un radical dialcoylamino ayant 2 à 8 atomes de carbone,
un radical alcoxycarbonylamino ayant 1 à 4 atomes de carbone dans la partie alcoxy,
un radical di(alcoxycarbonyl)amino ayant 2 à 8 atomes de carbone dans les parties alcoxy,
un radical hydroxyalcoyle ayant 1 à 4 atomes de carbone,
un radical alcoxycarbonyle ayant 1 à 4 atomes de carbone dans la partie alcoxy, et/ou
un radical alcoylcarbonyle ayant 1 à 4 atomes de carbone dans la partie alcoyle ;
R³ représente un radical phényle, qui peut être substitué une à quatre fois, de manière identique ou différente par
un atome d'halogène, un radical cyano, nitro, amino, hydroxy, formyle, carboxy, carbamoyle, thiocarbamoyle ;
un radical alcoyle, alcoxy, alcoylthio, alcoylsulfinyle ou alcoylsulfonyle chaque fois linéaire ou ramifié, ayant chaque fois, 1 à 6 atomes de carbone ;
un radical alcényle ou alcényloxy, chaque fois linéaire ou ramifié, ayant chaque fois, 2 à 6 atomes de carbone ;
un radical halogénoalcoyle, halogénoalcoxy, halogénoalcoylthio, halogénoalcoylsulfinyle ou halogénoalcoylsulfonyle chaque fois linéaire ou ramifié, ayant chaque fois, 1 à 6 atomes de carbone et 1 à 13 atomes d'halogène identiques ou différents ;
un radical halogénoalcényle ou halogénoalcényloxy, chaque fois linéaire ou ramifié, ayant chaque fois, 2 à 6 atomes de carbone et 1 à 11 atomes d'halogène identiques ou différents ;
un radical alcoylamino, dialcoylamino, alcoylcarbonyle, alcoylcarbonyloxy, alcoxycarbonyle, alcoylsulfonyloxy, hydroximinoalcoyle ou alcoximinoalcoyle chaque fois linéaire ou ramifié, ayant chaque fois, 1 à 6 atomes de carbone dans la partie alcoyle ;
un radical cycloalcoyle ayant 3 à 6 atomes de carbone ;
un radical 1,4-butandiyle, 1,3-propandiyle reliés en positions 2,3, méthylènedioxy (-O-CH₂-O-) ou 1,2-éthylènedioxy (-O-CH₂-CH₂-O-), où ces restes peuvent être substitués une ou plusieurs fois, de manière identique ou différente, par un atome d'halogène, un radical alcoyle ayant 1 à 4 atomes de carbone et/ou halogénoalcoyle ayant 1 à 4 atomes de carbone et 1 à 9 atomes d'halogène identiques ou différents ;
X¹ représente l'atome d'hydrogène, de fluor, de chlore ou de brome ;
X² représente le radical cyano, l'atome de fluor, de chlore, de brome, d'iode, le radical nitro, formyle, un radical halogénoalcoyle ayant 1 à 4 atomes de carbone et 1 à 9 atomes de fluor, de chlore et/ou de brome, un radical alcoyle ayant 1 à 4 atomes de carbone, un radical cycloalcoyle ayant 3 à 6 atomes de carbone, un radical thiocarbamoyle, alcoxycarbonyle ayant 1 à 4 atomes de carbone dans la partie alcoxy, alcoylcarbonyle ayant 1 à 4 atomes de carbone dans la partie alcoyle, hydroximinoalcoyle ayant 1 à 4 atomes de carbone dans la partie alcoyle ou alcoxyiminoalcoyle ayant 1 à 4 atomes de carbone dans la partie alcoxy et 1 à 4 atomes de carbone dans la partie alcoyle,
ainsi que les sels d'addition d'acide des composés de la formule (I), dans lesquels R¹ représente amino.

2. Pyrazolopyrimidines de la formule: 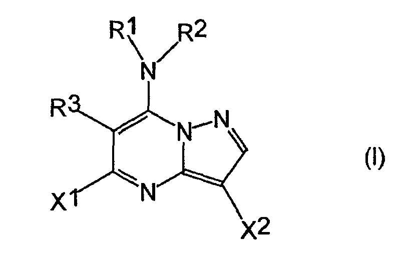 dans laquelle :
R¹ représente le radical hydroxy, amino, méthyle, éthyle, n-propyle, i-propyle, n-butyle, i-butyle, s-butyle, t-butyle, n-pentyle, i-pentyle, 1,2-diméthylpropyle, 2,2-diméthylpropyle, 1,2,2-triméthylpropyle, ou
R¹ représente le radical méthoxyméthyle, 2-méthoxyéthyle, méthylthiométhyle, 2-méthylthioéthyle, hydroximinométhyle; méthoximinométhyle, acétylméthyle, 2-hydroximinopropyle, 2-méthoximinopropyle, allyle, 2-méthylprop-2-ényle, propargyle, 2,2,2-trifluoroéthyle, 1-(trifluorométhyl)éthyle, 3,3,3-trifluoropropyle, cyclopropylméthyle, cycloproproxy, cyclobutyloxy, cyclopentyloxy, cyclohexyloxy, difluorométhoxy, trifluorométhoxy, difluorochlorométhoxy, trifluoroéthoxy, méthylamino, éthylamino, n- ou i-propylamino, n-, i-, s- ou t-butylamino, diméthylamino, diéthylamino, trifluoroéthylamino, cyclohexylamino, 2-cyanoéthylamino, allylamino, 1-cyclopropyléthylamino, cyclopropylamino, cyclobutylamino, cyclopentylamino, cyclohexylamino, 1-méthyléthylidèneamino,
un radical cyclopropyle, cyclobutyle, cyclopentyle, cyclohexyle, pyrrolidinyle, pipéridinyle, morpholinyle, thiamorpholinyle, pipérazinyle, chaque fois le cas échéant substitué une ou deux fois, de manière identique ou différente par l'atome de fluor, de chlore et/ou le radical méthyle, ou
un radical pyridylméthyloxy ou thiazolylméthoxy le cas échéant substitué, ou
R¹ représente le radical (2,2-dichlorocyclopropyl)méthyle, (2-furyl)méthyle, (2-tétrahydrofuryl)méthyle, (2-tétrahydropyrannyl)méthyle, 1,3-dioxolan-2-ylméthyle, 1-cyclopropyléthyle, benzyloxy, 2,4-dichlorobenzyloxy, 2,6-dichlorobenzyloxy, 2-chlorobenzyloxy, 2-fluorocyclopropyle, 2-hexahydropyrannyloxy, 2-thiénylméthyle, 2-trifluorométhylcyclohexyle, 3-(diméthylamino)propyle, 3,5-bis(trifluorométhyl)cyclohexyle, 3,5-dichlorobenzyloxy, 3-aminopropyle, 3-chlorobenzyloxy, 3-trifluorométhylbenzyloxy, 3-trifluorométhylcyclohexyle, 4-trifluorométhylcyclohexyle, 4-chlorobenzyloxy, 4-fluorobenzyloxy, 4-trifluorométhylbenzyloxy, -C(CH₃)₂-CF₃, -C(CH₃)₂-CH₂-COCH₃, -CH(CH₂OH)-COOCH₃, -CH(CH₃)-CH(O-CH₃)₂, -CH(CH₃)-CH=CH₂, -CH (CH₃)-CH₂-CH (CH₃)₂, -CH(CH₃)-CH₂-O-CH₃,-CH(CH₃)-CH₂-OH, -CH(CH₃)-COOCH₃, -CH(CH₃)-COO-t-butyle, -CH₂-C(CH₃)=CH₂, -CH₂-CH(OCH₃)₂, -CH₂-CH₂-CF₃, -CH₂-CH₂-Cl, -CH₂-CH₂-CN, -CH₂-CH₂-N (CH₃)₂, -CH₂-CH₂-NH₂, -CH₂-CHF₂, -CH₂-CN, -CH₂-COOC₂H₅, -CH₂-COOCH₃, i-butoxy, -NH-CH₂-CF₂-CHF₂, -NH-CH₂-CF₃, -NH-CH₂-CH(CH₃)₂, méthoxy, éthoxy, i-propoxy, t-butoxy ou -O-CH(CH₃)-CH₂-CH₃,
où les restes thiazolyle et pyridyle cités peuvent être substitués une ou deux fois dans le cas de thiazolyle et une à trois fois dans le cas de pyridyle, chaque fois de manière identique ou différente, par l'atome de fluor, de chlore, de brome, le radical méthyle, éthyle, n- ou i-propyle, n-, i-, s- ou t-butyle, méthoxy, éthoxy, n- ou i-propoxy, n-, i-, s- ou t-butoxy, méthylthio, éthylthio, n- ou i-propylthio, difluorométhoxy, trifluorométhoxy, difluorochlorométhoxy, trifluoroéthoxy, difluorométhylthio, difluorochlorométhylthio, dichlorofluorométhylthio, trifluorométhylthio et/ou phényle,
et où les restes benzyloxy cités peuvent être substitués une à trois fois dans la partie phényle, de manière identique ou différente, par
l'atome de fluor, de chlore, de brome, le radical cyano, nitro, amino, hydroxy, formyle, carboxy, carbamoyle, thiocarbamoyle, méthyle, éthyle, n- ou i-propyle, n-, i-, s- ou t-butyle, méthoxy, éthoxy, n- ou i-propoxy, méthylthio, éthylthio, n- ou i-propylthio, méthylsulfinyle, éthylsulfinyle, méthylsulfonyle ou éthylsulfonyle, trifluorométhyle, trifluoroéthyle, difluorométhoxy, trifluorométhoxy, difluorochlorométhoxy, trifluoroéthoxy, difluorométhylthio, difluorochlorométhylthio, trifluorométhylthio, trifluorométhylsulfinyle, trifluorométhylsulfonyle, méthylamino, éthylamino, n- ou i-propylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle, éthoximinoéthyle, cyclopropyle, cyclobutyle, cyclopentyle ou cyclohexyle,
un radical 1,3-propandiyle relié en positions 2,3, méthylènedioxy (-O-CH₂-O-) ou 1,2-éthylènedioxy (-O-CH₂-CH₂-O-), où ces restes peuvent être substitués une ou plusieurs fois, de manière identique ou différente, par l'atome de fluor, de chlore, le radical méthyle, éthyle, n-propyle, i-propyle et/ou trifluorométhyle ;
R² représente l'atome d'hydrogène, le radical méthyle, éthyle, n- ou i-propyle, n-, i-, s- ou t-butyle, méthoxyméthyle, 2-méthoxyéthyle, méthylthiométhyle, 2-méthylthioéthyle, hydroximinométhyle, méthoximinométhyle, acétylméthyle, 2-hydroximinopropyle, 2-méthoximinopropyle, allyle, propargyle, 2,2,2-trifluoroéthyle, 1-(1,1,1-trifluorométhyl)éthyle, cyclopropylméthyle, cyclobutylméthyle, cyclopentylméthyle ou cyclohexylméthyle, ou
R¹ et R², avec l'atome d'azote sur lequel ils sont liés, représentent un radical 1-pyrrolinyle, 3-pyrrolinyle, pyrrolidinyle, dihydropyridinyle, pipéridinyle, pyrazolinyle, pyrazolidinyle, imidazolinyle, imidazolidinyle, 1,2-diazinanyle, 1,3-diazinanyle, pipérazinyle, oxazolinyle, oxazolidinyle, isoxazolyle, isoxazolidinyle, tétrahydropyridazinyle, dihydrooxazinyle, morpholinyle, thiazolinyle, thiazolidinyle ou thiomorpholinyle, où les hétérocycles cités peuvent être substitués par
l'atome de fluor, de chlore, de brome, le radical cyano, nitro, amino, hydroxy, formyle, carboxy, carbamoyle, thiocarbamoyle, méthyle, éthyle, n- ou i-propyle, n-, i-, s- ou t-butyle, méthoxy, éthoxy, n- ou i-propoxy, méthylthio, éthylthio, n- ou i-propylthio, méthylsulfinyle, éthylsulfinyle, méthylsulfonyle ou éthylsulfonyle, trifluorométhyle, trifluoroéthyle, difluorométhoxy, trifluorométhoxy, difluorochlorométhoxy, trifluoroéthoxy, difluorométhylthio, difluorochlorométhylthio, trifluorométhylthio, trifluorométhylsulfinyle, trifluorométhylsulfonyle, méthylamino, éthylamino, n- ou i-propylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle, éthoximinoéthyle, cyclopropyle, cyclobutyle, cyclopentyle ou cyclohexyle,
par un cycle phényle condensé, ou
par un pont méthandiyle ou éthandiyle,
ou
R¹ et R² forment ensemble, un groupement de la formule : 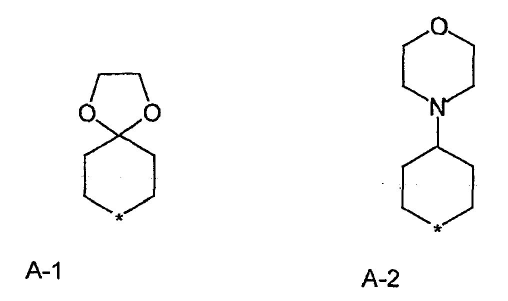 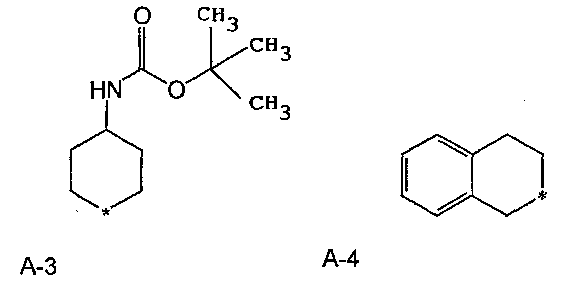 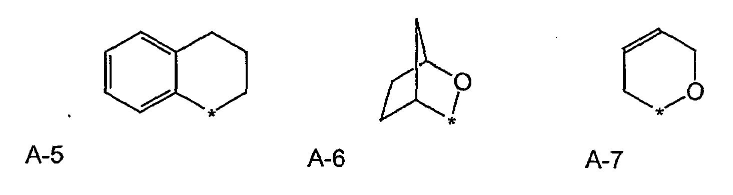 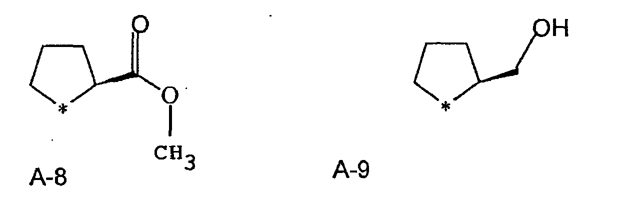 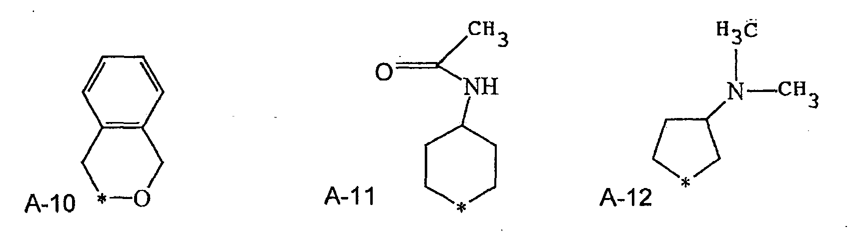 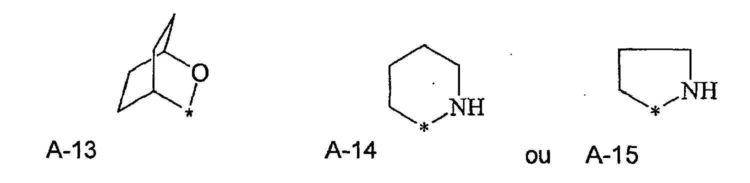
dans ces groupements, le site relié à l'atome d'azote est chaque fois **caractérisé par** *,
R³ représente un radical phényle, qui peut être substitué une à trois fois, de manière identique ou différente par
l'atome de fluor, de chlore, de brome, le radical cyano, nitro, formyle, méthyle, éthyle, n- ou i-propyle, n-, i-, s- ou t-butyle, allyle, propargyle, méthoxy, éthoxy, n- ou i-propoxy, méthylthio, éthylthio, n- ou i-propylthio, méthylsulfinyle, éthylsulfinyle, méthylsulfonyle, éthylsulfonyle, allyloxy, propargyloxy, trifluorométhyle, trifluoroéthyle, difluorométhoxy, trifluorométhoxy, difluorochlorométhoxy, trifluoroéthoxy, difluorométhylthio, difluorochlorométhylthio, trifluorométhylthio, trifluorométhylsulfinyle, trifluorométhylsulfonyle, trichloroéthynyloxy, trifluoroéthynyloxy, chloroallyloxy, iodopropargyloxy, méthylamino, éthylamino, n- ou i-propylamino, diméthylamino, diéthylamino, acétyle, propionyle, acétyloxy, méthoxycarbonyle, éthoxycarbonyle, hydroximinométhyle, hydroximinoéthyle, méthoximinométhyle, éthoximinométhyle, méthoximinoéthyle, éthoximinoéthyle, cyclopropyle, cyclobutyle, cyclopentyle ou cyclohexyle,
un radical 1,3-propandiyle relié en positions 2,3, méthylènedioxy (-O-CH₂-O-) ou 1,2-éthylènedioxy (-O-CH₂-CH₂-O-), où ces restes peuvent être substitués une ou plusieurs fois, de manière identique ou différente, par l'atome de fluor, de chlore, le radical méthyle, éthyle, n-propyle, i-propyle et/ou trifluorométhyle ;
X¹ représente l'atome d'hydrogène, de fluor ou de chlore ;
X² représente le radical cyano, l'atome de fluor, de chlore, de brome, d'iode, le radical formyle, trifluorométhyle, méthoxycarbonyle, méthylcarbonyle, hydroximinométhyle, méthoximinométhyle, thiocarbamoyle, nitro, méthyle, éthyle ou cyclopropyle.

3. Pyrazolopyrimidine de la formule (I) selon la revendication 1 ou 2, où
R³ représente phényle 2,4-, 2,5- ou 2,6-disubstitué, ou phényle 2-substitué ou phényle 2,4,6-trisubstitué.

4. Pyrazolopyrimidine de la formule (I) selon une ou plusieurs des revendications 1, 2 ou 3, où
R¹ represente le radical amino, hydroxy, méthyle, éthyle, n-propyle, i-propyle, n-butyle, i-butyle, s-butyle, t-butyle, 1,2-diméthylpropyle, 1,2,2-triméthylpropyle, 2,2-diméthylpropyle, trifluorométhyle, 2,2,2-trifluoroéthyle, 2,2-difluoroéthyle, 2,2,2-trifluoro-1-méthyléthyle, 3,3,3-trifluoropropyle, 2,2,2-trifluoro-1,1-diméthyléthyle, 3-méthylbutyle, allyle, 2-méthyl-prop-2-ényle, 2-méthoxyéthyle, 2,2-diméthoxyéthyle, cyclopropyle, cyclopentyle, cyclohexyle, 2-fluoro-cyclopropyle, 2-trifluorométhylcyclohexyle, 3-trifluorométhylcyclohexyle, 4-trifluorométhylcyclohexyle, 3,5-di(trifluorométhyl)cyclohexyle, cyclopropylméthyle, dichlorocyclopropylméthyle, 1-cyclohexyléthyle, 2-furylméthyle, 2-tétrahydrofurylméthyle, 2-thiénylméthyle, 1,3-dioxolan-2-ylméthyle, propargyle, méthoxycarbonylméthyle, éthoxycarbonylméthyle, 2-aminoéthyle, 3-aminopropyle, 2-diméthylaminoéthyle, cyanométhyle, 2-cyanoéthyle, 2-vinyloxyéthyle, pyrrolidinyle, pipéridinyle, morpholinyle, thiomorpholinyle ou pipérazinyle,
R² représente l'atome d'hydrogène, le radical méthyle, éthyle, n- ou i-propyle, n-, i-, s- ou t-butyle, allyle, propargyle, 2,2,2-trifluoroéthyle, 1-(1,1,1-trifluorométhyl) éthyle, cyclopropylméthyle, cyclobutylméthyle, cyclopentylméthyle, cyclohexyl-méthyle, ou cyclopropyle, ou
R¹ et R², avec l'atome d'azote sur lequel ils sont liés, représentent un radical pyrrolidinyle, pipéridinyle, morpholinyle, thiomorpholinyle, pipérazinyle, 5-méthyl-3,6-dihydro-1(2H)-pyridinyle, 5-éthyl-3,6-dihydro-1(2H)-pyridinyle ou tétrahydro-1(2H)-pyridazinyle, ou un groupement de la formule: 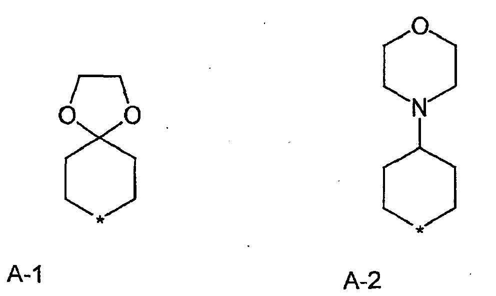 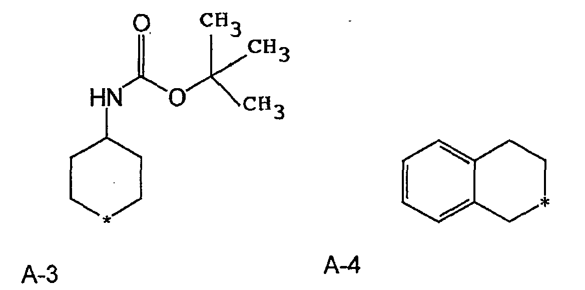 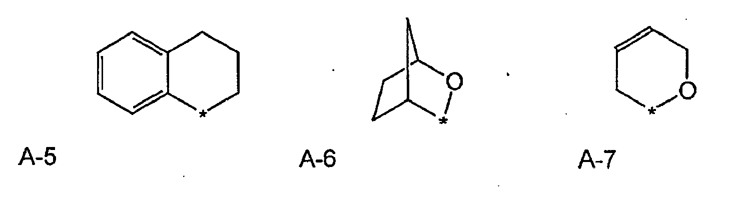 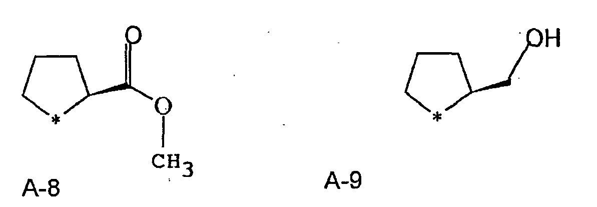 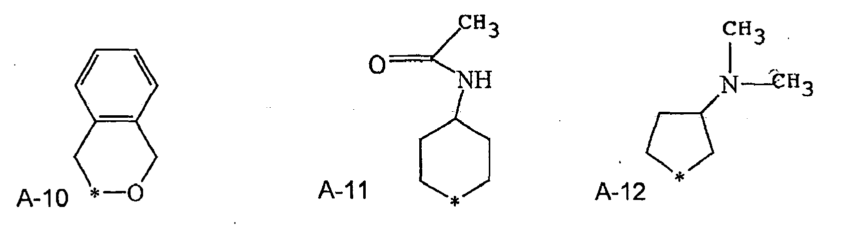 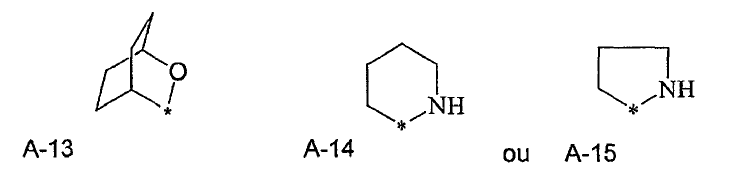
qui, le cas écheant, sont substitués une fois ou deux fois, de manière identique ou différente, par l'atome de fluor, chlore, brome, le radical cyano, hydroxy, méthyle, éthyle, trifluorométhyle, méthylcarbonyle, méthylcarbonylamino ou méthoxycarbonyle;
R³ représente phényle substitué une à trois fois en position 2, 4 et/ou 6, par l'atome de fluor et/ou l'atome de chlore,
ou
R³ représente 2-trifluorométhylphényle, 2-chloro-5-nitrophényle ou 2-chloro-4-méthoxyphényle,
X¹ représente l'atome d'hydrogène ou de chlore, et
X² représente l'atome de fluor, de chlore, de brome, d'iode, le radical cyano, nitro, méthyle, cyclopropyle, formyle, thiocarbamoyle, ou méthoximinométhyle.

5. Procédé de préparation de pyrazolopyrimidines de la formule (I) selon une ou plusieurs des revendications 1 à 4, **caractérisé en ce que**
a) on fait réagir les halogénopyrazolopyrimidines de la formule : 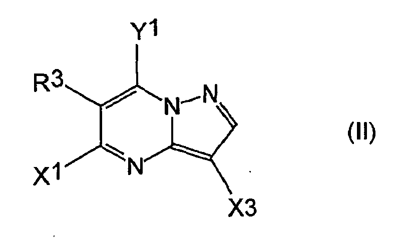 dans laquelle
R³ et X¹ ont les significations données ci-dessus,
X³ représente un atome d'halogène, le radical cyano, nitro, alcoyle, halogénoalcoyle, cycloalcoyle, thiocarbamoyle, alcoxycarbonyle ou alcoxylcarbonyle, et
Y¹ représente un atome d'halogène,
avec des amines de la formule : 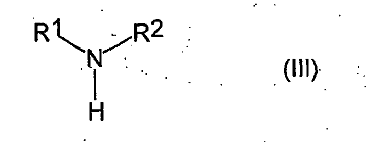 dans laquelle
R¹ et R² ont les significations données ci-dessus,
le cas échéant en présence d'un agent de dilution, le cas échéant en présence d'un catalyseur et le cas échéant, en présence d'un accepteur d'acide,
ou
b) on fait réagir les pyrazolopyrimidines de la formule : 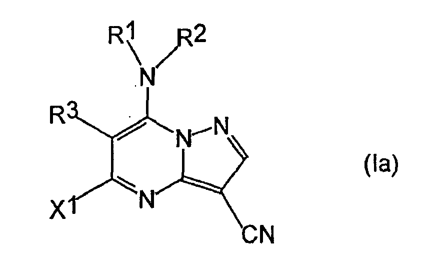 dans laquelle
R¹, R², R³ et X¹ ont les significations données ci-dessus,
avec l'hydrure de diisobutyl-aluminium, en présence d'une solution aqueuse de chlorure d'ammonium, ainsi qu'en présence d'un agent de dilution organique,
ou
c) on fait réagir les pyrazolopyrimidines de la formule : 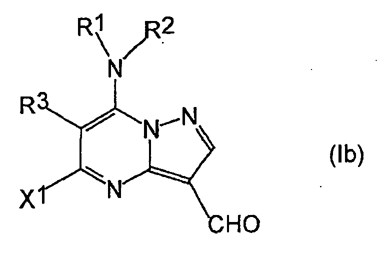 dans laquelle
R¹, R², R³ et X¹ ont les significations données ci-dessus,
avec des composés amino de la formule :
H₂N-OR⁴ (IV)
dans laquelle
R⁴ représente l'atome d'hydrogène ou un radical alcoyle,
en présence d'un agent de dilution et le cas échéant, en présence d'un catalyseur, où les composés amino de la formule (IV) peuvent également être mis en oeuvre sous la forme de leurs sels d'addition d'acide,
et le cas échéant, sur les composés de la formule (I) ainsi obtenus, dans lesquels
R¹ représente un radical amino,
on additionne un acide.

6. Moyen pour lutter contre les organismes nuisibles, **caractérisé par** une teneur en au moins un pyrazolopyrimidine de la formule (I) selon une ou plusieurs des revendications 1 à 4 ou un sel d'addition d'acide de celui-ci, avec des diluants et/ou agents tensioactifs.

7. Utilisation des pyrazolopyrimidines de la formule (I) selon une ou plusieurs des revendications 1 à 4 ou de leurs sels d'addition d'acide pour lutter contre les organismes nuisibles dans la protection des végétaux et des matériaux.

8. Procédé pour lutter contre les organismes nuisibles, **caractérisé en ce que** l'on applique des pyrazolopyrimidines de la formule (I) selon une ou plusieurs des revendications 1 à 4 ou leurs sels d'addition d'acide sur les organismes nuisibles et/ou leur biotope.

9. Procédé de préparation d'agents pour lutter contre les organismes nuisibles, **caractérisé en ce que** l'on mélange des pyrazolopyrimidines de la formule (I) selon une ou plusieurs des revendications 1 à 4 ou leurs sels d'addition d'acide, avec des diluants et/ou agents tensioactifs.

10. Agent pour lutter contre les parasites, **caractérisé par** une teneur en au moins un pyrazolopyrimidine de la formule (I) selon une ou plusieurs des revendications 1 à 4 ou un sel d'addition d'acide de celui-ci, avec des diluants et/ou agents tensioactifs.

11. Utilisation des pyrazolopyrimidines de la formule (I) selon une ou plusieurs des revendications 1 à 4 ou de leurs sels d'addition d'acide pour lutter contre les parasites.

12. Procédé pour lutter contre les parasites, **caractérisé en ce que** l'on applique des pyrazolopyrimidines de la formule (I) selon une ou plusieurs des revendications 1 à 4 ou leurs sels d'addition d'acide sur les parasites et/ou leur biotope.

13. Procédé de préparation d'agents pour lutter contre les parasites, **caractérisé en ce que** l'on mélange des pyrazolopyrimidines de la formule (I) selon une ou plusieurs des revendications 1 à 4 ou leurs sels d'addition d'acide, avec des diluants et/ou agents tensioactifs.

14. Halogénopyrazolopyrimidines de la formule : 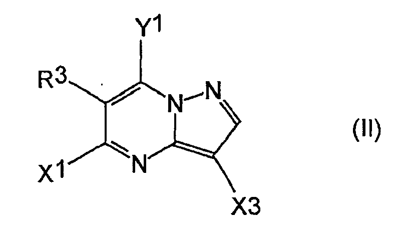 dans laquelle
R³ a la signification donnée à la revendication 1,
X¹ représente un atome d'hydrogène ou d'halogène, et
X³ représente un atome d'halogène, un radical cyano, nitro, alcoyle, thiocarbamoyle, cycloalcoyle, halogénoalcoyle, alcoxycarbonyle ou alcoylcarbonyle, et
Y¹ représente un atome d'halogène.

15. Procédé de préparation des halogénopyrazolopyrimidines de la formule (II) selon la revendication 14, **caractérisé en ce que**
d) on fait réagir les hydroxypyrazolopyrimidines de la formule : 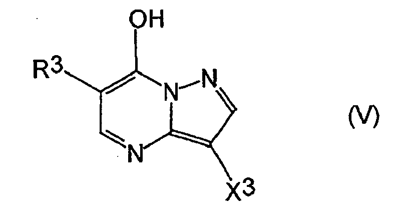 dans laquelle
R³ et X³ ont les significations données ci-dessus,
avec des agents d'halogénation, le cas échéant en présence d'un agent de dilution,
ou
e) on fait réagir les dihydroxypyrazolopyrimidines de la formule : 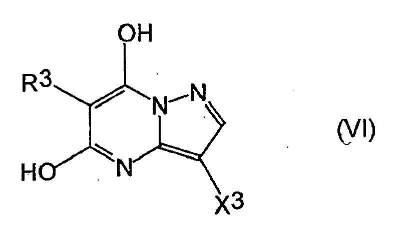 dans laquelle
R³ et X³ ont les significations données ci-dessus,
avec des agents d'halogénation, le cas échéant en présence d'un agent de dilution.

16. Hydroxypyrazolopyrimidines de la formule : 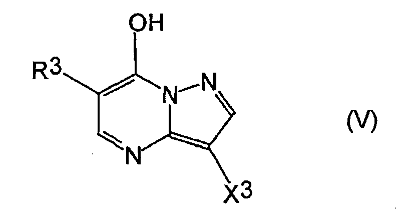 dans laquelle
R³ a la signification donnée à la revendication 1,
X³ représente un atome d'halogène, un radical cyano, nitro, alcoyle, thiocarbamoyle, cycloalcoyle, halogénoalcoyle, alcoxycarbonyle ou alcoylcarbonyle.

17. Procédé de préparation des hydroxypyrazolopyrimidines de la formule (V) selon la revendication 16, **caractérisé en ce que**
f) on fait réagir les esters d'acide acrylique de la formule: 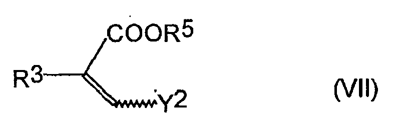 dans laquelle
R³ a la signification donnée ci-dessus,
R⁵ représente un radical alcoyle, et
Y² représente un radical alcoxy ou dialcoylamino,
avec des aminopyrazoles de la formule : 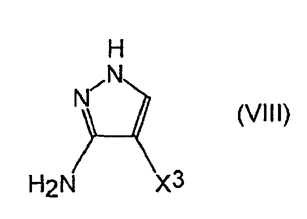 dans laquelle
X³ a la signification donnée ci-dessus,
le cas échéant en présence d'un agent de dilution, et le cas échéant, en présence d'une base.

18. Dihydroxypyrazolopyrimidines de la formule : 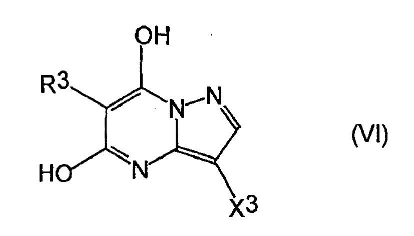 dans laquelle
R³ a la signification donnée à la revendication 1,
X³ représente un atome d'halogène, un radical cyano, nitro, alcoyle, thiocarbamoyle, cycloalcoyle, halogénoalcoyle, alcoxycarbonyle ou alcoylcarbonyle.

19. Procédé de préparation des dihydroxypyrazolopyrimidines de la formule (VI) selon la revendication 18, **caractérisé en ce que**
g) on fait réagir les esters d'acide malonique de la formule : 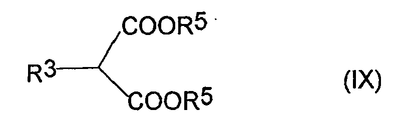
dans laquelle
R³ a la signification donnée ci-dessus,
R⁶ représente un radical alcoyle,
avec des aminopyrazoles de la formule : 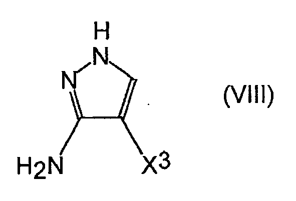 dans laquelle
X³ a la signification donnée ci-dessus,
le cas échéant en présence d'un agent de dilution, et le cas échéant, en présence d'une base forte.
